(19) Europäisches Patentamt
European Patent Office
Office européen des brevets

(11) EP 1 468 076 B1

(12) **EUROPEAN PATENT SPECIFICATION**

(45) Date of publication and mention of the grant of the patent:
**31.10.2012 Bulletin 2012/44**

(21) Application number: **02806301.4**

(22) Date of filing: **30.12.2002**

(51) Int Cl.:
***C12N 1/20*** *(2006.01)*

(86) International application number:
**PCT/AU2002/001768**

(87) International publication number:
**WO 2003/060105 (24.07.2003 Gazette 2003/30)**

(54) **AroQ-deficient Bordetella strain and uses thereof**

AroQ-defizienter Bordetella Stamm und dessen Verwendung

Souche de Bordetella déficiente en AroQ et son utilisation

(84) Designated Contracting States:
**AT BE BG CH CY CZ DE DK EE ES FI FR GB GR IE IT LI LU MC NL PT SE SI SK TR**

(30) Priority: **28.12.2001 AU PR977601**

(43) Date of publication of application:
**20.10.2004 Bulletin 2004/43**

(73) Proprietor: **Mukkur, Trilochan Kanwaljit Singh**
**Toowoomba, QLD 4350 (AU)**

(72) Inventors:
- **CORNFORD-NAIRN, Renee**
**Mt Rascal, Queensland 4350 (AU)**
- **DAGGARD, Grant, Edward**
**Toowoomba, Queensland 4350 (AU)**
- **MUKKUR, Trilochan, Kanwaljit, Singh**
**Toowoomba, Queensland 4350 (AU)**
- **ROSSETTI, Tony, Robert**
**Auchenflower, Queensland 4066 (AU)**

(74) Representative: **Mallalieu, Catherine Louise et al**
**D Young & Co LLP**
**120 Holborn**
**London EC1N 2DY (GB)**

(56) References cited:
**EP-A2- 1 108 790** **EP-B1- 0 400 958**
**EP-B1- 0 574 466** **AU-B1- 709 385**

- **DATABASE EMBL [Online] 7 December 2001 (2001-12-07), "Ralstonia solanacearum GMI1000 chromosome, complete sequence; segment 15/19" XP002367185 retrieved from EBI accession no. EM_PRO:AL646071 Database accession no. AL646071**
- **DATABASE EMBL [Online] 6 January 1999 (1999-01-06), "Aeromonas salmonicida salmonicida type II 3-dehydroquinase (aroD) gene, complete cds, and acetyl-CoA carboxylase subunit (fabE) gene, partial cds." XP002367186 retrieved from EBI accession no. EM_PRO: AF011408 Database accession no. AF011408**
- **DATABASE PROTEIN DATA BANK 13 September 2000 (2000-09-13), ROSZAK, A.W. ET AL.: "Crystal structure of type II dehydroquinase from Streptomyces coelicolor complexed with phosphate ions" XP002367187 retrieved from PROTEIN DATA BANK Database accession no. 1D0I -& ROSZAK ALEKSANDER W ET AL: "The structure and mechanism of the type II dehydroquinase from Streptomyces coelicolor" STRUCTURE (CAMBRIDGE), vol. 10, no. 4, April 2002 (2002-04), pages 493-503, XP002367179 ISSN: 0969-2126**
- **DATABASE EPO Proteins [Online] 14 December 2001 (2001-12-14), "Sequence 4970 from Patent WO0190366." XP002367188 retrieved from EBI accession no. EPOP:AX311985 Database accession no. AX311985**
- **SIMMONS CAMERON P ET AL: "Attenuation and vaccine potential of aroQ mutants of Corynebacterium pseudotuberculosis" INFECTION AND IMMUNITY, vol. 65, no. 8, 1997, pages 3048-3056, XP002367180 ISSN: 0019-9567**
- **MCARTHUR J.D. ET AL.: 'An aromatic amino acid auxotrophic mutant of Bordetella bronchiseptica is attenuated and immunogenic in a mouse model of infection' FEMS MICROBIOL. LETT. vol. 221, no. 1, April 2003, pages 7 - 16, XP002999002**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- ROBERTS M. ET AL.: 'Construction and characterization in vivo of Bordetella pertussis aroA mutants' INFECT. IMMUN. vol. 58, no. 3, March 1990, pages 732 - 739, XP009015169
- DATABASE EMBL [Online] 14 November 1997 'Pseudomonas aeruginosa protein-disulfide reductase (dipZ) and catabolic dehydroquinase (aroQ) genes, complete cds.' Retrieved from EBI, accession no. EMBL:AF010322 Database accession no. AF010322
- DATABASE EMBL [Online] 06 January 1999 'Aeromonas salmonicida salmonicida type II 3-dehydroquinase (aroD) gene, complete cds, and acetyl-CoA carboxylase subunit (fabE) gene, partial cds.' Retrieved from EBI, accession no. EMBL:AF011408 Database accession no. AF011408
- DATABASE UNIPROT [Online] 27 April 2001 'RecName: Full=3-dehydroquinate dehydratase; Short=3-dehydroquinase; EC=<A HREF="http://srs.ebi.ac.uk/srsbin/cgi-bin/w getz?[enzyme-ECNumber:4.2.1.10]+-e">4.2.1.1 0</A>; AltName: Full=Type II DHQase;' Retrieved from EBI, accession no. UNIPROT:P57903 Database accession no. P57903
- DATABASE UNIPROT [Online] 01 January 1998 'RecName: Full=3-dehydroquinate dehydratase 1; Short=3-dehydroquinase 1; EC=<A HREF="http://srs.ebi.ac.uk/srsbin/cgi-bin/w getz?[enzyme-ECNumber:4.2.1.10]+-e">4.2.1.1 0</A>; AltName: Full=Type II DHQase 1;' Retrieved from EBI, accession no. UNIPROT: O30557 Database accession no. O30557
- DATABASE UNIPROT [Online] 01 May 1999 'SubName: Full=Type II 3-dehydroquinase;' Retrieved from EBI, accession no. UNIPROT: Q9ZIW1 Database accession no. Q9ZIW1
- DATABASE UNIPROT [Online] 01 November 1995 'RecName: Full=3-dehydroquinate dehydratase; Short=3-dehydroquinase; EC=<A HREF="http://srs.ebi.ac.uk/srsbin/cgi-bin/w getz?[enzyme-ECNumber:4.2.1.10]+-e">4.2.1.1 0</A>; AltName: Full=Type II DHQase;' Retrieved from EBI, accession no. UNIPROT:P43877 Database accession no. P43877
- DATABASE UNIPROT [Online] 01 October 2000 'RecName: Full=3-dehydroquinate dehydratase; Short=3-dehydroquinase; EC=<A HREF="http://srs.ebi.ac.uk/srsbin/cgi-bin/w getz?[enzyme-ECNumber:4.2.1.10]+-e">4.2.1.1 0</A>; AltName: Full=Type II DHQase;' Retrieved from EBI, accession no. UNIPROT:Q9KV60 Database accession no. Q9KV60

## Description

### FIELD OF THE INVENTION

THE INVENTION relates generally to attenuated *Bordetella* strains of pathogenic origin. More particularly, the present invention relates to genetically modified *Bordetella* strains, which have been attenuated by disruption or inactivation of a gene encoding a metabolic protein, specifically a gene encoding a protein necessary for the biosynthesis of aromatic amino acids, and more specifically, the *aroQ* gene that encodes a dehydroquinase enzyme. The genetically modified *Bordetella* strain of the present invention has a reduced capacity to propagate in a mammalian host, but remains viable in the host for a period of time sufficient to induce a protective immune response against the natural pathogenic *Bordetella* counterpart. The present invention, therefore, also relates to the use of such genetically modified *Bordetella* strains in immunopotentiating compositions for treating and/or preventing *inter alia Bordetella* infections, and particularly pathogenic infections, caused by *Bordetella.* The present invention is also directed to the structure and sequence of *aroQ* from *Bordetella pertussis,* which are useful *inter alia* for the production of the genetically modified attenuated *Bordetella* strains of the present invention and for detecting and isolating variant *aroQ* genes and expression products.

Bibliographic details of various publications referred to by author in this specification are collected at the end of the description.

### BACKGROUND OF THE INVENTION

Whooping cough (pertussis), a respiratory disease caused by *Bordetella pertussis,* accounts for more than 300,000 deaths annually worldwide (Galazka 1992). *B. pertussis* is a non-invasive pathogen which localises to the tracheobronchial tree and produces a large array of potential virulence factors, which may play a role in the pathogenesis of pertussis (Weiss and Hewlett, 1986). A killed whole cell pertussis vaccine, generally given in combination with diphtheria and tetanus toxoids, has been available in many countries for over 40 years, and while its use seems to control pertussis epidemics, concerns over the reactogenicity, ranging from high fever, persistent crying, pain at the site of injection and possible existence of acute encephalopathy (Baraff *et al.* 1984; Pollock *et al.* 1984; Howson & Fineberg 1992) led to the development of acellular pertussis vaccines. Children under the age of two and up to the age of five years are extremely susceptible to whooping cough, however, the recent concern that young adults (vaccinated during their childhood) with waning immunity against whooping may serve as a reservoir for the pathogen, and thus may be passing it on to their children, have stimulated interest in the development of an alternative vaccine which can also be used safely in the adult population. The protective efficacy of the acellular vaccine has been the subject of controversy, however. Apart from the controversy on the efficacy of the acellular vaccine (DTaP), recent reports have highlighted that serious side reactions, particularly extensive swelling of the injected limb, occur in a notable percentage of children receiving booster vaccinations with DTaP (Rennels *et al*., 2000). The alternatives suggested by Mills (2001) are either to reduce the number of booster shots with ensuing reduced levels of immunity or to find a replacement adjuvant, which, unlike alum, favours the induction of Th1 responses that have been shown to be responsible for long-term protection against whooping cough.

Although the exact nature of immunity against infection with *B. pertussis* is still not completely understood, animal experiments are providing increasing evidence that while circulating antibodies may play a role in toxin neutralisation and prevention of bacterial attachment to respiratory epithelial cells, other immunological mechanisms such as cell-mediated immunity (CMI) may also be necessary for complete long-term protection (Mills *et al.* 1993). This suggestion is supported by the observation that *B. pertussis* is not exclusively an extracellular pathogen and can be internalised and survive within mammalian cells including macrophages (Ewanowich *et al.* 1989; Saukkonen *et al.* 1991). These findings suggest that protection against facultative intracellular pathogens, such as *B. pertussis,* may depend upon acquired cell-mediated resistance and activation of macrophages by interferon (IFN)-$\gamma$-producing T lymphocytes. Another cytokine, interleukin 12 (IL-12), a heterodimeric cytokine produced by monocyte/macrophages and B cells, which is known to increase activity of natural killer (NK) cells, has also been reported (Manetti *et al.* 1993) to shift the cytokine pattern of the CD4 cell types from a Th2 (IL4, IL5) to a Th1 cytokine secretion pattern (IFN-$\gamma$ and IL-2).

Redhead *et al.* (1993) reported that mice either convalescing from an active infection or those immunised with the inactivated whole cell vaccine eliminated bacteria significantly more effectively than those receiving an acellular vaccine. This finding was attributed to the induction of Th1-mediated cellular immune response by the inactivated whole cell vaccine and Th2-mediated humoral immune response by the acellular vaccine. These findings were contradicted by Zepp *et al.* (1996) who reported the induction of both the humoral and Th1-lymphocyte-mediated cytokine profiles.

More recently Canthaboo *et al.* (2000) compared the humoral and cellular immune responses of mice following vaccination with the killed whole cell pertussis vaccine (WCV: DTPw) and with DTaP and reported that the DTPw, although inducing a lower antibody titres to the pertussis toxin, filamentous haemagglutinin and pertactin, was more effective in activating macrophages and more protective as judged in intracerebral challenge and bacterial lung clearance experiments than the DTaP. These authors suggested that cell-mediated immunity may play a crucial role in eliminating bacteria which escape humoral defence mechanisms.

In the development of vaccines against many intracellular pathogens such as *Salmonella* species and *Shigella* species, live attenuated vaccines such as *aroA*-inactivated *S. typhimurium* and *aroD*-inactivated *Shigella flexneri* have been developed and found to stimulate both antibody and cell-mediated immune responses, which are protective in mouse models and the target species (Mukkur *et al.* 1987; Verma & Lindberg 1991). However, the inactivation of a particular gene in one pathogen that leads to an attenuated phenotype with effective stimulation of antibody and cell-mediated immune responses is generally not extrapolatable to other pathogens. For example, Roberts *et al.* (1990) developed an aromatic-dependent mutant (*aroA*) of *B. pertussis*, but it was found to persist in the lungs of mice for only a short period of time (4 days at reasonable numbers) thus casting doubt on its ability to stimulate the cell-mediated immunity (CMI) required for long-lasting protection. This result was unexpected given previous reports regarding the success of the *aroA* mutant of *Salmonella* species (Hoiseth and Stocker, 1981; Mukkur *et al.* 1987) as a successful vaccine. On the other hand, the *aroA* mutants of *Shigella* species were found to be poorer vaccines than the *aroD* deletion mutants of the same species (Verma and Lindberg 1991).

To date, it has not been possible to inactivate any specific gene of a *Bordetella* strain to permit the production of defective strain that is able to replicate only at a very low level in the host and that persists in the host long enough to allow the induction of a host-protective immune response, particularly cell-mediated immunity.

## SUMMARY OF THE INVENTION

In work leading up to the present invention, the inventors determined that *B. pertussis* contains a gene with amino acid sequence homology to the *aroQ*-encoded 3-dehydroquinase enzyme of *Actinobacillus pleuropneumoniae,* which has been reported previously to have homology with the eukaryotic genes in the quinic acid catabolic pathway of *Aspergillus nidulans.* Even though the sequence of this gene indicated its involvement in the catabolic pathway, the inventors discovered that it rescued an *E. coli aroD* mutant and that it, therefore, was capable of functioning in the aromatic amino acid biosynthetic pathway of *B. pertussis.* Further investigation also confirmed that *B. pertussis* did not contain an *aroD* gene, supporting the concept that there was only one gene encoding a 3-dehydroquinase in *B. pertussis.* It was also discovered that inactivation of the *aroQ* gene is effective in attenuating *B. pertussis* whilst retaining its capacity to stimulate both an antibody immune response and a cell-mediated immune response. This finding is surprising in the light of a previous report showing that deletion of *aroQ* in *Corynebacterium pseudotuberculosis* results in over-attenuation (Simmons *et al.* 1998), thereby making the recombinant strain unsuitable as a vaccine against the disease syndrome caused by the parent strain, caseous lymphadenitis (CLA) in sheep. By contrast, the *aroQ* mutant of *B. pertussis* is not too highly attenuated and hence is more attractive as a vaccine candidate against whooping cough. In accordance with the present invention, it is predicted that the inactivation of *aroQ* in other pathogenic strains of *Bordetella* would also confer the same properties. Thus, the present invention provides for the first time genetically modified *Bordetella* strains of pathogenic origin that have been attenuated by disruption or inactivation of a gene encoding a metabolic protein, particularly a gene encoding a protein necessary for the biosynthesis of aromatic amino acids, and more particularly, the *aroQ* gene that codes for a 3-dehydroquinase enzyme.

Accordingly, in one aspect of the present invention, there is provided a genetically modified *Bordetella* strain having a partial or complete loss of function in the endogenous *aroQ* gene and a lower capacity to propagate in a mammalian host but remaining viable in the host for a period of time sufficient to induce an immune response against a pathogenic *Bordetella* strain, preferably a natural pathogenic *Bordetella* counterpart of the genetically modified *Bordetella* strain, or related organism. In a preferred embodiment, the genetically modified strain comprises a disruption in the endogenous *aroQ* gene. Suitably, the disruption has been introduced into the genome of a pathogenic strain of *Bordetella* by homologous recombination with a DNA targeting construct such that the targeting construct is stably integrated in that genome, wherein the disruption of the *aroQ* gene results in an inability of the genetically modified *Bordetella* strain to produce a functional 3-dehydroquinase or detectable levels of the dehydroquinase. In an alternate embodiment, the genetically modified strain comprises an exogenous nucleic acid sequence in its genome, or on an extrachromosomal element such as a plasmid, which is capable of abolishing or otherwise reducing the expression of *aroQ* or the level and/or functional activity of the 3-dehydroquinase encoded by *aroQ.* Suitably, the nucleic acid sequence comprises at least a portion of *aroQ*, in the sense or anti-sense orientation, which is operably linked to a transcriptional control element. Alternatively, the nucleic acid sequence comprises a ribozyme-encoding polynucleotide that is operably linked to a transcriptional control element, wherein the ribozyme specifically binds to or otherwise interacts with a transcript of the *aroQ* gene.

In a related aspect, the present invention contemplates a genetically modified *Bordetella* strain as broadly described above comprising at least one exogenous gene which is capable of expressing an antigen that is heterologous or foreign to the *Bordetella* strain. This embodiment is particularly useful for the design of immunopotentiating compositions against unrelated pathogens. Accordingly, the heterologous or foreign antigen is preferably derived from a pathogen that is unrelated to the *Bordetella* strain and, in a preferred embodiment, the pathogen is one that infects by the mucosal route.

As will be apparent from the foregoing, the structure and sequence of *aroQ* from *Bordetella pertussis* is useful for the production of the genetically modified attenuated *Bordetella* strains of the present invention. Accordingly, in another aspect of the present invention there is provided an isolated polynucleotide comprising a nucleotide sequence that corresponds or is complementary to at least a portion of the sequence set forth in SEQ ID NO: 1. In a preferred embodiment, the polynucleotide comprises the sequence set forth in SEQ ID NO: 3.

In one embodiment, the nucleotide sequence is a variant having - at least 80%, - more preferably at least 90%, and - even more preferably at least 95%, 96%, 97%, 98% or 99% sequence identity to at least a portion of the sequence set forth in SEQ ID NO: - 3. In another embodiment, the variant is capable of hybridising to at least a portion of the sequence set forth in SEQ ID NO: 1 or 3 under at least low stringency conditiopreferably under at least medium stringency conditions, and more preferably under high stringency conditions.

Suitably, the portion is at least 18 nucleotides, preferably at least 25 nucleotides, more preferably at least 50 nucleotides, even more preferably at least 100 nucleotides, even more preferably at least 150 nucleotides, even more preferably at least 200 nucleotides, even more preferably at least 300 nucleotides, even more preferably at least 400 nucleotides, and still even more preferably at least 500 nucleotides in length. Preferably, the portion is a biologically active fragment of the sequence set forth in SEQ ID NO: 1 or 3.

The 3-dehydroquinase enzyme encoded by the *aroQ* gene of the present invention is useful *inter alia* in the design of immunopotentiating compositions or for the production of antigen-binding molecule that are interactive with that enzyme, which could be utilised in screening for *aroQ*- mutants of *Bordetella* or for detecting or isolating variant 3-dehydroquinase enzymes. Thus, in yet another aspect of the present invention there is provided an isolated polypeptide comprising an amino acid sequence that corresponds to at least a portion of the sequence set forth in SEQ ID NO: 2.

Suitably, the amino acid sequence is a variant having at least 80%, - more preferably at least 90%, and - even more preferably at least 95%, 96%, 97%, 98% or 99% sequence identity to at least a portion of the sequence set forth in SEQ ID NO: 2.

Suitably, the portion is at least 6 amino acids, preferably at least 10 amino acids, more preferably at least 20 amino acids, even more preferably at least 30 amino acids, even more preferably at least 50 amino acids, even more preferably at least 70 amino acids, even more preferably at least 100 amino acids and still even more preferably at least 130 amino acids in length. Preferably the portion is a biologically active fragment of the sequence set forth in SEQ ID NO: 2.

Another aspect of the present invention pertains to a nucleic acid construct for disrupting an *aroQ* gene in a *Bordetella* cell, comprising: a) a non-homologous replacement portion; b) a first homology region located upstream of the non-homologous replacement portion, the first homology region having a nucleotide sequence with substantial identity to a first *aroQ* gene sequence; and c) a second homology region located downstream of the non-homologous replacement portion, the second homology region having a nucleotides sequences with substantial identity to a second *aroQ* gene sequence second *aroQ* gene sequence having a location downstream of the first *aroQ* gene sequence in a naturally occurring endogenous *aroQ* gene of the *Bordetella* cell. Additionally, the first and second homology regions are of sufficient length for homologous recombination to occur between the nucleic acid construct and the endogenous *aroQ* gene when the nucleic acid molecule is introduced into the *Bordetella* cell. In a preferred embodiment, the *aroQ* gene comprises the sequence set forth in SEQ ID NO: 1 or 3 or variant or derivative thereof.

In another aspect, the invention contemplates a vector comprising a nucleotide sequence that corresponds or is complementary to at least a portion of the sequence set forth in SEQ ID NO: 1 or 3, or a nucleic acid construct as broadly described above. In a preferred embodiment, the vector is a DNA targeting vector.

In yet another aspect, the invention envisions a host cell containing a vector as broadly described above.

In another aspect, the invention embraces an antigen-binding molecule that is specifically interactive with the polypeptide, portion, variant or derivative according to the present invention.

The present invention further provides a method for producing a genetically modified *Bordetella* strain, comprising:

- introducing the nucleic acid construct as broadly described above into a *Bordetella* cell under conditions such that the nucleic acid construct is homologously recombined into the *aroQ* gene in the genome of that cell to produce a genetically modified *Bordetella* cell containing a disrupted *aroQ* gene.

In a preferred embodiment, the genetically modified *Bordetella* cell containing the homologously recombined nucleic acid construct is further characterised by expressing reduced or undetectable levels of *aroQ.* In another preferred embodiment, the genetically modified *Bordetella* cell lacks the ability to produce a functional 3-dehydroquinase encoded by said *aroQ* gene.

The genetically modified *Bordetella* strain of the present invention is useful for the design of immunopotentiating compositions that are effective in eliciting an immune response, and preferably a protective immune response, against a pathogenic *Bordetella* strain, preferably a natural pathogenic *Bordetella* counterpart of the genetically modified *Bor-*

*detella* strain, or related organism. Thus, in yet another aspect, the invention contemplates a composition, comprising a genetically modified *Bordetella* strain as broadly described above, together with a pharmaceutically acceptable carrier. The composition may optionally comprise an adjuvant.

In a related aspect, the invention encompasses a composition of matter comprising dendritic cells which have been exposed to a genetically modified *Bordetella* strain as broadly described above for a time and under conditions sufficient to express a processed or modified antigen derived from the *Bordetella* strain for presentation to, and modulation of, T cells. This embodiment is particularly useful for the design of immunopotentiating compositions for eliciting a humoral and a cell mediated immune response. In one embodiment, the composition is in the form of an *in vitro* cell culture.

In a further aspect, the invention encompasses a method for modulating an immune response, which response is preferably against a pathogenic strain of *Bordetella* or related organism, comprising administering to a patient in need of such treatment an effective amount of a genetically modified *Bordetella* strain, or a composition, as broadly described above.

According to still a further aspect of the present invention, there is provided a method for the treatment and/or prophylaxis of whooping cough or related condition, comprising administering to a patient in need of such treatment an effective amount of a genetically modified *Bordetella* strain, or a composition, as broadly described above.

The invention also encompasses the use of a genetically modified *Bordetella* strain as broadly described above in the study, and modulation of an immune response, which is preferably against a pathogenic strain of *Bordetella* or related organism.

**BRIEF DESCRIPTION OF THE DRAWINGS**

Figure 1 is a diagrammatic representation showing a plasmid map of pUSQBord4, which restored *aroD* mutant, *E. coli* 583/90, to wild type.

Figure 2 is a diagrammatic representation showing a Pileup alignment of the *aroQ* gene sequence present in the 1 kb fragment of *B. pertussis* and other published *aroQ* sequences.

Figure 3 is a schematic representation illustrating the construction of shuttle vector pUSQBord10.

Figure 4 is a photographic representation of an agarose gel showing the results of a polymerase chain reaction (PCR) amplification of genomic DNA (gDNA) from a *B. pertussis* mutant that fails to grow in the absence of aromatic amino acid supplements. The results confirm the disruption of the *aroQ* gene by the kanamycin resistance cassette of pUSQBord10. Lanes are as follows: *Lane 1*, *Hin*dIII cut λ Standard (Gibco BRL); *Lane 2,* Blank; *Lane 3, B. pertussis aroQ* mutant gDNA; *Lane 4, B. pertussis* BP304; *Lane 5, E. coli* gDNA; and *Lane 6*, Low DNA Mass Ladder (Gibco BRL).

Figure 5A is a graphical representation showing the persistence of *B. pertussis aroQ* mutant in the lungs of mice at 1, 3, 5, 7, 14 and 21 days post inoculation (no microorganisms were isolated at day 21, resulting in the exclusion of that data point from the graph).

Figure 5B is a graphical representation showing the persistence of *B. pertussis aroQ* mutant in the lungs of mice at 2, 4, 6, 7, 8, 9, 10 , 11 and 12 days post inoculation.

Figure 5C is a graphical representation showing the persistence of *B. pertussis* Tahoma I in the lungs of mice following inoculation.

Figure 6 is a graphical representation showing clearance of infection in lungs of vaccinated and control mice after intranasal (I/N) challenge with a 2.0x $10^8$ CFU dose of virulent *B. pertussis* Tohama I.

Figure 7A is a graphical representation showing production of IL-2 by T-cells in pooled mouse spleen samples stimulated *in vitro* with PTxoid.

Figure 7B is a graphical representation showing production of IL-2 by T-cells in pooled mouse spleen samples stimulated *in vitro* with FHA.

Figure 8A is a graphical representation showing production of IFN-γ by T-cells in pooled mouse supernatants from spleen cells stimulated *in vitro* with PTxoid.

Figure 8B is a graphical representation showing production of IFN-γ by T-cells in pooled mouse supernatants from spleen cells stimulated *in vitro* with FHA.

**BRIEF DESCRIPTION OF THE SEQUENCES: SUMMARY TABLE**

***TABLE A***

| SEQUENCE ID NUMBER | SEQUENCE | LENGTH |
|---|---|---|
| SEQ ID NO: 1 | *B. pertussis aroQ* gene | 942 nts |
| SEQ ID NO: 2 | 3-Dehydroquinase encoded by SEQ ID NO: 1 | 144 aa |
| SEQ ID NO: 3 | *B. pertussis aroQ* coding sequence | 435 nts |
| SEQ ID NO: 4 | FORKAN primer | 30 nts |
| SEQ ID NO: 5 | BACKAN primer | 30 nts |
| SEQ ID NO: 6 | FORQ primer | 20 nts |
| SEQ ID NO: 7 | BACQ2 primer | 20 nts |

**DETAILED DESCRIPTION OF THE INVENTION**

***1. Definitions***

Unless defined otherwise, all technical and scientific terms used herein have the same meaning as commonly understood by those of ordinary skill in the art to which the invention belongs. Although any methods and materials similar or equivalent to those described herein can be used in the practice or testing of the present invention, preferred methods and materials are described. For the purposes of the present invention, the following terms are defined below.

The articles *"a "* and "*an* " are used herein to refer to one or to more than one (i.e. to at least one) of the grammatical object of the article. By way of example, "an element" means one element or more than one element.

The term "*about* " is used herein to refer to the position or location of a subunit in a polymer (*e.g*., the location of a particular nucleotide in a nucleic acid sequence) that varies by as much as 10 subunits, preferably by as much 5 subunits, and more preferably by as much as 2 subunits from a specified position or location. Alternatively, the term "*about*" is used herein to refer to conditions (*e.g*., amounts, concentrations, time etc) that vary by as much as 30%, preferably by as much as 20%, and more preferably by as much as 10% to a specified condition.

"*Amplification product"* refers to a nucleic acid product generated by nucleic acid amplification techniques.

By *"antigen-binding molecule"* is meant a molecule that has binding affinity for a target antigen. It will be understood that this term extends to immunoglobulins, immunoglobulin fragments and non-immunoglobulin derived protein frameworks that exhibit antigen-binding activity.

*"Antigenic or immunogenic activity"* refers to the ability of a polypeptide, fragment, variant or derivative according to the invention to produce an antigenic or immunogenic response in an animal, preferably a mammal, to which it is administered, wherein the response includes the production of elements which specifically bind to the polypeptide or fragment thereof.

By *"biologically active fragment"* is meant a fragment of a full-length parent polypeptide which fragment retains the activity of the parent polypeptide (*e.g*., 3-dehydroquinase activity or antigenic or immunogenic activity). As used herein, the term *"biologically active fragment"* includes deletion variants and small peptides, for example of at least 6, preferably at least 10, more preferably at least 20 and even more preferably at least 30 contiguous amino acids, which comprise the above activity. Peptides of this type may be obtained through the application of standard recombinant nucleic acid techniques or synthesised using conventional liquid or solid phase synthesis techniques. For example, reference may be made to solution synthesis or solid phase synthesis as described, for example, in Chapter 9 entitled "Peptide Synthesis" by Atherton and Shephard which is included in a publication entitled *"*Synthetic Vaccines " edited by Nicholson and published by Blackwell Scientific Publications. Alternatively, peptides can be produced by digestion of a polypeptide of the invention with proteinases such as endoLys-C, endoArg-C, endoGlu-C and staphylococcus V8-protease. The digested fragments can be purified by, for example, high performance liquid chromatographic (HPLC) techniques.

The term *"complementary"* refers to the topological capability or matching together of interacting surfaces of a test polynucleotide and its target oligonucleotide, which may be part of a larger polynucleotide. Thus, the test and target polynucleotides can be described as complementary, and furthermore, the contact surface characteristics are complementary to each other. Complementary includes base complementarity such as A is complementary to T or U, and C is complementary to G in the genetic code. However, this invention also encompasses situations in which there is non-traditional base-pairing such as Hoogsteen base pairing which has been identified in certain transfer RNA molecules and postulated to exist in a triple helix. In the context of the definition of the term "complementary", the terms "match" and "mismatch" as used herein refer to the hybridisation potential of paired nucleotides in complementary nucleic acid strands. Matched nucleotides hybridise efficiently, such as the classical A-T and G-C base pair mentioned above. Mismatches are other combinations of nucleotides that hybridise less efficiently.

Throughout this specification, unless the context requires otherwise, the words *"comprise "*, *"comprises"* and *"comprising"* will be understood to imply the inclusion of a stated step or element or group of steps or elements but not the exclusion of any other step or element or group of steps or elements.

By "*corresponds to"* or *"corresponding to"* is meant (a) a polynucleotide having a nucleotide sequence that is substantially identical or complementary to all or a portion of a reference polynucleotide sequence or encoding an amino acid sequence identical to an amino acid sequence in a peptide or protein; or (b) a peptide or polypeptide having an amino acid sequence that is substantially identical to a sequence of amino acids in a reference peptide or protein.

By *"derivative"* is meant a polypeptide that has been derived from the basic sequence by modification, for example by conjugation or complexing with other chemical moieties or by post-translational modification techniques as would be understood in the art. The term *"derivative"* also includes within its scope alterations that have been made to a parent sequence including additions or deletions that provide for functional equivalent molecules.

The term *"foreign"* or *"exogenous"* or *"heterologous"* refers to any molecule (*e.g.*, a polynucleotide or polypeptide) which is introduced into a host by experimental manipulations and may include gene sequences found in that host so long as the introduced gene contains some modification (*e.g.*, a point mutation, the presence of a selectable marker gene, the presence of a recombination site, etc.) relative to the naturally-occurring gene.

The term "*gene* " as used herein refers to any and all discrete coding regions of the cell's genome, as well as associated non-coding and regulatory regions. Thus, the term "*aroQ gene*" is used generically herein to designate *aroQ* genes, *e.g.* variants from different *Bordetella* species. The gene is also intended to mean the open reading frame encoding specific polypeptides and adjacent 5' and 3' non-coding nucleotide sequences involved in the regulation of expression, up to about 1 kb beyond the coding region, but possibly further in either direction. In this regard, the gene may further comprise endogenous (*i.e.*, naturally associated with a given gene) or heterologous control signals such as promoters, enhancers, translational control elements such a Shine-Dalgamo sequence, initiation codon, termination codons and/or transcriptional termination signals. The gene may be introduced into an appropriate vector for extrachromosomal maintenance or for integration into the host.

By *"effective amount"*, in the context of modulating an activity or of treating or preventing a condition is meant the administration of that amount of active to an individual in need of such modulation, treatment or prophylaxis, either in a single dose or as part of a series, that is effective for modulation of that effect or for treatment or prophylaxis of that condition or for ameliorating the symptoms associated with that condition. The effective amount will vary depending upon the health and physical condition of the individual to be treated, the taxonomic group of individual to be treated, the formulation of the composition, the assessment of the medical situation, and other relevant factors. It is expected that the amount will fall in a relatively broad range that can be determined through routine trials.

"*Homology*" refers to the percentage number of amino acids that are identical or constitute conservative substitutions as defined in Table B below. Homology may be determined using sequence comparison programs such as GAP (Deveraux et al. 1984, Nucleic Acids Research 12, 387-395). In this way sequences of a similar or substantially different length to those cited herein could be compared by insertion of gaps into the alignment, such gaps being determined, for example, by the comparison algorithm used by GAP.

*"Hybridisation"* is used herein to denote the pairing of complementary nucleotide sequences to produce a DNA-DNA hybrid or a DNA-RNA hybrid. Complementary base sequences are those sequences that are related by the base-pairing rules. In DNA, A pairs with T and C pairs with G. In RNA U pairs with A and C pairs with G. In this regard, the terms "match" and "mismatch" as used herein refer to the hybridisation potential of paired nucleotides in complementary nucleic acid strands. Matched nucleotides hybridise efficiently, such as the classical A-T and G-C base pair mentioned above. Mismatches are other combinations of nucleotides that do not hybridise efficiently.

Reference herein to "*interactive*" includes reference to any interaction, reaction, or other form of association between molecules and in particular where one of the molecules is, or mimics, a component of the immune system.

By "*isolated"* is meant material that is substantially or essentially free from components that normally accompany it in its native state.

By *"modulating"* is meant increasing or decreasing, either directly or indirectly, the level and/or functional activity of a target molecule. For example, an agent may indirectly modulate the said level/activity by interacting with a molecule other than the target molecule. In this regard, indirect modulation of a gene encoding a target polypeptide includes within its scope modulation of the expression of a first nucleic acid molecule, wherein an expression product of the first nucleic acid molecule modulates the expression of a nucleic acid molecule encoding the target polypeptide.

By *"obtained from "* is meant that a sample such as, for example, a polynucleotide extract or polypeptide extract is isolated from, or derived from, a particular source of the host. For example, the extract can be obtained from a cell, tissue or a biological fluid isolated directly from the host.

The term *"oligonucleotide"* as used herein refers to a polymer composed of a multiplicity of nucleotide residues (deoxyribonucleotides or ribonucleotides, or related structural variants or synthetic analogues thereof) linked via phosphodiester bonds (or related structural variants or synthetic analogues thereof). Thus, while the term "oligonucleotide" typically refers to a nucleotide polymer in which the nucleotide residues and linkages between them are naturally occurring, it will be understood that the term also includes within its scope various analogues including, but not restricted to, peptide nucleic acids (PNAs), phosphoramidates, phosphorothioates, methyl phosphonates, 2-O-methyl ribonucleic acids, and the like. The exact size of the molecule can vary depending on the particular application. An oligonucleotide is typically rather short in length, generally from about 10 to 30 nucleotide residues, but the term can refer to molecules of any length, although the term "polynucleotide" or "nucleic acid" is typically used for large oligonucleotides.

By *"operably linked"* is meant that transcriptional and translational regulatory polynucleotides are positioned relative to a polypeptide-encoding polynucleotide in such a manner that the polynucleotide is transcribed and the polypeptide is translated.

The term "*patient*" refers to patients of human or other mammal and includes any individual it is desired to examine or treat using the methods of the invention. However, it will be understood that "*patient*" does not imply that symptoms are present. Suitable mammals that fall within the scope of the invention include, but are not restricted to, primates, livestock animals (*e.g*., sheep, cows, horses, donkeys, pigs), laboratory test animals (*e.g*., rabbits, mice, rats, guinea pigs, hamsters), companion animals *(e.g.,* cats, dogs) and captive wild animals (*e.g*., foxes, deer, dingoes).

By "*pharmaceutically acceptable carrier*" is meant a solid or liquid filler, diluent or encapsulating substance that can be safely used in topical or systemic administration to a mammal.

The term *"polynucleotide"* or *"nucleic acid"* as used herein designates mRNA, RNA, cRNA, cDNA or DNA. The term typically refers to oligonucleotides greater than 30 nucleotide residues in length.

The terms *"polynucleotide variant* " and "*variant*" refer to polynucleotides displaying substantial sequence identity with a reference polynucleotide sequence or polynucleotides that hybridise with a reference sequence under stringent conditions that are defined hereinafter. These terms also encompasses polynucleotides which differ from a reference polynucleotide by the addition, deletion or substitution of at least one nucleotide. In this regard, it is well understood in the art that certain alterations inclusive of mutations, additions, deletions and substitutions can be made to a reference polynucleotide whereby the altered polynucleotide retains the biological function or activity of the reference polynucleotide. The terms *"polynucleotide variant"* and *"variant"* also include naturally occurring allelic variants.

*"Polypeptide", "peptide"* and *"protein"* are used interchangeably herein to refer to a polymer of amino acid residues and to variants and synthetic analogues of the same. Thus, these terms apply to amino acid polymers in which one or more amino acid residues is a synthetic non-naturally occurring amino acid, such as a chemical analogue of a corresponding naturally occurring amino acid, as well as to naturally-occurring amino acid polymers.

The term *"polypeptide variant"* refers to polypeptides which differ from a reference polypeptide by the addition, deletion or substitution of at least one amino acid. It is well understood in the art for example that some amino acids may be changed to others with broadly similar properties without changing the nature of the activity of the polypeptide (conservative substitutions) as described hereinafter.

By *"primer"* is meant an oligonucleotide which, when paired with a strand of DNA, is capable of initiating the synthesis of a primer extension product in the presence of a suitable polymerising agent. The primer is preferably single-stranded for maximum efficiency in amplification but can alternatively be double-stranded. A primer must be sufficiently long to prime the synthesis of extension products in the presence of the polymerisation agent. The length of the primer depends on many factors, including application, temperature to be employed, template reaction conditions, other reagents, and source of primers. For example, depending on the complexity of the target sequence, the oligonucleotide primer typically contains 15 to 35 or more nucleotide residues, although it can contain fewer nucleotide residues. Primers can be large polynucleotides, such as from about 200 nucleotide residues to several kilobases or more. Primers can be selected to be "substantially complementary" to the sequence on the template to which it is designed to hybridise and serve as a site for the initiation of synthesis. By "substantially complementary", it is meant that the primer is sufficiently complementary to hybridise with a target polynucleotide. Preferably, the primer contains no mismatches with the template to which it is designed to hybridise but this is not essential. For example, non-complementary nucleotide residues can be attached to the 5' end of the primer, with the remainder of the primer sequence being complementary to the template. Alternatively, non-complementary nucleotide residues or a stretch of non-complementary nucleotide residues can be interspersed into a primer, provided that the primer sequence has sufficient complementarity with the sequence of the template to hybridise therewith and thereby form a template for synthesis of the extension product of the primer.

*"Probe* " refers to a molecule that binds to a specific sequence or sub-sequence or other moiety of another molecule. Unless otherwise indicated, the term "probe" typically refers to a polynucleotide probe that binds to another polynucleotide, often called the "target polynucleotide", through complementary base pairing. Probes can bind target polynucleotides lacking complete sequence complementarity with the probe, depending on the stringency of the hybridisation conditions. Probes can be labelled directly or indirectly.

The term *"recombinant polynucleotide"* as used herein refers to a polynucleotide formed *in vitro* by the manipulation of a polynucleotide into a form not normally found in nature. For example, the recombinant polynucleotide can be in the form of an expression vector. Generally, such expression vectors include transcriptional and translational regulatory polynucleotide operably linked to the polynucleotide.

By *"recombinant polypeptide"* is meant a polypeptide made using recombinant techniques, i.e., through the expression of a recombinant or synthetic polynucleotide.

By *"reporter molecule"* as used in the present specification is meant a molecule that, by its chemical nature, provides an analytically identifiable signal that allows the detection of a complex comprising an antigen-binding molecule and its target antigen. The term "reporter molecule" also extends to use of cell agglutination or inhibition of agglutination such as red blood cells on latex beads, and the like.

Terms used to describe sequence relationships between two or more polynucleotides or polypeptides include "reference sequence", "comparison window", "sequence identity", "percentage of sequence identity" and "substantial identity". A *"reference sequence"* is at least 12 but frequently 15 to 18 and often at least 25 monomer units, inclusive of nucleotides and amino acid residues, in length. Because two polynucleotides may each comprise (1) a sequence (*i.e.*, only a portion of the complete polynucleotide sequence) that is similar between the two polynucleotides, and (2) a sequence that is divergent between the two polynucleotides, sequence comparisons between two (or more) polynucleotides are typically performed by comparing sequences of the two polynucleotides over a "comparison window" to identify and compare local regions of sequence similarity. A *"comparison window"* refers to a conceptual segment of at least 50 contiguous positions, usually about 50 to about 100, more usually about 100 to about 150 in which a sequence is compared to a reference sequence of the same number of contiguous positions after the two sequences are optimally aligned. The comparison window may comprise additions or deletions (*i.e.*, gaps) of about 20% or less as compared to the reference sequence (which does not comprise additions or deletions) for optimal alignment of the two sequences. Optimal alignment of sequences for aligning a comparison window may be conducted by computerised implementations of algorithms (GAP, BESTFIT, FASTA, and TFASTA in the Wisconsin Genetics Software Package Release 7.0, Genetics Computer Group, 575 Science Drive Madison, WI, USA) or by inspection and the best alignment (*i.e.*, resulting in the highest percentage homology over the comparison window) generated by any of the various methods selected. Reference also may be made to the BLAST family of programs as for example disclosed by Altschul et al., 1997, Nucl. Acids Res. 25:3389. A detailed discussion of sequence analysis can be found in Unit 19.3 of Ausubel et al., "Current Protocols in Molecular Biology", John Wiley & Sons Inc, 1994-1998, Chapter 15.

The term *"sequence identity"* as used herein refers to the extent that sequences are identical on a nucleotide-by-nucleotide basis or an amino acid-by-amino acid basis over a window of comparison. Thus, a *"percentage of sequence identity"* is calculated by comparing two optimally aligned sequences over the window of comparison, determining the number of positions at which the identical nucleic acid base (*e.g.*, A, T, C, G, I) or the identical amino acid residue (*e.g.*, Ala, Pro, Ser, Thr, Gly, Val, Leu, Ile, Phe, Tyr, Trp, Lys, Arg, His, Asp, Glu, Asn, Gln, Cys and Met) occurs in both sequences to yield the number of matched positions, dividing the number of matched positions by the total number of positions in the window of comparison (*i.e.*, the window size), and multiplying the result by 100 to yield the percentage of sequence identity. For the purposes of the present invention, *"sequence identity"* will be understood to mean the *"match percentage"* calculated by the DNASIS computer program (Version 2.5 for windows; available from Hitachi Software engineering Co., Ltd., South San Francisco, California, USA) using standard defaults as used in the reference manual accompanying the software.

*"Stringency"* as used herein, refers to the temperature and ionic strength conditions, and presence or absence of certain organic solvents, during hybridisation and washing procedures. The higher the stringency, the higher will be the degree of complementarity between immobilised target nucleotide sequences and the labelled probe polynucleotide sequences that remain hybridised to the target after washing.

*"Stringent conditions"* refers to temperature and ionic conditions under which only nucleotide sequences having a high frequency of complementary bases will hybridise. The stringency required is nucleotide sequence dependent and depends upon the various components present during hybridisation and subsequent washes, and the time allowed for these processes. Generally, in order to maximise the hybridisation rate, non-stringent hybridisation conditions are selected; about 20 to 25° C lower than the thermal melting point ($T_m$). The $T_m$ is the temperature at which 50% of specific target sequence hybridises to a perfectly complementary probe in solution at a defined ionic strength and pH. Generally, in order to require at least about 85% nucleotide complementarity of hybridised sequences, highly stringent washing conditions are selected to be about 5 to 15° C lower than the $T_m$. In order to require at least about 70% nucleotide complementarity of hybridised sequences, moderately stringent washing conditions are selected to be about 15 to 30° C lower than the $T_m$. Highly permissive (low stringency) washing conditions may be as low as 50° C below the $T_m$, allowing a high level of mis-matching between hybridised sequences. Those skilled in the art will recognise that other physical and chemical parameters in the hybridisation and wash stages can also be altered to affect the outcome of a detectable hybridisation signal from a specific level of homology between target and probe sequences.

The term *"transgene"* is used herein to describe genetic material that has been or is about to be artificially inserted into the genome or onto an extrachromosomal element of a cell, particularly a bacterial cell and more particularly a *Bordetella* cell or related cell thereof. The transgene is used to transform a cell, meaning that a permanent or transient genetic change, preferably a permanent genetic change, is induced in a cell following incorporation of exogenous DNA. A permanent genetic change is generally achieved by introduction of the DNA into the genome of the cell. Vectors for stable integration include plasmids, bacteriophages and other bacterial viruses and the like.

By *"vector"* is meant a polynucleotide molecule, preferably a DNA molecule derived, for example, from a plasmid, bacteriophage, yeast or virus, into which a polynucleotide can be inserted or cloned. A vector preferably contains one or more unique restriction sites and can be capable of autonomous replication in a defined host cell including a target cell or tissue or a progenitor cell or tissue thereof, or be integrable with the genome of the defined host such that the cloned sequence is reproducible. Accordingly, the vector can be an autonomously replicating vector, *i.e.*, a vector that exists as an extrachromosomal entity, the replication of which is independent of chromosomal replication, *e.g.*, a linear or closed circular plasmid, an extrachromosomal element, a minichromosome, or an artificial chromosome. The vector can contain any means for assuring self-replication. Alternatively, the vector can be one which, when introduced into the host cell, is integrated into the genome and replicated together with the chromosome(s) into which it has been integrated. A vector system can comprise a single vector or plasmid, two or more vectors or plasmids, which together contain the total DNA to be introduced into the genome of the host cell, or a transposon. The choice of the vector will typically depend on the compatibility of the vector with the host cell into which the vector is to be introduced. In the present case, the vector is preferably a bacterial or bacteriophage vector, which is operably functional in a *Bordetella* strain of interest, or related organism. The vector can also include a selection marker such as an antibiotic resistance gene that can be used for selection of suitable transformants. Examples of such resistance genes are known to those of skill in the art and include the *nptII* gene that confers resistance to the antibiotics kanamycin and G418 (Geneticin®) and the *cat* gene which confers resistance to the antibiotic chloramphenicol.

The terms *"wild-type* " and *"normal"* are used interchangeably to refer to the phenotype that is characteristic of most of the members of the species occurring naturally and contrast for example with the phenotype of a mutant.

As used herein, underscoring or italicising the name of a gene shall indicate the gene, in contrast to its protein product, which is indicated by the name of the gene in the absence of any underscoring or italicising. For example, *"aroQ*" shall mean the *aro*Q gene, whereas "AroQ" shall indicate the protein product of the *"aroQ"* gene. The terms *"AroQ"* and *"3-dehydroquinase"* are used interchangeably herein.

### *2. Polynucleotides of the invention*

#### *2.1 Bordetella aroQ* polynucleotides

The present invention is predicated in part on the determination of the full-length sequence of the *aroQ* gene from *B. pertussis,* which is useful *inter alia* for the production of genetically modified *Bordetella* strains of pathogenic origin that are attenuated by disruption or inactivation of their corresponding *aroQ* gene. The invention, therefore, provides in one aspect an isolated polynucleotide comprising at least a portion of an *aroQ* gene from a *Bordetella* species or related organism. Suitably, the polynucleotide comprises the entire sequence of nucleotides set forth in SEQ ID NO: 1. SEQ ID NO: 1 corresponds to a 942 bp genomic sequence for *aroQ* of *B. pertussis.* This sequence defines: promoter elements comprising (i) a -35 region from about nucleotide 19 to about nucleotide 24 and (ii) a -10 region from about nucleotide 37 to about nucleotide 42; a ribosome binding site from about nucleotide 52 to about nucleotide 60; and a coding sequence from about nucleotide 73 to about nucleotide 504 as set forth in SEQ ID NO: 3.

A Bestfit comparison, with standard defaults, of the *aroQ* gene from *B. pertussis* and from other microorganisms reveals that the *B. pertussis aroQ* gene displays 68.9%, 60.6%, 57.0% and 59.9% to the *Haemophilus influenzae, Actinobacillus pleuropneumoniae, Helicobacter pylori* and *Aspergillus nidulans* counterpart *aroQ* genes, respectively.

#### *2.2 Variant aroq* polynucleotides

The present invention is also directed to variants of the *aroQ* gene of the present invention. In general, polynucleotide variants according to the invention comprise regions that show at least 80%, more preferably at least 90% and still even more preferably at least 95%, 96%, 97%, 98% or 99% sequence identity over a reference polynucleotide sequence of identical size (*"comparison window"*) or when compared to an aligned sequence in which the alignment is performed by a computer homology program known in the art. In one embodiment, the reference polynucleotide is selected from the sequence set forth in SEQ ID NO: 1 or 3.

The *aroQ* gene sequence, including flanking promoter regions and coding regions, may be modified or mutated in various ways known in the art to generate targeted changes in promoter strength, sequence of the encoded protein, etc. The sequence changes may be substitutions, insertions or deletions. Deletions may include large changes, such as deletions of a domain-encoding region. Other modifications of interest include epitope tagging, *e.g.* with the FLAG system, HA, etc. For studies of subcellular localisation, fusion proteins with green fluorescent proteins (GFP) may be used. Such mutated genes may be used to study structure-function relationships of AroQ polypeptides, or to alter properties of the proteins that affect their function or regulation.

For example, a polynucleotide according to SEQ ID NO: 1 or 3 can be mutated using random mutagenesis (*e.g.*, transposon mutagenesis), oligonucleotide-mediated (or site-directed) mutagenesis, PCR mutagenesis and cassette mutagenesis of an earlier prepared variant or non-variant version of an isolated natural promoter according to the invention.

Oligonucleotide-mediated mutagenesis is a preferred method for preparing nucleotide substitution variants of a polynucleotide of the invention. This technique is well known in the art as, for example, described by Adelman et al. (1983, DNA 2:183). Briefly, a polynucleotide according to SEQ ID NO: 1 or 3 is altered by hybridising an oligonucleotide encoding the desired mutation to a template DNA, wherein the template is the single-stranded form of a plasmid or bacteriophage containing the unaltered or parent DNA sequence. After hybridisation, a DNA polymerase is used to synthesise an entire second complementary strand of the template that will thus incorporate the oligonucleotide primer, and will code for the selected alteration in said parent DNA sequence.

Generally, oligonucleotides of at least 25 nucleotides in length are used. An optimal oligonucleotide will have 12 to 15 nucleotides that are completely complementary to the template on either side of the nucleotide(s) coding for the mutation. This ensures that the oligonucleotide will hybridise properly to the single-stranded DNA template molecule.

The DNA template can be generated by those vectors that are either derived from bacteriophage M13 vectors, or those vectors that contain a single-stranded phage origin of replication as described by Viera et al. (1987, Methods Enzymol. 153:3). Thus, the DNA that is to be mutated may be inserted into one of the vectors to generate single-stranded template. Production of single-stranded template is described, for example, in Sections 4.21-4.41 *of Sambrook et al.* (1989, *supra).*

Alternatively, the single-stranded template may be generated by denaturing double-stranded plasmid (or other DNA) using standard techniques.

For alteration of the native DNA sequence, the oligonucleotide is hybridised to the single-stranded template under suitable hybridisation conditions. A DNA polymerising enzyme, usually the Klenow fragment of DNA polymerase I, is then added to synthesise the complementary strand of the template using the oligonucleotide as a primer for synthesis. A heteroduplex molecule is thus formed such that one strand of DNA encodes the mutated form of the polypeptide or fragment under test, and the other strand (the original template) encodes the native unaltered sequence of the polypeptide or fragment under test. This heteroduplex molecule is then transformed into a suitable host cell, usually a prokaryote such as *E. coli.* After the cells are grown, they are plated onto agarose plates and screened using the oligonucleotide primer having a detectable label to identify the bacterial colonies having the mutated DNA. The resultant mutated DNA fragments are then cloned into suitable expression hosts such as *E. coli* using conventional technology and clones that retain the desired antigenic activity are detected. Where the clones have been derived using random mutagenesis techniques, positive clones would have to be sequenced in order to detect the mutation.

Alternatively, linker-scanning mutagenesis of DNA may be used to introduce clusters of point mutations throughout a sequence of interest that has been cloned into a plasmid vector. For example, reference may be made to Ausubel *et al., supra,* (in particular, Chapter 8.4) which describes a first protocol that uses complementary oligonucleotides and requires a unique restriction site adjacent to the region that is to be mutagenised. A nested series of deletion mutations is first generated in the region. A pair of complementary oligonucleotides is synthesised to fill in the gap in the sequence of interest between the linker at the deletion endpoint and the nearby restriction site. The linker sequence actually provides the desired clusters of point mutations as it is moved or "scanned" across the region by its position at the varied endpoints of the deletion mutation series. An alternate protocol is also described by Ausubel *et al*., *supra,* which makes use of site directed mutagenesis procedures to introduce small clusters of point mutations throughout the target region. Briefly, mutations are introduced into a sequence by annealing a synthetic oligonucleotide containing one or more mismatches to the sequence of interest cloned into a single-stranded M13 vector. This template is grown in an *E. coli* *dut*$^-$ *ung*$^-$ strain, which allows the incorporation of uracil into the template strand. The oligonucleotide is annealed to the template and extended with T4 DNA polymerase to create a double-stranded heteroduplex. Finally, the heteroduplex is introduced into a wild-type *E. coli* strain, which will prevent replication of the template strand due to the presence of apurinic sites (generated where uracil is incorporated), thereby resulting in plaques containing only mutated DNA.

Region-specific mutagenesis and directed mutagenesis using PCR may also be employed to construct polynucleotide variants according to the invention. In this regard, reference may be made, for example, to Ausubel *et al*., *supra*, in particular Chapters 8.2A and 8.5.

Alternatively, suitable polynucleotide sequence variants of the invention may be prepared according to the following procedure:

(a) creating primers which are optionally degenerate wherein each comprises a portion of a reference polynucleotide including, but not restricted to, a *aroQ* gene sequence as for example set forth in SEQ ID NO: 1 or 3, or a sequence encoding a reference polypeptide or fragment of the invention, or a complement of said sequence, said polypeptide preferably encoding the sequence set forth in any one of SEQ ID NO: 2;

(b) obtaining a nucleic acid extract from an organism, which is preferably a microorganism, and more preferably a bacterium including, but not restricted to, bacteria from the genera *Pasteurella, Actinobacillus, Haemophilus, Helicobacter, Aspergillus* and *Pseudomonus,* and still even more preferably a bacterium belonging to the genus *Bor-*

*detella,* or related organism; and

(c) using said primers to amplify, *via* nucleic acid amplification techniques, at least one amplification product from said nucleic acid extract, wherein said amplification product corresponds to a polynucleotide variant.

Suitable nucleic acid amplification techniques are well known to the skilled addressee, and include polymerase chain reaction (PCR) as for example described in Ausubel *et al.* (*supra*); strand displacement amplification (SDA) as for example described in U.S. Patent No 5,422,252; rolling circle replication (RCR) as for example described in Liu et al., (1996, J. Am. Chem. Soc. 118:1587-1594 and International application WO 92/01813) and Lizardi et al., (International Application WO 97/19193); nucleic acid sequence-based amplification (NASBA) as for example described by Sooknanan et al., (1994, Biotechniques 17:1077-1080); and Q-β replicase amplification as for example described by Tyagi et al., (1996, Proc. Natl. Acad. Sci. USA 93: 5395-5400).

Typically, polynucleotide variants that are substantially complementary to a reference polynucleotide are identified by blotting techniques that include a step whereby nucleic acids are immobilised on a matrix (preferably a synthetic membrane such as nitrocellulose), followed by a hybridisation step, and a detection step. Southern blotting is used to identify a complementary DNA sequence; northern blotting is used to identify a complementary RNA sequence. Dot blotting and slot blotting can be used to identify complementary DNA/DNA, DNA/RNA or RNA/RNA polynucleotide sequences. Such techniques are well known by those skilled in the art, and have been described in Ausubel *et al.* (1994-1998, *supra*) at pages 2.9.1 through 2.9.20.

According to such methods, Southern blotting involves separating DNA molecules according to size by gel electrophoresis, transferring the size-separated DNA to a synthetic membrane, and hybridising the membrane-bound DNA to a complementary nucleotide sequence labelled radioactively, enzymatically or fluorochromatically. In dot blotting and slot blotting, DNA samples are directly applied to a synthetic membrane prior to hybridisation as above.

An alternative blotting step is used when identifying complementary polynucleotides in a cDNA or genomic DNA library, such as through the process of plaque or colony hybridisation. A typical example of this procedure is described in Sambrook et al. ("Molecular Cloning. A Laboratory Manual", Cold Spring Harbour Press, 1989) Chapters 8-12.

Typically, the following general procedure can be used to determine hybridisation conditions. Polynucleotides are blotted/transferred to a synthetic membrane, as described above. A reference polynucleotide such as a polynucleotide of the invention is labelled as described above, and the ability of this labelled polynucleotide to hybridise with an immobilised polynucleotide is analysed.

A skilled artisan will recognise that a number of factors influence hybridisation. The specific activity of radioactively labelled polynucleotide sequence should typically be greater than or equal to about $10^8$ dpm/mg to provide a detectable signal. A radiolabelled nucleotide sequence of specific activity $10^8$ to $10^9$ dpm/mg can detect approximately 0.5 pg of DNA. It is well known in the art that sufficient DNA must be immobilised on the membrane to permit detection. It is desirable to have excess immobilised DNA, usually 10 μg. Adding an inert polymer such as 10% (w/v) dextran sulfate (MW 500,000) or polyethylene glycol 6000 during hybridisation can also increase the sensitivity of hybridisation (see Ausubel *supra* at 2.10.10).

To achieve meaningful results from hybridisation between a polynucleotide immobilised on a membrane and a labelled polynucleotide, a sufficient amount of the labelled polynucleotide must be hybridised to the immobilised polynucleotide following washing. Washing ensures that the labelled polynucleotide is hybridised only to the immobilised polynucleotide with a desired degree of complementarity to the labelled polynucleotide.

It will be understood that polynucleotide variants according to the invention will hybridise to a reference polynucleotide under at least low stringency conditions. Reference herein to low stringency conditions includes and encompasses from at least about 1% v/v to at least about 15% v/v formamide and from at least about 1 M to at least about 2 M salt for hybridisation at 42° C, and at least about 1 M to at least about 2 M salt for washing at 42° C. Low stringency conditions also may include 1% Bovine Serum Albumin (BSA), 1 mM EDTA, 0.5 M $NaHPO_4$ (pH 7.2), 7% SDS for hybridisation at 65° C, and (i) 2xSSC, 0.1% SDS; or (ii) 0.5% BSA, 1 mM EDTA, 40 mM $NaHPO_4$ (pH 7.2), 5% SDS for washing at room temperature.

Suitably, the polynucleotide variants hybridise to a reference polynucleotide under at least medium stringency conditions. Medium stringency conditions include and encompass from at least about 16% v/v to at least about 30% v/v formamide and from at least about 0.5 M to at least about 0.9 M salt for hybridisation at 42° C, and at least about 0.1 M to at least about 0.2 M salt for washing at 55° C. Medium stringency conditions also may include 1% Bovine Serum Albumin (BSA), 1 mM EDTA, 0.5 M $NaHPO_4$ (pH 7.2), 7% SDS for hybridisation at 65° C, and (i) 2 x SSC, 0.1% SDS; or (ii) 0.5% BSA, 1 mM EDTA, 40 mM $NaHPO_4$ (pH 7.2), 5% SDS for washing at 60-65° C.

Preferably, the polynucleotide variants hybridise to a reference polynucleotide under high stringency conditions. High stringency conditions include and encompass from at least about 31% v/v to at least about 50% v/v formamide and from about 0.01 M to about 0.15 M salt for hybridisation at 42° C, and about 0.01 M to about 0.02 M salt for washing at 55° C. High stringency conditions also may include 1% BSA, 1 mM EDTA, 0.5 M $NaHPO_4$ (pH 7.2), 7% SDS for hybridisation at 65° C, and (i) 0.2 x SSC, 0.1% SDS; or (ii) 0.5% BSA, 1mM EDTA, 40 mM $NaHPO_4$ (pH 7.2), 1% SDS for washing at a temperature in excess of 65° C.

Other stringent conditions are well known in the art. A skilled addressee will recognise that various factors can be manipulated to optimise the specificity of the hybridisation. Optimisation of the stringency of the final washes can serve to ensure a high degree of hybridisation. For detailed examples, see Ausubel *et al*., *supra* at pages 2.10.1 to 2.10.16 and Sambrook *et al.* (1989, *supra*) at sections 1.101 to 1.104.

While stringent washes are typically carried out at temperatures from about 42° C to 68° C, one skilled in the art will appreciate that other temperatures may be suitable for stringent conditions. Maximum hybridisation rate typically occurs at about 20° C to 25° C below the $T_m$ for formation of a DNA-DNA hybrid. It is well known in the art that the $T_m$ is the melting temperature, or temperature at which two complementary polynucleotide sequences dissociate. Methods for estimating $T_m$ are well known in the art (see Ausubel *et al*., *supra* at page 2.10.8).

In general, the $T_m$ of a perfectly matched duplex of DNA may be predicted as an approximation by the formula:

$$T_m = 81.5 + 16.6 (\log_{10} M) + 0.41 (\%G{+}C) - 0.63 (\% \text{ formamide}) - (600/\text{length})$$

wherein: M is the concentration of $Na^+$, preferably in the range of 0.01 molar to 0.4 molar; %G+C is the sum of guanosine and cytosine bases as a percentage of the total number of bases, within the range between 30% and 75% G+C; % formamide is the percent formamide concentration by volume; length is the number of base pairs in the DNA duplex.

The $T_m$ of a duplex DNA decreases by approximately 1° C with every increase of 1% in the number of randomly mismatched base pairs. Washing is generally carried out at $T_m$ -15° C for high stringency, or $T_m$ - 30° C for moderate stringency.

In a preferred hybridisation procedure, a membrane (*e.g*., a nitrocellulose membrane or a nylon membrane) containing immobilised DNA is hybridised overnight at 42° C in a hybridisation buffer (50% deionised formamide, 5xSSC, 5x Denhardt's solution (0.1% ficoll, 0.1% polyvinylpyrollidone and 0.1% bovine serum albumin), 0.1% SDS and 200 mg/mL denatured salmon sperm DNA) containing labelled probe. The membrane is then subjected to two sequential medium stringency washes (*i.e*., 2xSSC, 0.1% SDS for 15 min at 45° C, followed by 2xSSC, 0.1% SDS for 15 min at 50° C), followed by two sequential higher stringency washes (*i.e*., 0.2xSSC, 0.1% SDS for 12 min at 55° C followed by 0.2xSSC and 0.1%SDS solution for 12 min at 65-68° C).

Methods for detecting a labelled polynucleotide hybridised to an immobilised polynucleotide are well known to practitioners in the art. Such methods include autoradiography, phosphorimaging, and chemiluminescent, fluorescent and colorimetric detection.

For the most part, DNA fragments will be of at least 15 nucleotides, usually at least 18 nucleotides, more usually at least about 50 nucleotides. Such small DNA fragments are useful as primers for PCR, hybridisation screening, etc. Larger DNA fragments, *i.e.* greater than 100 nucleotides are useful for production of the encoded polypeptide. For use in amplification reactions, such as PCR, a pair of primers will be used.

### *3. Genetically modified Bordetella strains*

The subject genetically modified *Bordetella* strains of pathogenic origin typically, but not exclusively, comprise a foreign or exogenous polynucleotide sequence or transgene present as an extrachromosomal element or integrated in the bacterial chromosome. The *Bordetella* strains are preferably, but not exclusively, selected from *Bordetella avium*, *Bordetella bronchiseptica, Bordetella holmesii, Bordetella parapertussis* and *Bordetella pertussis.* The genetically modified *Bordetella* strains that may be constructed in accordance with the present invention generally fall into two groups, colloquially termed "knockouts" and "knockins". In the present invention, knockouts have a partial or complete loss of function in the endogenous *aroQ* gene. Knockins have an introduced transgene (*i.e.*, foreign gene) with altered genetic sequence and function from the endogenous gene. Increased (including ectopic) or decreased expression may be achieved by introduction of an additional copy of the target gene, or by operatively inserting a regulatory sequence that provides for enhanced expression of an endogenous copy of the target gene. These changes may be constitutive or conditional, *i.e.* dependent on the presence of an activator or repressor. The foreign gene is usually either from a different species than the host, or is otherwise altered in its coding or non-coding sequence. The introduced gene may be a wild-type gene, naturally occurring polymorphism, or a genetically manipulated sequence, for example having deletions, substitutions or insertions in the coding or non-coding regions. The introduced sequence may encode an AroQ polypeptide, or may utilise the *aroQ* promoter operably linked to a reporter gene. Where the introduced gene is a coding sequence, it is usually operably linked to a promoter, which may be constitutive or inducible, and other regulatory sequences required for expression in the host bacterium. A knockin and a knockout may be combined, such that the naturally occurring gene is disabled, and an altered form introduced.

Preferably, a genetically modified *Bordetella* strain of the present invention has a partial or complete loss of function in the endogenous *aroQ* gene and thus falls into the "knockout" group of genetically modified organisms. A knockout may be achieved by a variety of mechanisms, including introduction of a disruption of the coding sequence, *e.g*. insertion of one or more stop codons, insertion of a DNA fragment, etc., deletion of coding sequence, substitution of stop codons for coding sequence, etc. In some cases the foreign transgene sequences are ultimately deleted from the genome, leaving a net change to the native sequence. Different approaches may be used to achieve the "knockout". A chromosomal deletion of all or part of the native *aroQ* may be induced, including deletions of the non-coding regions, particularly the promoter region, 3' regulatory sequences, transcriptional regulators, or deletion of a gene that activates expression of *aroQ*. A functional knockout may also be achieved by the introduction of an anti-sense construct (anti-sense suppression) or a sense construct (co-suppression) that block expression of the native *aroQ* gene. "Knockouts" also include conditional knock-outs, for example where alteration of the target gene occurs upon exposure of the *Bordetella* strain to a substance that promotes target gene alteration, introduction of an enzyme that promotes recombination at the target gene site (*e.g*. Cre in the Cre-lox system), or other method for directing the target gene alteration.

In a preferred embodiment, the partial or complete loss of function in the *aroQ* gene is effected by disruption of that gene. In accordance with the present invention, the disruption suitably results in an inability of the genetically modified *Bordetella* strain to produce a corresponding functional expression product or detectable levels of that expression product. Accordingly, a disruption in the endogenous *aroQ* gene may result in a reduced level and/or functional activity of the 3-dehydroquinase (AroQ) encoded by the *aroQ* gene or in an inability of the strain to produce a functional 3-dehydroquinase or detectable levels of 3-dehydroquinase relative to a corresponding cell without the disruption.

A disruption typically comprises an insertion of a nucleic acid sequence into one region of the native genomic sequence (usually the *aroQ* open reading frame) and/or the promoter region of a gene so as to decrease or prevent expression of that gene in the cell as compared to the wild-type or naturally occurring sequence of the gene. By way of example, a nucleic acid construct can be prepared containing a selectable marker gene which is inserted into a targeting nucleic acid sequence that is complementary to a genomic sequence (promoter and/or coding region) to be disrupted. Useful genomic sequences to be disrupted include, but are not restricted to, *aroQ* open reading frames encoding polypeptides or domains and adjacent 5' and 3' non-coding nucleotide sequences involved in regulation of gene expression. Accordingly, a targeting sequence may comprise some or part of the nucleic acid present between the initiation codon and the stop codon of the *aroQ* open reading frame, as defined in the listed sequences. It may further include the 3' and 5' untranslated regions found in the mRNA. It may further include specific transcriptional and translational regulatory sequences, such as promoters, operators, etc., including about 1 kb, but possibly more, of flanking genomic DNA at either the 5' or 3' end of the transcribed region. When the nucleic acid construct is then introduced into a cell, the construct will integrate into the genomic DNA. Thus, many progeny of the cell will no longer express the gene at least in some cells, or will express it at a decreased level, as the genomic sequence is now disrupted by the selection marker.

In another embodiment, an individual disruption reduces, abrogates or otherwise impairs the expression of a *aroQ* gene and in this regard, the disruption may reside in the deletion of at least a portion of the transcriptional and/or translational regulatory sequences associated with said *aroQ* gene.

In a preferred embodiment, the disruption comprises a defined deletion in the *aroQ* gene. The deletion in the *aroQ* gene can be introduced by allelic exchange due to a double cross-over recombinational event, or any other method wherein a DNA replacement event in which two separate DNA recombination events result in the exchange of a piece of the intact gene for a homologous piece containing a deletion. The deletion is preferably large enough such that the gene is inactivated in the first attempt and to reduce the likelihood of a recombinational repair. Other genes, or at least portions thereof, which can be deleted include the *pur* genes (*e.g*., *purA, purE* or *purH*), other *aro* genes (*e.g*., *aroA, aroB*, *aroC* or *aroE*, or pertussis toxin gene, or any other gene which contributes to survival in the host and/or to bacterial virulence, or any combination of such genes. Any strain of *Bordetella* can be used to introduce such an attenuating mutation. The resulting attenuated strain can be tested for the deletion of the targeted gene by methods known in the art such as Southern blot hybridisation, and the level of attenuation tested in a mouse model as described in the Examples below. Any deletion in the *aroQ* gene would result in the attenuation of the bacteria unless the deletion was small and allowed for functional *aroQ* expression. Any deletion which inactivates the *aroQ* gene expression or blocks function of its gene product will result in sufficient attenuation of a pathogenic strain of *Bordetella* such that it has a lower capacity to propagate in a mammalian host but remain viable in the host for a period of time sufficient to induce an immune response against a pathogenic *Bordetella* strain and preferably the same strain that was the subject of attenuation

In another embodiment of the present invention, the genetically modified *Bordetella* strain having the deletion in the *aroQ* gene as described above additionally contains another deletion in a different gene. The advantage of having two deletions is to further reduce the possibility of reversion, and to additionally attenuate the bacteria. However, for use as an immunopotentiating composition or live vaccine, a certain amount of replication is necessary in the host. Therefore, in a preferred embodiment, the genetically modified strain is tested for its ability to survive in the host. These tests can be carried out *in vivo,* for example in the mouse model described herein, or, as a second step, in non-human primates.

In yet another embodiment, the genetically modified *Bordetella* strain of the present invention may be used as a vector or delivery vehicle of antigens from organisms other than said *Bordetella* strain. Illustrative embodiments of such heterologous antigens to be expressed by the genetically modified *Bordetella* strain of the present invention include, antigens bacteria, viruses, fungi, protozoa, metazoan parasites or the like. The heterologous structural genes may encode envelope proteins, capsid proteins, surface proteins such as outer membrane proteins and capsids, toxins such as exotoxins or enterotoxins, or the genes of interest may specify proteins, enzymes, or oligosaccharide antigen or for modification of a saccharide-containing antigen, such as LPS, of the host bacterial strain, or for synthesis of a polypeptide antigen, or variants or derivatives thereof. Specific examples of genes of interest include HIV glycoproteins, malarial circumsporozoite protein, HBV core protein, and arboviral coat protein, to name a few. Alternatively, or additionally, the heterologous antigen may be selected from disease associated antigens such as cancer-related antigens (*i.e.*, tumour-associated antigens such as the melanoma-associated MAGE antigens). In a preferred embodiment, the heterologous antigen is from other respiratory bacterial pathogens such as, but not limited to, *Helicobacter pylori*, *Haemophilus influenzae* and *Neisseria meningitidis* or from viral respiratory pathogens including those associated with measles, mumps or rubella.

One or more structural genes coding for desired heterologous antigen(s) may be introduced into the genetically modified *Bordetella* strain as an expression cassette. The expression cassette comprises the heterologous gene or genes of interest under the regulatory control of the transcriptional and translational initiation and termination elements which naturally border the gene of interest. Where bacterial or bacteriophage heterologous genes are involved, the natural or wild-type regulatory regions will usually, but not always, suffice. It may be necessary to join regulatory regions recognised by the genetically modified *Bordetella* strain to structural genes for antigens isolated from eukaryotes and occasionally prokaryotes.

The expression cassette may be a construct or may form part of a naturally occurring plasmid. If the expression cassette is a construct, it may be joined to a replication system for episomal maintenance or it may be introduced into the genetically modified *Bordetella* strain under conditions for recombination and integration into said strain's chromosomal DNA.

### *4. Targeting constructs*

The invention provides a targeting vector for producing a genetically modified *Bordetella* strain of the present invention, comprising at least a portion of a polynucleotide of the invention or variant or derivative of thereof. Specific constructs or vectors of interest include, but are not limited to, anti-sense *aroQ* constructs comprising a sequence complementary to a polynucleotide, fragment, variant or derivative as herein described, which will block native *aroQ* expression and expression of dominant negative AroQ mutations.

In a preferred embodiment, the vector is used to disrupt the *aroQ* gene, which results in partial or complete loss of function of that gene. Any polynucleotide sequence capable of disrupting an endogenous gene of interest by introducing a deletion, insertion or replacement of at least one nucleotide, and preferably from 10 to 20 nucleotides therein, (*e.g.*, resulting in a nonsense mutation, a missense mutation or a frame-shift mutation) may be employed in this regard. In a preferred embodiment, the vector, or an ancillary vector, comprises a positive selectable marker gene. The disruption may reduce or prevent the expression of *aroQ* or may render the resulting AroQ polypeptide completely non-functional. Reduced levels of AroQ refer to a level of AroQ which is lower than that found in a wild-type *Bordetella* strain. The level of AroQ produced in a *Bordetella* strain of interest may be determined by a variety of methods including Western blot analysis of protein extracted from that strain. A lack of ability to produce functional AroQ includes within its scope the production of undetectable levels of functional AroQ (*e.g.*, by Western blot analysis). In contrast, a functional AroQ is a molecule which retains the biological activity of the wild-type AroQ and which preferably is of the same molecular weight as the wild-type molecule.

Targeting vectors for homologous recombination (*i.e.*, allelic exchange) will comprise at least a portion of the *aroQ* gene with the desired genetic modification, and will include regions of homology to the target locus. Those regions may be non-isogenic, but are preferably isogenic, to the target locus. Conveniently, markers for positive and negative selection are included. Methods for generating cells having targeted gene modifications through homologous recombination are known in the art.

In certain embodiments, the targeting construct may contain more than one selectable marker gene. The selectable marker is preferably a polynucleotide which encodes an enzymatic activity that confers resistance to an antibiotic or drug upon the cell in which the selectable marker is expressed. Selectable markers may be "positive"; positive selectable markers typically are dominant selectable markers, *i.e.*, genes which encode an enzymatic activity which can be detected in any bacterial cell. Examples of dominant selectable markers include the bacterial aminoglycoside 3' phosphotransferase gene (also referred to as the *kan* gene) which confers resistance to the kanamycin ($Km^R$), the bacterial chloramphenicol acetyl transferase gene (also referred to as the *cat* gene) which confers resistance to the chloramphenicol ($Cm^R$). Selectable markers may be 'negative'; negative selectable markers (or otherwise called 'counter-selectable markers') encode an enzymatic activity whose expression is cytotoxic to the cell when grown in an appropriate selective medium, such as but not limited to a gene encoding *E. coli* S12 ribosomal protein, which renders a streptomycin resistant ($Sm^R$) host streptomycin sensitive ($Sm^s$) or a *sacB* gene as for example described by Pelicic et al. (1996, Mol. Microbiol. 20: 919-925).

When more than one selectable marker gene is employed, the targeting vector preferably contains a positive selectable marker (*e.g.*, the *kan* gene) and a counter-selectable marker (*e.g.*, *S12* gene). The presence of the positive selectable marker permits the selection of recombinant cells containing an integrated copy of the targeting vector whether this integration occurred at the target site or at a random site. The presence of the counter-selectable marker permits the identification of cells containing the targeting vector at the targeted site (*i.e.*, which has integrated by virtue of homologous recombination into the target site); cells which survive when grown in medium which selects against the expression of the counter-selectable marker do not contain a copy of the counter-selectable marker.

Preferred targeting vectors of the present invention are of the "replacement-type", wherein integration of a replacement-type vector results in the insertion of a selectable marker into the target gene. Replacement-type targeting vectors may be employed to disrupt a gene resulting in the generation of a null gene (*i.e.*, a gene incapable of expressing a functional protein; null genes may be generated by deleting a portion of the coding region, deleting the entire gene, introducing an insertion and/or a frameshift mutation, etc.) or may be used to introduce a modification (*e.g.*, one or more point mutations) into a gene.

The targeting construct or vector is then introduced into a host strain by any convenient means such as, but not limited to, conjugation, transformation, transfection, transduction, translocation, etc.

In a particularly preferred embodiment, as described in more detail below, a replacement-type targeting vector was used to introduce a deletion in the *aroQ* gene of *Bordetella pertussis* by allelic exchange with a copy of the *aroQ* gene present on the vector. The *aroQ* gene to be exchanged with the wild type version contained a deletion into which a kanamycin resistance gene was cloned. A copy of the *S12* gene was also present on the vector. Once the vector was introduced into *Bordetella pertussis,* by conjugation in this example, a double cross-over recombinational event occurred such that the vector *aroQ* gene containing the deletion, was exchanged for the chromosomal wild type *aroQ*. The resulting recombinant *Bordetella pertussis* strain was $Km^R$, $Sm^s$ and contained a defined mutation which conferred attenuation as described herein.

### *5. Polypeptides of the invention*

#### *5.1 Bordetella* AroQ polypeptides

The invention encompasses the use of AroQ polypeptides from *Bordetella* in immunopotentiating compositions. For example, such AroQ polypeptides may be used in concert with the genetically modified *Bordetella* strain of the present invention to elicit an immune response against *Bordetella* strains of pathogenic origin. An exemplary *Bordetella pertussis* sequence for AroQ is set forth in SEQ ID NO: 2.

#### 5.2 Biologically active fragments

Biologically active fragments may be produced according to any suitable procedure known in the art. For example, a suitable method may include first producing a fragment of said polypeptide and then testing the fragment for the appropriate biological activity. In one embodiment, the fragment may be tested for 3-dehydroquinase activity. In another embodiment, biological activity of the fragment is tested by introducing a polynucleotide from which a fragment of the polypeptide can be translated into a cell, and detecting 3-dehydroquinase activity, which is indicative of the fragment being a biologically active fragment.

The invention also contemplates biological fragments of the above polypeptides of at least 6 and preferably at least 8 amino acids in length, which can elicit an immune response in an animal for the production of antigen-binding molecules that are interactive with a 3-dehydroquinase of the invention. For example exemplary polypeptide fragments of 8 residues in length, which could elicit an immune response, include but are not limited to residues 1-8, 9-16, 17-24, 25-32, 33-40, 41-48, 49-56, 57-64, 65-72, 73-80, 81-88, 89-96, 97-104, 105-112, 113-120, 121-128, 129-136 and 137-144 of SEQ ID NO: 2.

#### 5.3 Variant AroQ polypeptides

The invention contemplates the use of variants of AroQ polypeptides from *Bordetella* in immunopotentiating compositions. Suitable methods of producing polypeptide variants include replacing at least one amino acid of a parent polypeptide comprising the sequence set forth in SEQ ID NO: 2, or a biologically active fragment thereof, with a different amino acid to produce a modified polypeptide, and testing said modified polypeptide for an activity of the parent AroQ polypeptide, including 3-dehydroquinase activity, which indicates that the modified polypeptide is a polypeptide variant.

In another embodiment, a polypeptide variant is produced by replacing at least one amino acid of a parent polypeptide comprising the sequence set forth in SEQ ID NO: 2, or a biologically active fragment thereof, with a different amino acid to produce a modified polypeptide, introducing said polypeptide or a polynucleotide from which the modified polypeptide can be translated into a cell having a deletion of, or disruption in, the corresponding *aroQ* gene, and detecting an activity of the parent AroQ polypeptide, including 3-dehydroquinase activity, which indicates that the modified polypeptide is a polypeptide variant. For example, one may test the recombinant cell for growth in the absence of aromatic amino acids.

In general, variants will be at least 80%, - more preferably at least 85%, even more preferably at least 90% and still even more preferably at least 95% homologous to a polypeptide as for example shown in SEQ ID NO: 2, or in fragments thereof. Suitably, variants will have at least 80%, - more preferably at least 85%, even more preferably at least 90% and still even more preferably at least 95% sequence identity to the sequence set forth in SEQ ID NO: 2.

Variant peptides or polypeptides, resulting from rational or established methods of mutagenesis or from combinatorial chemistries, for example, may comprise conservative amino acid substitutions. Exemplary conservative substitutions in a polypeptide or polypeptide fragment according to the invention may be made according to the following table:

**TABLE B**

| *Original Residue* | *Exemplary Substitutions* |
|---|---|
| Ala | Ser |
| Arg | Lys |
| Asn | Gln, His |
| Asp | Glu |
| Cys | Ser |
| Gln | Asn |
| Glu | Asp |
| Gly | Pro |
| His | Asn, Gln |
| Ile | Leu, Val |
| Leu | Ile, Val |
| Lys | Arg, Gln, Glu |
| Met | Leu, Ile, |
| Phe | Met, Leu, Tyr |
| Ser | Thr |
| Thr | Ser |
| Trp | Tyr |
| Tyr | Trp, Phe |
| Val | Ile, Leu |

Substantial changes in function are made by selecting substitutions that are less conservative than those shown in TABLE B. Other replacements would be non-conservative substitutions and relatively fewer of these may be tolerated. Generally, the substitutions which are likely to produce the greatest changes in a polypeptide's properties are those in which (a) a hydrophilic residue (*e.g.*, Ser or Asn) is substituted for, or by, a hydrophobic residue (*e.g.*, Ala, Leu, Ile, Phe or Val); (b) a cysteine or proline is substituted for, or by, any other residue; (c) a residue having an electropositive side chain (*e.g.*, Arg, His or Lys) is substituted for, or by, an electronegative residue (*e.g.*, Glu or Asp) or (d) a residue having a smaller side chain (*e.g.*, Ala, Ser) or no side chain (*e.g.*, Gly) is substituted for, or by, one having a bulky side chain (*e.g.*, Phe or Trp).

5.4 Polypeptide derivatives

With reference to suitable derivatives of the invention, such derivatives include amino acid deletions and/or additions to a polypeptide, fragment or variant of the invention, wherein said derivatives comprise an activity of an AroQ polypeptide, including 3-dehyroquinase activity. *"Additions"* of amino acids may include fusion of the polypeptides, fragments and polypeptide variants of the invention with other polypeptides or proteins. For example, it will be appreciated that said polypeptides, fragments or variants may be incorporated into larger polypeptides, and that such larger polypeptides may also be expected to modulate an activity as mentioned above.

The polypeptides, fragments or variants of the invention may be fused to a further protein, for example, which is not derived from the original host. The further protein may assist in the purification of the fusion protein. For instance, a polyhistidine tag or a maltose binding protein may be used in this respect as described in more detail below. Other possible fusion proteins are those which produce an immunomodulatory response. Particular examples of such proteins include Protein A or glutathione S-transferase (GST).

Other derivatives contemplated by the invention include, but are not limited to, modification to side chains, incorporation of unnatural amino acids and/or their derivatives during peptide, polypeptide or protein synthesis and the use of crosslinkers and other methods which impose conformational constraints on the polypeptides, fragments and variants of the invention. Examples of side chain modifications contemplated by the present invention include modifications of amino groups such as by acylation with acetic anhydride; acylation of amino groups with succinic anhydride and tetrahydrophthalic anhydride; amidination with methylacetimidate; carbamoylation of amino groups with cyanate; pyridoxylation of lysine with pyridoxal-5-phosphate followed by reduction with $NaBH_4$; reductive alkylation by reaction with an aldehyde followed by reduction with $NaBH_4$; and trinitrobenzylation of amino groups with 2, 4, 6-trinitrobenzene sulphonic acid (TNBS). The carboxyl group may be modified by carbodiimide activation via O-acylisourea formation followed by subsequent derivatisation, by way of example, to a corresponding amide. The guanidine group of arginine residues may be modified by formation of heterocyclic condensation products with reagents such as 2,3-butanedione, phenylglyoxal and glyoxal. Sulphydryl groups may be modified by methods such as performic acid oxidation to cysteic acid; formation of mercurial derivatives using 4-chloromercuriphenylsulphonic acid, 4-chloromercuribenzoate; 2-chloromercuri-4-nitrophenol, phenylmercury chloride, and other mercurials; formation of a mixed disulphides with other thiol compounds; reaction with maleimide, maleic anhydride or other substituted maleimide; carboxymethylation with iodoacetic acid or iodoacetamide; and carbamoylation with cyanate at alkaline pH. Tryptophan residues may be modified, for example, by alkylation of the indole ring with 2-hydroxy-5-nitrobenzyl bromide or sulphonyl halides or by oxidation with N-bromosuccinimide. Tyrosine residues may be modified by nitration with tetranitromethane to form a 3-nitrotyrosine derivative. The imidazole ring of a histidine residue may be modified by N-carbethoxylation with diethylpyrocarbonate or by alkylation with iodoacetic acid derivatives.

Examples of incorporating unnatural amino acids and derivatives during peptide synthesis include but are not limited to, use of 4-amino butyric acid, 6-aminohexanoic acid, 4-amino-3-hydroxy-5-phenylpentanoic acid, 4-amino-3-hydroxy-6-methylheptanoic acid, t-butylglycine, norleucine, norvaline, phenylglycine, ornithine, sarcosine, 2-thienyl alanine and/or D-isomers of amino acids. A list of unnatural amino acids contemplated by the present invention is shown in TABLE C.

**TABLE C**

| *Non-conventional amino acid* | *Non-conventional amino acid* |
|---|---|
| α-aminobutyric acid | L-N-methylalanine |
| α-amino-α-methylbutyrate | L-N-methylarginine |
| aminocyclopropane-carboxylate | L-N-methylasparagine |
| aminoisobutyric acid | L-N-methylaspartic acid |
| aminonorbornyl-carboxylate | L-N-methylcysteine |
| cyclohexylalanine | L-N-methylglutamine |
| cyclopentylalanine | L-N-methylglutamic acid |
| L-N-methylisoleucine | L-N-methylhistidine |
| D-alanine | L-N-methylleucine |
| D-arginine | L-N-methyllysine |
| D-aspartic acid | L-N-methylmethionine |
| D-cysteine | L-N-methylnorleucine |
| D-glutamate | L-N-methylnorvaline |
| D-glutamic acid | L-N-methylornithine |
| D-histidine | L-N-methylphenylalanine |
| D-isoleucine | L-N-methylproline |
| D-leucine | L-N-medlylserine |
| D-lysine | L-N-methylthreonine |
| D-methionine | L-N-methyltryptophan |
| D-omithine | L-N-methyltyrosine |
| D-phenylalanine | L-N-methylvaline |

(continued)

| *Non-conventional amino acid* | *Non-conventional amino acid* |
|---|---|
| D-proline | L-N-methylethylglycine |
| D-serine | L-N-methyl-t-butylglycine |
| D-threonine | L-norleucine |
| D-tryptophan | L-norvaline |
| D-tyrosine | α-methyl-aminoisobutyrate |
| D-valine | α-methyl-γ-aminobutyrate |
| D-α-methylalanine | α-methylcyclohexylalanine |
| D-α-methylarginine | α-methylcylcopentylalanine |
| D-α-methylasparagine | α-methyl-α-napthylalanine |
| D-α-methylaspartate | α-methylpenicillamine |
| D-α-methylcysteine | N-(4-aminobutyl)glycine |
| D-α-methylglutamine | N-(2-aminoethyl)glycine |
| D-α-methylhistidine | N-(3-aminopropyl)glycine |
| D-α-methylisoleucine | N-amino-α-methylbutyrate |
| D-α-methylleucine | α-napthylalanine |
| D-α-methyllysine | N-benzylglycine |
| D-α-methylmethionine | N-(2-carbamylediyl)glycine |
| D-α-methylornithiine | N-(carbamylmethyl)glycine |
| D-α-methylphenylalanine | N-(2-carboxyethyl)glycine |
| D-α-methylproline | N-(carboxymethyl)glycine |
| D-α-methylserine | N-cyclobutylglycine |
| D-α-methylthreonine | N-cycloheptylglycine |
| D-α-methyltryptophan | N-cyclohexylglycine |
| D-α-methyltyrosine | N-cyclodecylglycine |
| L-α-methylleucine | L-α-methyllysine |
| L-α-methylmethionine | L-α-methylnorleucine |
| L-α-methylnorvatine | L-α-methylornithine |
| L-α-methylphenylalanine | L-α-methylproline |
| L-α-methylserine | L-α-methylthreonine |
| L-α-methyltryptophan | L-α-methyltyrosine |
| L-α-methylvaline | L-N-methylhomophenylalanine |
| N-(N-(2,2-diphenylethyl carbamylmethyl)glycine | N-(N-(3,3-diphenylpropyl carbamylmethyl)glycine |
| 1-carboxy-1-(2,2-diphenyl-ethyl amino)cyclopropane | |

Also contemplated is the use of crosslinkers, for example, to stabilise 3D conformations of the polypeptides, fragments or variants of the invention, using homo-bifunctional cross linkers such as bifunctional imido esters having $(CH_2)_n$ spacer groups with n = 1 to n = 6, glutaraldehyde, N-hydroxysuccinimide esters and hetero-bifunctional reagents which usually contain an amino-reactive moiety such as N-hydroxysuccinimide and another group specific-reactive moiety such as maleimido or dithio moiety or carbodiimide. In addition, peptides can be conformationally constrained, for example, by introduction of double bonds between $C_\alpha$ and $C_\beta$ atoms of amino acids, by incorporation of $C_\alpha$ and $N_\alpha$-methylamino acids, and by formation of cyclic peptides or analogues by introducing covalent bonds such as forming an amide bond between the N and C termini between two side chains or between a side chain and the N or C terminus of the peptides or analogues. For example, reference may be made to: Marlowe (1993, Biorganic & Medicinal Chemistry Letters 3: 437-44) who describes peptide cyclisation on TFA resin using trimethylsilyl (TMSE) ester as an orthogonal protecting group; Pallin and Tam (1995, J. Chem. Soc. Chem. Comm. 2021-2022) who describe the cyclisation of unprotected peptides in aqueous solution by oxime formation; Algin et al (1994, Tetrahedron Letters 35: 9633-9636) who disclose solid-phase synthesis of head-to-tail cyclic peptides *via* lysine side-chain anchoring; Kates et al (1993, Tetrahedron Letters 34: 1549-1552) who describe the production of head-to-tail cyclic peptides by three-dimensional solid phase strategy; Tumelty et al (1994, J. Chem. Soc. Chem. Comm. 1067-1068) who describe the synthesis of cyclic peptides from an immobilised activated intermediate, wherein activation of the immobilised peptide is carried out with *N*-protecting group intact and subsequent removal leading to cyclisation; McMurray et al (1994, Peptide Research 7:

195-206) who disclose head-to-tail cyclisation of peptides attached to insoluble supports by means of the side chains of aspartic and glutamic acid; Hruby et al (1994, Reactive Polymers 22: 231-241) who teach an alternate method for cyclising peptides *via* solid supports; and Schmidt and Langer (1997, J. Peptide Res. 49: 67-73) who disclose a method for synthesising cyclotetrapeptides and cyclopentapeptides. The foregoing methods may be used to produce conformationally constrained polypeptides that modulate reproductive function.

The invention also contemplates polypeptides, fragments or variants of the invention that have been modified using ordinary molecular biological techniques so as to improve their resistance to proteolytic degradation or to optimise solubility properties or to render them more suitable as an immunogenic agent.

5.5 Methods of preparing *AroQ* polypeptides

AroQ polypeptides, fragments, variants or derivatives may be prepared by any suitable procedure known to those of skill in the art. For example, such polypeptides may be prepared by a procedure including the steps of (a) preparing a recombinant polynucleotide comprising a nucleotide sequence encoding a polypeptide comprising the sequence set forth in any one of SEQ ID NO: 2, or a biologically active fragment thereof, or variant or derivative of these, which nucleotide sequence is operably linked to regulatory elements; (b) introducing the recombinant polynucleotide into a suitable host cell; (c) culturing the host cell to express recombinant polypeptide from the recombinant polynucleotide; and (d) isolating the recombinant polypeptide. Preferred nucleotide sequences include, but are not limited to the sequences set forth in SEQ ID NO: 1 or 3.

The recombinant polynucleotide is preferably in the form of an expression vector that may be a self-replicating extra-chromosomal vector such as a plasmid, or a vector that integrates into a host genome. The regulatory elements will generally be appropriate for the host cell used for expression. Numerous types of appropriate expression vectors and suitable regulatory sequences are known in the art for a variety of host cells. Typically, the regulatory elements include, but are not limited to, promoter sequences, leader or signal sequences, ribosomal binding sites, transcriptional start and stop sequences, translational start and termination sequences, and enhancer or activator sequences. Constitutive or inducible promoters as known in the art are contemplated by the invention. The promoters may be either naturally occurring promoters, or hybrid promoters that combine elements of more than one promoter.

In a preferred embodiment, the expression vector contains a selectable marker gene to allow the selection of transformed host cells. Selection genes are well known in the art and will vary with the host cell used.

The expression vector may also include a fusion partner (typically provided by the expression vector) so that the recombinant polypeptide of the invention is expressed as a fusion polypeptide with said fusion partner. The main advantage of fusion partners is that they assist identification and/or purification of said fusion polypeptide. In order to express said fusion polypeptide, it is necessary to ligate a polynucleotide according to the invention into the expression vector so that the translational reading frames of the fusion partner and the polynucleotide coincide. Well known examples of fusion partners include, but are not limited to, glutathione-S-transferase (GST), Fc potion of human IgG, maltose binding protein (MBP) and hexahistidine ($HIS_6$), which are particularly useful for isolation of the fusion polypeptide by affinity chromatography. For the purposes of fusion polypeptide purification by affinity chromatography, relevant matrices for affinity chromatography are glutathione-, amylose-, and nickel- or cobalt-conjugated resins respectively. Many such matrices are available in "kit" form, such as the QIAexpress™ system (Qiagen) useful with ($HIS_6$) fusion partners and the Pharmacia GST purification system. In a preferred embodiment, the recombinant polynucleotide is expressed in the commercial vector pFLAG as described more fully hereinafter. Another fusion partner well known in the art is green fluorescent protein (GFP). This fusion partner serves as a fluorescent "tag" which allows the fusion polypeptide of the invention to be identified by fluorescence microscopy or by flow cytometry. The GFP tag is useful when assessing subcellular localisation of the fusion polypeptide of the invention, or for isolating cells which express the fusion polypeptide of the invention. Flow cytometric methods such as fluorescence activated cell sorting (FACS) are particularly useful in this latter application. Preferably, the fusion partners also have protease cleavage sites, such as for Factor $X_a$ or Thrombin, which allow the relevant protease to partially digest the fusion polypeptide of the invention and thereby liberate the recombinant polypeptide of the invention therefrom. The liberated polypeptide can then be isolated from the fusion partner by subsequent chromatographic separation. Fusion partners according to the invention also include within their scope "epitope tags", which are usually short peptide sequences for which a specific antibody is available. Well known examples of epitope tags for which specific monoclonal antibodies are readily available include c-Myc, influenza virus, haemagglutinin and FLAG tags.

The step of introducing into the host cell the recombinant polynucleotide may be effected by any suitable method including transfection, and transformation, the choice of which will be dependent on the host cell employed. Such methods are well known to those of skill in the art.

Recombinant polypeptides of the invention may be produced by culturing a host cell transformed with an expression vector containing nucleic acid encoding a polypeptide, biologically active fragment, variant or derivative according to the invention. The conditions appropriate for protein expression will vary with the choice of expression vector and the host cell. This is easily ascertained by one skilled in the art through routine experimentation.

Suitable host cells for expression may be prokaryotic or eukaryotic. One preferred host cell for expression of a polypeptide according to the invention is a bacterium. The bacterium used may be *E. coli.*

The recombinant protein may be conveniently prepared by a person skilled in the art using standard protocols as for example described in Sambrook, *et al.,* 1989, in particular Sections 16 and 17; Ausubel *et al.,* (1994-1998), in particular Chapters 10 and 16; and Coligan *et al.,* (1995-1997), in particular Chapters 1, 5 and 6.

Alternatively, the AroQ polypeptide, fragments, variants or derivatives may be synthesised using solution synthesis or solid phase synthesis as described, for example, in Chapter 9 of Atherton and Shephard (*supra*) and in Roberge *et al* (1995).

### *6. Antigen-binding molecules*

The invention also contemplates antigen-binding molecules that bind specifically to the aforementioned polypeptides, fragments, variants and derivatives. Preferably, an antigen-binding molecule according to the invention is interactive with the amino acid sequences set forth in SEQ ID NO: 2 or variants thereof. For example, the antigen-binding molecules may comprise whole polyclonal antibodies. Such antibodies may be prepared, for example, by injecting a polypeptide, fragment, variant or derivative of the invention into a production species, which may include mice or rabbits, to obtain polyclonal antisera. Methods of producing polyclonal antibodies are well known to those skilled in the art. Exemplary protocols which may be used are described for example in Coligan et al., CURRENT PROTOCOLS IN IMMUNOLOGY, (John Wiley & Sons, Inc, 1991), and Ausubel *et al.,* (1994-1998, *supra*), in particular Section III of Chapter 11. In lieu of the polyclonal antisera obtained in the production species, monoclonal antibodies may be produced using the standard method as described, for example, by Köhler and Milstein (1975, Nature 256, 495-497), or by more recent modifications thereof as described, for example, in Coligan *et al.,* (1991, *supra*) by immortalising spleen or other antibody producing cells derived from a production species which has been inoculated with one or more of the polypeptides, fragments, variants or derivatives of the invention.

The invention also contemplates as antigen-binding molecules Fv, Fab, Fab' and $F(ab')_2$ immunoglobulin fragments. Alternatively, the antigen-binding molecule may comprise a synthetic stabilised Fv fragment. Exemplary fragments of this type include single chain Fv fragments (sFv, frequently termed scFv) in which a peptide linker is used to bridge the N terminus or C terminus of a $V_H$ domain with the C terminus or N-terminus, respectively, of a $V_L$ domain. ScFv lack all constant parts of whole antibodies and are not able to activate complement. Suitable peptide linkers for joining the $V_H$ and $V_L$ domains are those which allow the $V_H$ and $V_L$ domains to fold into a single polypeptide chain having an antigen binding site with a three dimensional structure similar to that of the antigen binding site of a whole antibody from which the Fv fragment is derived. Linkers having the desired properties may be obtained by the method disclosed in U.S. Patent No 4,946,778. However, in some cases a linker is absent. ScFvs may be prepared, for example, in accordance with methods outlined in Kreber *et al* (Kreber et al. 1997, J. Immunol. Methods; 201(1): 35-55). Alternatively, they may be prepared by methods described in U.S. Patent No 5,091,513, European Patent No 239,400 or the articles by Winter and Milstein (1991, Nature 349:293) and Pliickthun et al (1996, In Antibody engineering: A practical approach. 203-252).

Alternatively, the synthetic stabilised Fv fragment comprises a disulphide stabilised Fv (dsFv) in which cysteine residues are introduced into the $V_H$ and $V_L$ domains such that in the fully folded Fv molecule the two residues will form a disulphide bond therebetween. Suitable methods of producing dsFv are described for example in (Glockscuther et al. Biochem. 29: 1363-1367; Reiter et al. 1994, J. Biol. Chem. 269: 18327-18331; Reiter et al. 1994, Biochem. 33: 5451-5459; Reiter et al. 1994. Cancer Res. 54: 2714-2718; Webber et al. 1995, Mol. Immunol. 32: 249-258).

Also contemplated as antigen-binding molecules are single variable region domains (termed dAbs) as for example disclosed in Ward et al. (1989, Nature 341: 544-546); Hamers-Casterman et al. (1993, Nature. 363: 446-448); Davies & Riechmann, (1994, FEBS Lett. 339: 285-290).

Alternatively, the antigen-binding molecule may comprise a "minibody". In this regard, minibodies are small versions of whole antibodies, which encode in a single chain the essential elements of a whole antibody. Suitably, the minibody is comprised of the $V_H$ and $V_L$ domains of a native antibody fused to the hinge region and CH3 domain of the immunoglobulin molecule as, for example, disclosed in U.S. Patent No 5,837,821.

In an alternate embodiment, the antigen binding molecule may comprise non-immunoglobulin derived, protein frameworks. For example, reference may be made to Ku & Schultz, (1995, Proc. Natl. Acad. Sci. USA, 92: 652-6556) which discloses a four-helix bundle protein cytochrome b562 having two loops randomised to create complementarity determining regions (CDRs), which have been selected for antigen binding.

The antigen-binding molecule may be multivalent (*i.e.*, having more than one antigen binding site). Such multivalent molecules may be specific for one or more antigens. Multivalent molecules of this type may be prepared by dimerisation of two antibody fragments through a cysteinyl-containing peptide as, for example disclosed by Adams et al., (1993, Cancer Res. 53: 4026-4034) and Cumber et al. (1992, J. Immunol. 149: 120-126). Alternatively, dimerisation may be facilitated by fusion of the antibody fragments to amphiphilic helices that naturally dimerise (Pack P. Plünckthun, 1992, Biochem. 31: 1579-1584), or by use of domains (such as the leucine zippers jun and fos) that preferentially heterodimerise (Kostelny et al., 1992, J. Immunol. 148: 1547-1553). In an alternate embodiment, the multivalent molecule may comprise a multivalent single chain antibody (multi-scFv) comprising at least two scFvs linked together by a peptide linker. In this regard, non-covalently or covalently linked scFv dimers termed "diabodies" may be used. Multi-scFvs may be bispecific or greater depending on the number of scFvs employed having different antigen binding specificities. Multi-scFvs may be prepared for example by methods disclosed in U.S. Patent No. 5,892,020.

The antigen-binding molecules of the invention may be used for affinity chromatography in isolating a natural or recombinant polypeptide or biologically active fragment of the invention. For example reference may be made to immunoaffinity chromatographic procedures described in Chapter 9.5 of Coligan *et al.,* (1995-1997, *supra*).

The antigen-binding molecules can be used to screen expression libraries for variant polypeptides of the invention as described herein. They can also be used to detect and/or isolate the polypeptides, fragments, variants and derivatives of the invention. Thus, the invention also contemplates the use of antigen-binding molecules to isolate 3-dehydroquinase enzymes using , for example, any suitable immunoaffinity based method including, but not limited to, immunochromatography and immunoprecipitation. A preferred method utilises solid phase adsorption in which anti-3-dehydroquinase antigen-binding molecules are attached to a suitable resin, the resin is contacted with a sample suspected of containing 3-dehydroquinases, and the 3-dehydroquinases, if any, are subsequently eluted from the resin. Preferred resins include: Sepharose® (Pharmacia), Poros® resins (Roche Molecular Biochemicals, Indianapolis), Actigel Superflow™ resins (Sterogene Bioseparations Inc., Carlsbad Calif.), and Dynabeads™ (Dynal Inc., Lake Success, N.Y.).

Alternatively, the antigen-binding molecules can be used to screen for *aroQ*$^-$ mutants of *Bordetella* as a facile means of identifying attenuated *Bordetella* strains of the pathogenic origin in accordance with the present invention.

### *7. Immunopotentiating compositions*

The genetically modified *Bordetella* strain of the present invention is useful for the design of immunopotentiating compositions that are effective in eliciting an immune response, and preferably a protective immune response, against a pathogenic *Bordetella* strain, more suitably a natural pathogenic *Bordetella* counterpart of the genetically modified *Bordetella* strain, or related organism and will, therefore, find utility in treating and/or preventing whooping cough or related conditions. Thus, the present invention additionally encompasses immunopotentiating compositions comprising one ore more genetically modified *Bordetella* strains described above in combination with a pharmaceutically acceptable carrier (such as, for example, saline buffer), and optionally in combination with at least one adjuvant such as aluminum hydroxide or a compound belonging to the muramyl peptide family.

Various methods for achieving adjuvant effect for the vaccine include the use of agents such as aluminum hydroxide or phosphate (alum), commonly used as 0.05 to 0.1 percent solution in phosphate buffered saline, admixture with synthetic polymers of sugars (Carbopol) used as 0.25% solution. Other suitable adjuvant compounds can be selected from DDA (dimethyldioctadecyl-ammonium bromide), microparticle poly-lactide-co-glycolide (PLG) or nanoparticle PLG formulations (Conway et al., 2001), as well as immune modulating substances, such as lymphokines (e.g., interferon (IFN) -gamma, interleukin (IL) -1, IL-2 and IL-12) or IFN-gamma inducers compounds, such as poly I:C or other immunostimulatory formulations or compounds including natural products particularly traditional medicines of different ethnobotanical origins.

The immunopotentiating composition according to the present invention may be prepared as an injectable form (either as liquid solution or suspension). However, solid forms suitable for solution in or suspension in, liquid prior to injection may also be prepared. In addition, if desired, the immunopotentiating composition may contain minor amounts of auxiliary substances such as wetting or emulsifying agents, pH buffering agents, or adjuvants which enhance the effectiveness of the vaccines.

The immunopotentiating compositions of the invention are administered in a manner compatible with the dosage formulation, and in such amount as will be therapeutically effective and immunogenic. The quantity to be administered depends on the subject to be treated including, e.g., the capacity of the individual's immune system to induce an immune response.

In a preferred embodiment, the immunopotentiating composition is formulated for intranasal and/or inhalational administration. The genetically modified *Bordetella* strains-containing compositions of the present invention are preferably used as immunogenic agents for the treatment and/or prevention of diseases associated with infection by pathogenic strains of *Bordetella* and related organisms. Thus, such compositions are preferably delivered to the lower and middle respiratory tracts by an inhalation route. For administration by inhalation into the lower respiratory tract (*e.g.,* the bronchioles), the genetically modified *Bordetella* can be formulated into a solution and/or a suspension of particles in a carrier appropriate for inhalation. Such carriers are also well known to the ordinary artisan familiar with inhalants for the delivery of fine droplets and insufflations and for the delivery of inhalable fine particles. The mean particle size of the droplet or powder for inhalation administration into the lower respiratory tract (*e.g.*, the bronchioles) is commonly, but not exclusively, in the order of from about 0.5 to about 10 microns, and typically ranges from about 0.5 to about 1.0 micron, comprised of powders, mists or aerosols, into the lower respiratory tract (Remington's, Id., at page 1451). For inhalation administration into the middle respiratory tract (*e.g.*, the oro-pharynx) the mean particle size of the droplet or powder is commonly, but not exclusively, in the order of from about 1 to about 50 microns, and typically from about 1 to about 10 micron. The mean particle size of the droplet or powder for inhalation administration into the upper respiratory tract (*e.g.*, the nasal mucosa) is commonly, but not exclusively, in the order of from about 10 to 355 microns, and typically from about 20 to about 200 micron. In one embodiment for delivery of an active to the middle and lower respiratory tract, the mean particle size of the droplet or powder is preferably in the range of from about 0.8 to 1.2 microns. In determining aerosol compositions, reference may also be made, for example, to a text entitled "Pharmaceutical Inhalation Aerosol Technology" edited by A. J. Hickey and published by Marcel Dekker, which discusses the importance of particle size in therapeutic aerosols for effective delivery of a medicament to a destination site.

Compositions in the form of droplets, mists or aerosols typically comprise surfactants to ensure good dispersion of a powdered medicament and also to provide for smooth operation of the valve through which the composition is dispensed. Conventional surfactants include, but are not restricted to, sorbitan triolate and oleic acid. Solvents have also been used to increase the solubility of the surfactant in the propellant.

For inhalation of droplets, mists and aerosols various devices such as nebulisers or pressurised aerosol generators are readily available. In addition, such devices can be metered to provide uniformity of dosing (Remington's, Id.). Chlorofluorocarbons (CFC) can be used as propellants but other propellants that are more environmentally friendly such as hydrofluorocarbon (HFC) propellants could also be employed and in this regard reference may be made to various HFC propellants disclosed in International Publications WO92/08447, WO92/06675 and WO91/04011.

When providing a patient with live bacterial immonpotentiating compositions or vaccines, the dosage of the composition will depend upon the route of administration and will vary according to the age of the patient to be vaccinated and, to a lesser degree, the size, weight, height, sex, general medical condition, previous medical history etc of the person to be vaccinated as well as the excipient or adjuvant present in the composition. In general, it is desirable to provide the recipient with a dosage range of from about $10^4$ colony forming units (cfu) to $10^{11}$ cfu, more suitably of from about $10^5$ cfu to $10^{10}$ cfu although a lower or higher dosage may be administered. Most preferably, the vaccine composition according to the present invention is administered *via* an intranasal and/or inhalational route and as part of a single or multiple administrations.

In the case of neonates, the dose will be approximately four times less than for an adult, and in the case of young children (4-6 years old), the dose will be approximately half the dose used for an adult healthy patient.

In some instances, it will be necessary to proceed with multiple administrations of the composition according to the present invention, usually not exceeding six administrations, more usually not exceeding four administrations, and preferably one or more, usually at least about three administrations. The administrations will normally be at from two to twelve week intervals, more usually from three to five week intervals. Periodic boosters at intervals of 1-5 years, usually three years, will be desirable to maintain the desired levels of protective immunity.

### *8. Dendritic cell embodiments*

The invention further provides a composition of matter for eliciting a cellular and/or a humoral immune response against a target antigen, comprising dendritic cells which have been exposed to a genetically modified *Bordetella* strain as broadly described above for a time and under conditions sufficient to express a processed or modified antigen derived from said *Bordetella* strain for presentation to, and modulation of, T cells. *Bordetella* antigen-primed dendritic cells may be prepared by a method including contacting dendritic cells with a genetically modified *Bordetella* as broadly described above, for a time and under conditions sufficient to permit said *Bordetella* to be internalised by the dendritic cells; and culturing the *Bordetella* antigen-containing dendritic cells for a time and under conditions sufficient for the modified antigen to be processed for presentation by the dendritic cells.

#### <u>8.1 Sources of dendritic cells</u>

The dendritic cells used in this invention can be isolated by methods known to those of skill in the art. They can be autologous or allogeneic with respect to the subject to be treated. Suitably, mammalian and preferably human dendritic cells are used from an appropriate tissue source, which is suitably blood or bone marrow. Dendritic cell precursors, from which the immature dendritic cells for use in antigen internalisation according to the invention, are present in blood as peripheral blood mononuclear cells (PBMCs). Although most easily obtainable from blood, the precursor cells may also be obtained from any tissue in which they reside, including bone marrow and spleen tissue. Peripheral blood precursors may be purified using monoclonal antibodies, density gradients or centrifugation or any combination of these. Circulating frequency may be increased *in vivo* using flt-3 ligand. When cultured in the presence of cytokines such as a combination of GM-CSF and IL-4 or IL-13 as described below, the non-proliferating precursor cells give rise to immature dendritic cells for use in this invention.

An exemplary method for culturing pluripotential PBMCs to produce immature dendritic cells is described by Albert et al. (International Publication WO 99/42564). In this respect, cultures of immature dendritic cells, *ie*. antigen-capturing phagocytic dendritic cells, may be obtained by culturing non-proliferating precursor cells (PBMCs) in the presence of cytokines which promote their differentiation. A combination of GM-CSF and IL-4 produces significant quantities of the immature dendritic cells, ie. antigen-capturing phagocytic or internalisation-competent dendritic cells. Other cytokines that promote differentiation of precursor cells into immature dendritic cells include, but are not limited to, IL-13.

Maturation of dendritic cells requires the addition to the cell environment, preferably the culture medium, of a dendritic cell one or more maturation factors which may be selected from monocyte conditioned medium and/or factors including TNF-$\alpha$, IL-6, IFN-$\alpha$ and IL-1. Alternatively, a mixture of necrotic cells or necrotic cell lysate may be added to induce maturation. Maturation can be induced *in vitro* using plastic adherence, cytokines, LPS, bacteria, DNA containing CpG repeats, RNA or polyIC, CD40-ligation, necrotic cells. In this regard, reference may be made to Steinman et al. (International Publication WO 97/29182) who describe methods and compositions for isolation and maturation of dendritic cells.

Other methods for isolation, expansion and/or maturation of dendritic cells for the purpose of the present invention are described for example by Takamizawa et al. (1997, J Immunol, 158 (5): 2134-2142), Thomas and Lipsky (1994, J Immunol, 153 (9): 4016-4028), O'Doherty et al. (1994, Immunology, 82 (3): 487-93), Fearnley et al. (1997, Blood, 89 (10): 3708-3716), Weissman et al. (1995, Proc Natl Acad Sci U S A, 92 (3): 826-830), Freudenthal and Steinman (1990, Proc Natl Acad Sci U S A, 87 (19): 7698-7702), Romani et al. (1996, J Immunol Methods, 196 (2): 137-151), Reddy et al. (1997, Blood, 90 (9): 3640-3646), Thurnher et al. (1997, Exp Hematol, 25 (3): 232-237), Caux et al. (1996, J Exp Med, 184 (2): 695-706; 1996, Blood, 87 (6): 2376-85), Luft et al. (1998, Exp Hematol, 26 (6): 489-500; 1998, J Immunol, 161 (4): 1947-1953), Cella et al. (1999, J Exp Med, 189 (5): 821-829; 1997, Nature, 388 (644): 782-787; 1996, J Exp Med, 184 (2): 747-572), Ahonen et al. (1999, Cell Immunol, 197(1): 62-72) and Piemonti et al. (1999, J Immunol, 162 (11): 6473-6481).

Alternatively, transformed or immortalised dendritic cell lines may be produced using oncogenes such as *v-myc* as for example described by Paglia et al. (1993, J Exp Med, 178 (6): 1893-1901).

8.2 Antigen priming of dendritic cells

The number of a genetically modified *Bordetella* cells to be placed in contact with dendritic cells can be determined empirically by persons of skill in the art. Dendritic cells are incubated with *Bordetella* cells for 1-2 hr at 37° C. For most antigens , 10 $\mu$g/mL to 1-10 million dendritic cells is suitable for priming the dendritic cells. In a preferred embodiment, immature dendritic cells are utilised for the antigen internalisation.

The genetically modified *Bordetella* cells should be exposed to the dendritic cells for a period of time sufficient for the dendritic cells to internalise the antigen. The time necessary for the cells to internalise and present the processed *Bordetella* antigens may be determined using pulse-chase protocols in which exposure to *Bordetella* cells is followed by a washout period. Once the minimum time necessary for cells to express processed *Bordetella* antigen on their surface is determined, a protocol may be used to prepare cells and *Bordetella* cells for eliciting immunogenic responses. Those of skill in the art will recognise in this regard that the length of time necessary for an antigen-presenting cell to phagocytose or internalise an antigen may vary depending on the antigen used. Efficiency of priming of the dendritic cells can be determined by assaying T cell cytolytic activity *in vitro* or using dendritic cells as targets of CTLs. Other methods known to practitioners in the art, which can detect the presence of antigen on the surface of dendritic cells after exposure to the *Bordetella* cells, are also contemplated by the presented invention.

The primed dendritic cells of the present invention have the capacity to efficiently present a processed *Bordetella* antigen in the form of peptides on both MHC class I and class II molecules. Modified antigens are acquired by dendritic cells through the exogenous pathway by phagocytosis and, as a result, efficiently charge MHC class II molecules. Accordingly, both $CD4^+$ T helper lymphocytes and CTL may be activated by dendritic cells presenting *Bordetella* antigen in the context of MHC class II. These lymphocytes can provide critical sources of help, both for generating active $CD8^+$ CTL and can in some circumstances be primed as $CD4^+$ CTL with specificity for the target antigen during the acute response to antigen, and for generating the memory that is required for long term resistance and vaccination. Further, *Bordetella* cell uptake and presentation by dendritic cells, allows these cells to tailor the peptides that are appropriate for an individual's MHC products, and increases the number of specialised stimulatory antigen-presenting cells. Moreover, dendritic cells can be charged with multiple antigens on multiple MHCs to yield polyclonal or oligoclonal stimulation of T cells. Thus, by using the modified antigens of the present invention to charge MHC class I and class II molecules, efficient T cell modulation *in situ* can be achieved.

8.3 Therapeutic and prophylactic applications *of Bordetella-primed* dendritic cells

Genetically modified *Bordetella-primed* dendritic cells and antigen-specific T lymphocytes generated with these dendritic cells *infra* can be used as actives in immunomodulating compositions for prophylactic or therapeutic applications. The primed cells, which are preferably mature dendritic cells, can be injected by any method that elicits an immune response into a syngeneic animal or human. Preferably, dendritic cells are injected back into the same animal or human from whom the source cells were obtained, and are, therefore, autologous dendritic cells. The injection site may be subcutaneous, intraperitoneal, intramuscular, intradermal, or intravenous. The number of conjugate-primed dendritic cells reinjected back into the animal or human in need of treatment may vary depending on *inter alia,* the virulence of the *Bordetella* cells and size of the individual. This number may range for example between about $10^4$ and $10^8$, and more preferably between about $10^6$ and $10^7$ conjugate-primed dendritic cells. The dendritic cells should be administered in a pharmaceutically acceptable carrier, which is non-toxic to the cells and the individual. Such carrier may be the growth medium in which the dendritic cells were grown, or any suitable buffering medium such as phosphate buffered saline.

A key feature in the function of dendritic cells *in situ* is the capacity to migrate or home to the T-dependent regions of lymphoid tissues, where the dendritic cells would be in an optimal position to select the requisite antigen-reactive T cells from the pool of recirculating quiescent lymphocytes and thereby initiate the T-dependent response.

In a preferred embodiment, the *Bordetella-primed* dendritic cells of the invention are suitable for treatment or prophylaxis of infection by a strain, preferably a pathogenic strain, of *Bordetella.* In another embodiment, the *Bordetella-primed* dendritic cells of the invention could also be used for generating large numbers of $CD8^+$ or CD4+ CTL, for adoptive transfer to immunosuppressed individuals who are unable to mount normal immune responses. For example, antigen-specific $CD8^+$ CTL can be adoptively transferred for therapeutic purposes in individuals afflicted with HIV infection (Koup et al., 1991, J. Exp. Med., 174: 1593-1600; Carmichael et al., 1993, J. Exp. Med., 177: 249-256; and Johnson et al., 1992, J. Exp. Med., 175: 961-971), malaria (Hill et al., 1992, Nature, 360: 434-439) and malignant tumours such as melanoma (Van der Brogen et al., 1991, Science, 254: 1643-1647; and Young and Steinman, 1990, J. Exp. Med., 171: 1315-1332).

In yet another embodiment, the genetically modified *Bordetella* is used as a delivery vehicle for heterologous antigens as described above and thus the resulting *Bordetella*-primed dendritic cells of the invention may be useful *inter alia* for treatment or prophylaxis of a viral, bacterial or parasitic infection or for treatment of a condition such as cancer. Viral infections contemplated by the present invention include, but are not restricted to, infections caused by HIV, Hepatitis, Influenza, Japanese encephalitis virus, Epstein-Barr virus and respiratory syncytial virus. Bacterial infections include, but are not restricted to, those caused by *Neisseria* species, *Meningococcal* species, *Haemophilus* species *Salmonella* species, *Streptococcal* species, *Legionella* species and *Mycobacterium* species. Parasitic infections encompassed by the invention include, but are not restricted to, those caused by *Plasmodium* species, *Schistosoma* species, *Leishmania* species, *Trypanosoma* species, *Toxoplasma* species and *Giardia* species. Cancers which could be suitably treated in accordance with the practices of this invention include cancers of the lung, breast, ovary, cervix, colon, head and neck, pancreas, prostate, stomach, bladder, kidney, bone liver, oesophagus, brain, testicle, uterus, melanoma and the various leukemias and lymphomas.

The effectiveness of the immunisation may be assessed using any suitable technique. For example, CTL lysis assays may be employed using stimulated splenocytes or peripheral blood mononuclear cells (PBMC) on peptide coated or recombinant virus infected cells using $^{51}$Cr labelled target cells. Such assays can be performed using for example primate, mouse or human cells (Allen et al., 2000, J. Immunol. 164(9): 4968-4978 also Woodberry *et al., infra*). Alternatively, the efficacy of the immunisation may be monitored using one or more techniques including, but not limited to, HLA class I tetramer staining - of both fresh and stimulated PBMCs (see for example Allen *et al., supra*), proliferation assays (Allen *et al., supra*), ELISPOT assays and intracellular IFN-gamma staining (Allen *et al., supra*), ELISA Assays - for linear B cell responses; and Western blots of cell sample expressing the synthetic polynucleotides

In order that the invention may be readily understood and put into practical effect, particular preferred embodiments will now be described by way of the following nonlimiting examples.

## EXAMPLES

### *EXAMPLE 1*

*Bacterial strains, plasmids, media and growth conditions*

A complete list of bacterial strains and plasmids used in this study is provided in Table 1. *E. coli* strains were routinely cultured in LB broth or on LB agar (Oxoid) overnight at 37° C. *B. pertussis* was routinely grown in a modified version of Verwey liquid medium (Farrell 2000) or Stainer Scholte medium (Stainer & Scholte 1971) and on Bordet-Gengou (BG) agar (Becton Dickinson) containing 15% sterile-defibrinated sheep blood for 2-3 days at 35-37° C. Cohen-Wheeler (CW) agar (Cohen & Wheeler 1946) containing 10% sterile-defibrinated sheep blood and 10 mM $MgCl_2$ was used for *B. pertussis* growth during conjugation experiments.

***TABLE 1***

| *Bacterial Strains and plasmids* | | |
|---|---|---|
| *Strains* | *Relevant properties* | *Source or reference* |
| Bacteria | | |
| E.coli | | |
| SM10λpir | Mobilising strain, recA::RP4-2-Tc::Mu | Roberts *et al.* (1990) |
| JM101 | Cloning host | |
| DH5α | Cloning host | |
| 583/90 | Cloning host, aroD mutant | |
| B. pertussis | | |
| ATCC9340 | $vir^+$ | Rosetti 1997 |
| BP304 | Tohama I spontaneous mutant, $Sm^R$, $vir^+$ | |
| Tohama I | $vir^+$ | 1950's Japan/ vaccine |
| Plasmids | | |
| pNEB193 | Cloning vector, $Ap^R$, LacZ | New England Biolabs, Inc. |
| pUC4K | Source of $Km^R$ cassette | Pharmacia Biotech |
| pRTP1 | $Ap^R$, rpsL, oriT, cos | Stibitz, Black & Falkow (1986) |

Cyclodextrin solid media (Imaizumi *et al.* 1983) was modified by removing the casamino acid component and increasing the amount of monosodium glutamate to 17 g (Frohlich *et al.* 1995). This modified CSM together with M9 minimal agar (Difco), with or without aromatic compounds (aromix), was used to test for the $aro^-$ phenotype of *B. pertussis* or *E. coli* mutants respectively. The final concentrations of aromix consisted of 40 μg/mL each of tryptophan, tyrosine and phenylalanine; and 10 μg/mL each of dihydroxybenzoic acid and *para*-aminobenzoic acid.

All antibiotic concentrations used for selection were as follows: kanamycin- 50 μg/mL, streptomycin-200 μg/mL and ampicillin- 100 μg/mL.

***EXAMPLE 2***

*Preparation and manipulation of DNA*

Extraction of plasmid DNA from *E. coli* strains or agarose gels was accomplished using a Prep-A-Gene DNA purification Kit (BioRad). Genomic DNA (gDNA) was purified from *B. pertussis* using a BioRad Genomic DNA Isolation Kit. All DNA manipulations were carried out using the protocols described elsewhere (Sambrook *et al.,* 1989). Restriction endonucleases, T4 DNA ligase and alkaline phosphatase were purchased from either MBI Fermentas, New England Biolabs or Amersham Pharmacia Biotech and were used according to the manufacturers recommendations. The PCR kit used was purchased from Fisher Biotec.

***EXAMPLE 3***

*Characterisation of the aroQ gene*

To detect whether *B. pertussis* possessed the *aroD* gene from the aromatic biosynthetic pathway, its genomic library was electroporated into the *aroD* mutant, *E. coli* 583/90. During these attempts an isolate was found which restored the mutant to wild type *E. coli* and allowed it to grow on media lacking aromatic compounds. It was assumed that the plasmid rescuing the isolate contained the *aroD* gene.

Restriction digestion and electrophoresis were used to determine an insert size of 1.5 Kb. To facilitate sequencing, the plasmid was further digested to produce a smaller 1 Kb fragment. This plasmid was subsequently named pUSQBord4 (Figure 1). Analysis of the pUSQBord4 insert sequence revealed that the approximate 500 bp gene was not similar to the consensus sequence of the *aroD* gene. A BLAST search using ANGIS showed that *B. pertussis* possessed the *aroQ* gene similar to the catabolic pathway of the fungus, *Aspergillus nidulans.* The *aroQ* genes from several other microorganisms were used in the ANGIS PILEUP program to confirm the identity of this gene (Figure 2).

*Methods*

Cloning of the *aroQ* gene

To create a gene library, the *B. pertussis* chromosomal DNA was partially digested with the *Sau*3AI restriction endonuclease. Ligation was attempted as described elsewhere (Sambrook *et al.,* 1989) with the different sized gDNA fragments and *Bam*HI-cleaved pNEB193 which had been previously alkaline phosphatased.

The ligation mix was electroporated into the *aroD E. coli* 583/90 by electroporation using 2.5 kV and 2 mm gap cuvettes to yield a desired pulse length of 6 ms (BTX ECM 600 Electrocell manipulator, Novex Australia). M9 minimal agar plates supplemented with ampicillin (100 μg/ml) were used to select for transformants.

DNA sequencing

DNA sequencing service was provided by the Australian Genome Research Facility (Ritchie Laboratories, University of Queensland). Sequences were then analysed at the University of Southern Queensland using the ANGIS (Australian National Genome Information Service) program. Programs used within this site were the Blast, Mapping and Pileup programs.

***EXAMPLE 4***

*Generation of a mutation in the aroQ gene*

The cloned *aroQ* gene was inactivated *in vitro* by double digesting the plasmid with the restriction enzymes *Ngo*MIV and *Bss*HI to remove a large portion of the gene (300 bp) as well as two *Bss*HI-*Bss*HI fragments (719 bp and 44 bp - see Figure 1). A kanamycin resistance cassette derived from pUC4K was amplified by the polymerase chain reaction (PCR) using primers with *Ngo*MIV and *Bss*HI restriction ends to create a fragment size of approximately 940 bp. This *Ngo*MIV-*Bss*HI cassette was then ligated into pUSQBord4 to replace the gene portion removed by digestion. This new plasmid was named pUSQBord7. The 719 bp *Bss*HI-*Bss*HI fragment was then re-ligated back into the plasmid and subsequently re-named pUSQBord8 after confirmation of the correct orientation of the fragment. This plasmid was then digested with *Eco*RI and *Bam*HI to break it into its original components (i.e. the insert and pNEB193) so that the *Eco*RI-*Bam*HI insert could be moved into a suicide vector.

In order to move the inactivated *aroQ* gene into *B. pertussis,* a suicide vector and mobilising strain were required. pRTP1 is mobilisable due to trans-acting factors encoded by chromosomal RP4 sequences present in *E. coli* SM10λ*pir* . pRTP1 replicates in this strain due to the presence of the *pir* gene product carried on a lysogenic phage (Roberts *et al.* 1990). The lack of this gene in *B. pertussis* provides the ideal conditions necessary for plasmid suicide, following conjugation. The gene for the *E. coli* S12 ribosomal protein is also encoded by the plasmid, which renders the Streptomycin resistant ($Sm^R$) host Sm sensative ($Sm^S$) (Stibitz *et al.* 1986). The *Eco*RI-*Bam*HI insert mentioned above was ligated directly into pRTP1 to produce third plasmid pUSQBord9.

To increase the chance of a recombinant event occurring it was necessary to add a scaffold sequence from the original 1.5 Kb fragment, upstream from the inactivated *aroQ* locus (located in pUSQBord1 not shown). A triple fragment ligation was performed (Figure 3) to produce a new shuttle vector ready for conjugation. Following electroporation of the complete plasmid into *E. coli* SM10λ*pir,* kanamycin resistant ($Km^R$) and ampicillin resistant ($Ap^R$) transconjugants were isolated on LB agar with appropriate antibiotics.

This new plasmid called pUSQBord10 was used to transform *E. coli* 583/90 to check whether the *aroQ* gene had been inactivated. Results showed that transformants could not grow without supplements of aromix, indicating that the gene was inactive.

*E. coli* SM10λ*pir* harbouring pUSQBord10 was conjugated with *B. pertussis* BP304 on CW agar and transconjugants were selected on CW agar containing kanamycin and streptomycin. It was assumed that *B. pertussis* transconjugates exhibiting $Km^R$ had incorporated the mutated *aroQ* gene into their genome. This should have occurred due to a crossover involving the remaining *aroQ* sequence flanking the $Km^R$ gene.

To verify that only the donor fragment has been incorporated and not the entire plasmid, $Km^R$ isolates were subcultured onto CW agar with streptomycin to again check for $Sm^R$. If vector integration had occurred *via* a single crossover, then the S12 allele on pRTP1 renders the isolates $Sm^s$ (Roberts *et al.* 1990).

*Methods*

PCR methods

For the kanamycin resistance cassette PCR, 2 μL of template DNA was added to 48 μL of master mixture (made immediately upon use) containing 5 μL of 10X PCR buffer; 10 μL of dNTPs h(2 mM); 2 μL $MgCl_2$ (50 mM); 20 pmoles (each) of primers FORKAN and BACKAN as set forth in Table 2; and 2.5 U of *Taq* polymerase. Amplification was performed in a programmable thermal controller (PTC-100, MJ Research inc.) with the following parameters: 5 min at 94° C followed by 5 initial cycles of 30 s at 35° C, 1 min at 72° C, and 30 s at 94° C; and 30 cycles of 30 s at 55° C, 1 min at 72° C, and 30 s at 94° C.

***TABLE 2***

| PCR Primers | |
|---|---|
| Primer Name | 5' → 3' Primer Sequence |
| FORKAN | GTGCCGGCGTGAATCTCTGATGTTACATTG |
| BACKAN | GGGCGCGCACTAGTGTTACAACCAATTAAC |
| FORQ | ATGGCGCAACGCATTCTTGT |
| BACQ2 | GTTTTGAGTTTTCGGAGGTC |

Bacterial conjugation

The method described by Roberts *et al.* (1990) was used for the conjugal transfer of DNA from *E.coli* to *B. pertussis.* Briefly, after 72 hours growth *B. pertussis* BP304 was swept from BG agar specifically using a sterile dacron swab and resuspended in phosphate buffered saline (PBS). The optical density of the suspension at 630 nm was determined and adjusted to an absorbance of 0.4. This procedure was repeated using *E. coli* SM10λ*pir* containing pUSQBord10 (see results section) except that growth was swept from LB agar supplemented with kanamycin.

A mixture of the above suspensions was made at a ratio of 10:1 1 and 100:1 *B. pertussis* to *E.coli* cells. These were placed onto sterile nitrocellulose resting on a foundation of CW agar and incubated at 37° C for 4 hours. Following incubation, growth was again swept from each piece of nitrocellulose and resuspended as above. Final suspensions of bacteria were plated onto CW agar supplemented with kanamycin, streptomycin and aromix. Plates were incubated for up to 5 days at 35-37° C.

***EXAMPLE 5***

*Confirmation of the transconjugants for aromatic-dependence*

Transconjugants displaying both $Km^R$ and $Sm^R$ were verified using CW agar plates with and without aromix. *B. pertussis aroQ* mutant colonies grown on medium with aromix were found to have a slower growth rate than that of the wild-type. Mutant colonies failed to grow without supplements of aromix.

To investigate the genotypic properties of the *aroQ* mutant, PCR incorporating *aroQ* gene primers was used to amplify a fragment containing the *aroQ* gene with an approximate size of 500 bp in the parental strain. It can be seen in Figure 4 that the *B. pertussis aroQ* mutant has a fragment size of 1150 bp This increase in fragment size approximately corresponds to the size of the *aroQ* fragment (500 bp) minus the 300 bp deletion plus the insertion of the 940 bp $Km^R$ cassette. To confirm this result, the 1150 bp fragment was extracted from an agarose gel and PCR with $Km^R$ gene primers was found to amplify the $Km^R$ gene (results not shown).

*Methods*

PCR methods

For the *aroQ* PCR, 1 μL of template DNA was added to 49 μL of master mix containing 5 μL of 10X PCR buffer; 5 μL of dNTPs (2 mM); 1.5 μL $MgCl_2$ (50 mM); 10 pmoles (each) of primers FORQ and BACQ2 as set forth in Table 2, *supra;* 5 μL 10X PCR Enhancer (Gibco BRL); and 2.5 U of *Taq* polymerase. Amplification was performed with the following parameters: 1 min at 95° C followed by 30 cycles of 45 s at 95° C, 30 s at 42° C and 1 min at 68° C.

***EXAMPLE 6***

*Determination of the presence or production of the major virulence factors by the aroQ mutant of B. pertussis*

Experiments carried out to determine the presence of the major virulence determinants including FHA, PT and pertactin, also included in the currently marketed acellular vaccine (PT is detoxified), in the *aroQ B. pertussis* revealed that all the above factors were present in or generated by the *aroQ* mutant vaccine strain (Table 3).

***TABLE 3***

| *Screening for the presence of FHA, PT and the 69 kD pertactin in the aroQ B. pertussis by western blotting using specific monoclonal antibodies* | | |
|---|---|---|
| *Fraction tested* | *Supernatant* | *Bacterial pellet* |
| FHA | + | + |
| PT | + | - |
| Pertactin | - | + |

***EXAMPLE 7***

*Safety of the aroQ mutant in vivo in mice*

Mice were inoculated with 6 X $10^7$, 6 X $10^8$ or 6 X $10^9$ CFUs of the *aroQ* mutant microorganisms by the intranasal route and observed over a period of 3 weeks for any morbidity or mortality. None of the vaccinated mice were found to suffer any ill effects (Figures 5A and 5B). In comparison, in mice inoculated with 9.3 X $10^6$ CFU of virulent *B. pertussis,* the virulent microorganisms were detectable in high numbers in the lungs of infected mice (Figure 5C).

*Method*

Determination of safety

In the first experiment, a total of 15 mice were inoculated with 6x$10^7$, 6x$10^8$ or 6x$10^9$ colony-forming units (CFUs) of the *aroQ* mutant microorganisms by the intranasal route and observed over a period of 4 weeks for any morbidity or mortality. In the second experiment, 24 mice were vaccinated with 8x$10^8$ CFU mutant microorganisms and their clearance from the lungs monitored at 1, 3, 5, 7, 14 and 21 days post-vaccination by the intranasal route. In another experiment, samples were taken at 2, 4, 6, 8, 10, 12 and 14 day intervals. Sera were also collected from mice in the second experiment for determination of antibodies and interleukin 12 (IL-12) levels.

***EXAMPLE 8***

*Persistence of the aroQ B. pertussis mutant in mice*

Two experiments were carried out. *B. pertussis* strains were grown on BG plates for 24-72 hrs, containing aromix when required. The bacteria were resuspended in PBS containing 1% casamino acids (*in vivo* grade). An absorbance of 0.1 at 630 nm was found to correspond to approximately 1 X $10^9$ colony-forming units (CFU) of the *B. pertussis aroQ* mutant and 3 X $10^9$ CFU of *B. pertussis* Tohama I. Ten-fold serial dilutions in PBS + 1% casamino acids were made to prepare vaccine and challenge doses. Dilutions were spotted onto BG plates using the standard Miles and Misra method to confirm the number of CFU.

Female 6-8 week old Balb/c mice were mildly sedated with xylazil (0.04 mL) and 20 $\mu$L of the bacterial suspension was placed onto the external nares of each mouse using a micropipette whereby the transfer from the nares to the respiratory tract of was achieved via inhalation by the mouse. At specific time intervals, depending upon the type of experiments, groups of mice were sacrificed and the trachea, lungs and spleen aseptically removed. The trachea and lungs were homogenised in 0.5ml PBS +1% casamino acids. 10-fold serial dilutions of each homogenate were plated onto BG agar (with aromix when required) using the standard Miles and Misra method and the number of CFU was determined after 2 days incubation at 37° C.

The results presented in Figures 5A and B show that the aroQ mutant was detectable in the lungs of mice up to day 11 post-administration by the intranasal route.

***EXAMPLE 9***

*The potential protective capacity of the aroQ B. pertussis mutant in mice*

The potential protective capacity of the *aroQ* mutant vaccine was assessed in mice, which had received 3 doses of the vaccine, by determining the rate of clearance of a sub-MMD50 dose ($2.0x10^8$) CFU of *B. pertussis* Tohama 1 strain from the lungs.

*Method and Results*

Experimental design for vaccination and challenge

A group of mice was vaccinated with either three doses of the mutant *aroQ* vaccine strain ($1x10^8$, $5x10^8$ and $1x10^9$ CFU) or the placebo solvent at days 1, 7 and 14 by the intranasal route. All mice were challenged on day 28 with virulent *B. pertussis* Tohama I by the intranasal route. It was discovered that the vaccinated mice had cleared the virulent microorganisms by day 6 post-challenge (Figure 6). On the other hand, the virulent microorganisms were isolated from the lung homogenates of placebo vaccinated group of mice until day 30 post-challenge (Figure 6). Sera and lung homogenates from these mice were collected for determination of total antibody levels and isotypes. Antigens used were killed whole cells of *B. pertussis* in the Indirect ELISA or purified FHA or PT in the Western dot blotting assays. Serum IL-12 levels were determined using commercial sandwich ELISA kits (Biosource). Determination of IL-2, IL-5 and INF-$\gamma$ in splenocyte culture supernatants obtained after stimulation with either FHA or chemically-inactivated pertussis toxin (PTxoid) were also carried out using commercially available sandwich ELISA kits (Biosource).

***EXAMPLE 10***

*Serum antibody and IL-12 response of mice vaccinated with the aroQ B. pertussis in mice*

Analysis of sera from the safety experiment described in Example 7 revealed that all mice immunised with the *aroQ B. pertussis* mutant developed antibodies against FHA and PT (Table 5).

***TABLE 5***

| *FHA- and PT-specific serum antibody titres in mice immunised with the aroQ B. pertussis mutant* | | |
|---|---|---|
| | *Antibody titre against* | |
| *Day post-immunisation* | *FHA* | *PT* |
| 1 | >50 | ND* |
| 3 | >50 | ND* |
| 5 | >50 | ND* |
| 7 | >50 | >50 |
| 14 | >50 | >50 |
| ND*: Not detectable at a dilution of 1/50 of the serum sample | | |

However anti-PT antibody titre was greater than 50 on days 5 and 7 post-vaccination but not detectable at that dilution of the serum on days 1, 3 and 5 post-immunisation and as such probably less than 50. There was also an induction of a serum IL-12 response beginning day 3 and reaching a reasonably high value at day 5 post-immunisation (data not shown). It is clear, however, that three vaccine doses are required to generate a significant anti-*B. pertussis* IgA and IgG in the lung homogenates although vaccination with one dose was sufficient to initiate the induction of the serum IgG response (Table 6A).

***TABLE 6A***

| *Antibody titres against whole-cell* B. pertussis *Tohama I in pooled mouse samples* | | | | | |
|---|---|---|---|---|---|
| Schedule: | Lung homogenates | | Serum | | |
| | IgA | IgG | IgA | IgG | Polyvalent |
| 7 days post 1 vaccination | 0 | 0 | 0 | 11 | 25 |

(continued)

| Antibody titres against whole-cell B. pertussis Tohama I in pooled mouse samples | | | | | |
|---|---|---|---|---|---|
| Schedule: | Lung homogenates | | Serum | | |
| | IgA | IgG | IgA | IgG | Polyvalent |
| 7 days post 2 vaccinations | 0 | 0 | 60 | 167 | 125 |
| 7 days post 3 vaccinations | 240 | 52 | 80 | 650 | 600 |
| 14 days post 3 vaccinations | 640 | 84 | 80 | 779 | 1280 |
| 21 days post 3 vaccinations | 960 | 974 | 800 | >1067 | 1625 |
| 28 days post 3 vaccinations | 320 | ND | 460 | >1067 | >2560 |
| 35 days post 3 vaccinations | 2080 | ND | 320 | >1067 | 825 |
| Values represent reciprocal end point titres<br>ND = not determined | | | | | |

It can be seen from the Table 6A that the titre of the antibodies of the IgA isotype was significant in mice immunised with three doses of the attenuated vaccine post-challenge with virulent *B. pertussis.*

***TABLE 6B***

| *Antibody titres against whole-cell B. pertussis Tohama I in pooled samples from control and vaccinated mice post-challenge* | | | | | | | | |
|---|---|---|---|---|---|---|---|---|
| | Lung homogenates | | | | Serum | | | |
| | IgA | | IgG | | IgA | | IgG | |
| Days post Challenge | control mice | vaccinated mice | control mice | vaccinated mice | control mice | vaccinated mice | control mice | vaccinated mice |
| -1 | 0 | >1200 | 0 | 990 | 0 | 29 | 0 | >960 |
| 2 | 0 | 1200 | 0 | 2550 | 0 | 21 | 0 | >960 |
| 4 | 0 | 1200 | 0 | 1190 | 0 | 253 | 0 | >640 |
| 6 | 0 | >1200 | 0 | >1270 | 0 | 213 | 0 | >640 |
| 8 | 0 | >1200 | 20 | >1270 | 0 | 253 | 0 | >640 |
| 10 | 1200 | >1200 | 70 | >1270 | 17 | 157 | 133 | >640 |
| 12 | ND | ND | ND | ND | 101 | 79 | 160 | >640 |
| 14 | 1200 | >1200 | 290 | >1270 | 77 | 93 | >960 | >640 |
| 16 | 1200 | >1200 | 630 | >1270 | 29 | 65 | >960 | >640 |
| 21 | >1200 | >1200 | 630 | >1060 | 37 | 39 | >960 | >640 |
| 30 | >1200 | >1200 | 750 | >1270 | 37 | 237 | >960 | >640 |
| -1 = pre-challenge<br>Values represent reciprocal end point titres | | | | | | | | |

There was no antibody response in control mice post-challenge until day 8 post-challenge (Table 6B). However the IgA antibody titres in the lung homogenates *versus* the serum IgA titres of vaccinated mice were significantly greater at all intervals post-challenge. Furthermore the IgG antibody levels in the lung homogenates could be considered to be at least at the same level, if not greater, than its serum IgG antibody counterpart.

On the other hand, mice vaccinated with an acellular vaccine (DTaP; Infantrix™) showed no IgA production with only small amount of IgG in the lung homogenates but with significant amounts of IgG in the serum at day 28 post-immunisation with 1/10$^{th}$ the human dose at day 1 and 21 (data not shown).

*Method*

Determination of antibody and interleukin 12 (IL-12) levels in the sera of vaccinated mice

Antibody titres of sera against *B. pertussis* were determined against the 2 most significant virulence factors, purified FHA and PT, in dot blot assays using the procedure described by Towbin *et al.* (1979) except that no SDS-PAGE (sodium dodecyl polyacrylamide gel electrophoresis) was carried out. Determination of IL-12 was carried out using a commercial kit (BioSource, Australia). The level of IL-12 was accomplished using commercial kit (Biosource, Australia). The anti-IgG and IgA antibody titres against killed whole B pertussis cells were determined using a standard indirect ELISA assay.

Determination of the presence or production of FHA, PT or Pertactin

Determination of the production of FHA, PT and pertactin by the mutant strain was carried out using specific monoclonal antibodies by western blotting essentially as described by Towbin *et al* (1979). Briefly the *aroQ* mutant of *B. pertussis* was grown in Stainer and Scholte medium for a period of 48-72 hours and subjected to centrifugation at 4000xg for 10 mins. The supernatant concentrated by pervaporation and the lysed bacterial sediment subjected to SDS-PAGE followed by western blotting. The blot was developed using monoclonal antibodies against FHA, PT or pertactin (obtained through the courtesy of CSL Ltd, Parkville).

***EXAMPLE 11***

*Determination of the interleukins IL-2 and INF-γ in the antigen stimulated splenocyte culture supernatants*

Both the interleukin-2 and interferon-γ (IFN-γ) titres were determined using a Cytoscreen Mouse IFN-γ kit (Biosource International, Camarillo, CA). The procedure was conducted as per instructions. A standard curve was plotted from the standard data, and interleukin concentrations for the splenocyte supernatants calculated. The antigens used for stimulation of the splenocyte cultures were chemically-inactivated pertussis toxoid or pure filamentous hamagglutinin (FHA).

The results presented in Figures 7 and 8 were obtained from pooled spleen samples from both the control and vaccinated mice pre- and post-challenge. There was significant production of IL-2 following challenge at days 10, 14, 21 and day 30 post-challenge with PTxoid. There was also a significant production of IL-2 at days 10 and 30 post-challenge with FHA. The low response at day 14 post-challenge was not expected and as such it is considered to be an operator error. There was significant production of INF-γ in the vaccinated mice pre- and post-challenge, indicating the induction and promotion of Th1 type responses considered to be responsible for long term protection (see Figure 8).

***DISCUSSION OF THE EXAMPLES***

During an attempt to clone the *aroD* gene from the *B. pertussis* genome, a fragment containing a gene with amino acid sequence homology to the *aroQ*-encoded 3-3-dehydroquinase enzyme of *Actinobacillus pleuropneumoniae* (Lalonde *et al.* 1994), reported previously to have homology with the eukaryotic genes in the quinic acid catabolic pathway of *Aspergillus nidulans,* was discovered (Rosetti 1997). Even though the sequence of the gene indicated its involvement in the catabolic pathway, the fact that it rescued the *E. coli aroD* mutant suggested that it was capable of functioning in the aromatic amino acid biosynthetic pathway. PCR with *aroD* primers also confirmed that *B. pertussis* did not contain an *aroD* gene, supporting the concept that there was only one gene encoding a 3-dehydroquinase in *B. pertussis.*

The sequence discovered by Rosetti (1997) was confirmed in a BLAST search using the published complete genome sequence of *Bordetella pertussis* [ORF 5420 (http://pedant.gsf.de/cgi bin/wwwfly.pl?Set=Bordetella_pertussis_Tohama_I&Page=index) Last updated: 2002-01-07 14:25:19].

Roberts *et al.* (1990) reported the construction of an *aroA* mutant of *B. pertussis* which was found to be effective in eliminating small numbers of virulent challenge parent microorganisms (ranging from $10^3$-$10^4$ CFU per mouse) from the lungs of mice vaccinated with 3 doses of the mutant strain by the intranasal route. This strain was apparently considered to be unsuitable as a vaccine candidate because of its complete disappearance from most immunised mice by essentially day 4 post-aerosolisation into the lungs.

The *aroQ* mutant generated in this investigation was found to survive in the reasonable numbers in all vaccinated mice at least until day 11 and in some mice until day 12 post-intranasal instillation. Challenge of mice immunised with three doses of the *aroQ* vaccine resulted in complete clearance of the virulent strain at day 6 post-challenge. The number of challenge microorganisms administered to mice in this investigation was nearly 500-fold higher than the *aroA* mutants lodged in the mouse lungs in experiments conducted by Roberts *et al.* (1990). Since the challenge dose administered to mice by Roberts *et al.* (1990) was via aerosolisation of the *B. pertussis* culture, it is difficult to compare the present data with theirs. In fact Guiso *et al.* (1999) have reported that the intranasal mode of administrating the challenge microorganism, also employed in this investigation, is a better predictor of protection in human infants than the aerosolisation method used by Roberts *et al.* (1990). Investigators from the Bordetella Laboratory, Pasteur Institute and SmithKline Beecham Biologicals (Guiso *et al.* 1999) reported that the tricomponent acellular vaccine containing FHA, PT (Toxoid) and pertactin (PRN) were significantly superior at clearing bacteria from the lungs of infected mice than the bicomponent vaccines containing FHA and PRN. These authors also reported that the efficiency of bacterial clearance was dose-dependent. The bacterial clearance pattern was almost identical to that reported by Guiso *et al.* (1999) and followed a similar trend in mice immunised with the *aroQ* mutants in this investigation. Mice immunised with the *aroQ B. pertussis* mutants also elicited antibodies against the two major virulence factors, FHA and PT, tested in this investigation. However since the *aroQ* mutant also possessed pertactin, an antibody response is also expected to be generated against this protein as well in immunised mice. The generation of CMI response in mice immunised with the *aroQ B. pertussis* before or after challenge with virulent *B. pertussis* is clearly an advantage not reported previously with any other live attenuated mucosal vaccine against whooping cough.

An attenuated strain of *B. pertussis,* designated BPRA, with a chromosomal deletion in the pertussis toxin gene was reported recently to be successful in producing long-lasting protection in the mouse model (Mielcarek *et al.* 1998). However, the protection observed was not equivalent to that induced by the wild-type *B. pertussis* (Mielcarek *et al.* 1998). This result indicated that even though pertussis toxin was not essential for protection, it definitely played an important role in protection against whooping cough. The *aroQ* mutant generated and tested in this investigation was found to produce FHA, PT and PRN and as such constitutes an ideal vaccine strain for further development and evaluation as a potential vaccine against whooping cough. For example, in accordance with the information described herein, it is possible to construct a vaccine strain that not only incorporates two genetically unlinked mutations in order to prevent reversion of the mutant strain to a wild-type phenotype but also to detoxify the major toxins such as pertussis toxin and adenylate cyclase. It is also possible to raise therapeutic antibodies using standard methodologies for treating infections caused by *Bordetella* species, or related organisms.

In summary, the genetically modified *Bordetella* strain of the present invention, when administered by the noninvasive intranasal route, has the following properties:

- The strain enhances the level of IL-2, IL-12 and IFN-$\gamma$ post challenge (IFN-$\gamma$ present pre-challenge) indicating a Th1 response;
- IL-12 induces the synthesis of IFN-$\gamma$, induces growth of Tc and B cells and activates NK cells;
- IFN-$\gamma$ produced by Th1 cells is a potent activator of NK cells and macrophages and is necessary for clearance of *B. pertussis* from the lungs; and
- high levels of the IgA type antibodies in the lung homogenates and significant production of interleukins 2 and IFN-$\gamma$, the use of the attenuated vaccine strain appears to be an ideal potential vaccine against whooping cough.

The citation of any reference herein should not be construed as an admission that such reference is available as "Prior Art" to the instant application

Throughout the specification the aim has been to describe the preferred embodiments of the invention without limiting the invention to any one embodiment or specific collection of features. Those of skill in the art will therefore appreciate that, in light of the instant disclosure, various modifications and changes can be made in the particular embodiments exemplified without departing from the scope of the present invention. All such modifications and changes are intended to be included within the scope of the appended claims.

**BIBLIOGRAPHY**

Baraff, L. J., C. L. Cody, and J. D. Cherry. 1984. DTP associated reactions: an analysis by injection site, manufacturer, prior reactions and dose. Pediatrics, 73: 31-36.

Canthaboo, C., L. Williams, D. K. L. Xing, and M. J. Corbel. 2000. Investigation of cellular and humoral immune responses to whole cell and acellular pertussis vaccines. Vaccine, 19: 637-643.

Cohen, S.M. and M.W. Wheeler. 1946. Pertussis vaccine prepared with phase-I cultures grown in fluid medium.

Am. J. Pub. Health. 36: 371

Ewanowich, C. A., A.R. Melton, A. A. Weiss, R. K. Sherburne, and A. Peppler. 1989. Invasion of HeLa 299 cells by virulent Bordetella pertussis. Infec. Immun. 57: 2698-2704.

Farrell, D. J. 2000. Bordetella pertussis: diagnosis, infection and immunity. PhD Dissertation; University of Southern Queensland.

Frohlich, B. T., E. R. De Bernardez Clark, G. R. Siber, and R. W. Swartz. 1995. Improved pertussis toxin production by Bordetella pertussis through adjusting the growth medium's ionic composition. J. Biotech. 39: 205-219.

Galazka, A. 1992. Control of pertussis in the world. World. Health Stat. Q. 45: 238-247.

Guiso, N., C. Capiau, G. Carletti, J. Poolman, and P. Hauser. 1999. Intranasal murine model of protection in human infants by acellular vaccines. Vaccine, 17: 2366-2376.

Hoiseth, S. K., and B. A. D. Stocker. 1981. Aromatic-dependent Salmonella typhimurium are non-virulent and effective as live vaccines. Nature (London). 291: 238-239.

Howson, C. P., and H. V. Fineberg. 1992. Adverse events following pertussis and rubella vaccines. JAMA. 267: 392-396.

Imaizumi, A., Y. Suzuki, S. Ono, H. Sato, and Y. Sato. 1983. Heptakis (2,6-O-dimethyl)β-cyclodextrin: a novel growth stimulent for Bordetella pertussis phase I. J. Clin. Microbiol. 17: 781-786.

Lalonde, G., P. D. O'Hanley, B. A. D. Stocker, and K. T. Denich. 1994. Characterisation of a 3-dehydroquinase gene from Actinobacilus pleuropneumoniae with hology to the eukaryotic genes qa-2 and QUTE. Mol. Microbiol. 11: 273-280.

Manetti, R., P. Parronchi, M. G. Giudizi, M. P. Piccinni, E. Maggi., G. Trinchieri., and S. Romagnani. 1993. Natural killer cell stimulatory factor (interleukin 12 [IL-12]) induces T helper type 1 (Th1)-specific immune response and inhibits the development of IL-4-producing Th cells. J. Exp. Med. 177: 199-204.

Marble, M., and K. K. Key. 1996. Bioengineered Bordetella pertussis used for intranasal immunisation. Disease Weekly Plus. 8/9/96, 23-24.

Mielcarek, N., G. Riveau, F. Remoué, R. Antoine, A. Capron, and C. Locht. 1998. Homologous and heterologous protection after single intranasal administration of live attenuated recombinant Bordetella pertussis. Nature Biotech. 16: 454-457.

Mills, K. H. G., A. Barnard, J. Watkins, and K. Redhead. 1993. Cell-mediated immunity to Bordetella pertussis: role of Th1 cella in bacterial clearance in a murine respiratory infection model. Infect. Immun. 61: 399-410.

Mills, K.H.G., 2001. Immunity to Bordetella pertussis. Microbes Infect 3: 655-677.

Mukkur, T. K. S., G. H. McDowell, B. A. D. Stocker, and A. K. Lascelles. Protection against experimental salmonellosis in mice and sheep by immunisation with aromatic-dependent Salmonella typhimurium. J. Med. Microbiol. 24: 11-19.

Pollock, T. M., J. Y. Mortimer, E. Miller, and G. Smith. 1984. Symptoms after primary immunisation with DTP and DT vaccine. Lancet, ii: 146-149.

Redhead, K., J. Watkins, A. Barnard, K.H.G. Mills. 1993. Effective immunization against Bordetella pertussis respiratory infection in mice is dependent on induction of cell-mediated immunity. Infect. Immun. 61: 3190-3198.

Rennels, M.B., Deloria, M.A., Pichichero, M.E., Losonsky, G.A., Englund, J.A., Mead, B.D., Anderson, E.L., Steinhoff, M.C. and Edwards, K.M. 2000. Extensive swelling after booster doses of acellular pertussis-tetanus-diphtheria vaccines. Pediatrics 1: e1-12.

Roberts, M., D. Maskell, P. Novotny, and G. Dougan. 1990. Construction and characterisation in vivo of Bordetella pertussis aroA mutants. Infect. Immun. 58: 732-739.

Rossetti, T. R. 1997. Cloning the aroD gene of Bordetella pertussis (BSc. Hons. Thesis). University of Southern Queensland, Toowoomba.

Sambrook, J., E. F. Fritsch, and T. Maniatis. 1989. Molecular Cloning: a laboratory manual, 2nd ed. Cold Spring Harbour Laboratory Press, Cold Spring Harbour, N.Y.

Saukkonen, K., C. Cabellos, M. Burroughs, S. Prasad, E. Tuomanen. 1991. Integrin mediated localization of Bordetella pertussis within macrophages: Role in pulmonary colonization. J. Exp. Med. 173: 1143-1149.

Simmons, C. P., S. J. Dunstan, M. Tachedjian, J. Krywult, A. L. Hodgson, and R. A. Strugnell. 1998. Vaccine potential of attenuated mutants of Corynebacterium pseudotuberculosis in sheep. Infect. Immun. 66: 474-479.

Stainer, D. W, and M. J. Scholte. 1971. A simple and chemically defined medium for the production of phase I Bordetella pertussis. J. Gen. Microbiology. 34: 778-784

Stibitz, S., G. W. Black, and S. Falkow. 1986. The construction of a cloning vector designed for gene replacement in Bordetella pertussis. Gene. 50: 133-140.

Towbin, H., T. Stachelin, and J Gordon. 1979. Electrophoretic transfer of proteins from polyacrylamide gels to nitrocellulose sheets: procedure and some applications. Proc. Natl. Acad. Sci., USA. 76: 4350-4354.

Verma, N.K., and A. A. Lindberg. 1991. Construction of aromatic dependent Shigella flexneri 2a live vaccine candidate strains: deletion mutations in the aroA and the aroD genes. Vaccine. 9: 6-9.

Weiss, A. A. and E. L. Hewlett. 1986. Virulence factors of Bordetella pertussis. Ann. Rev. Microbiol. 40: 661-686.

Zepp, F., M. Knuf, P. Habermehl, J. H. Scmitt, C. Rebsch, P. Schmidtke, R. Clemens and M. Slaoui. 1996. Pertussis-specific cell-mediated immunity in infants after vaccination with tricomponent acellular pertussis vaccine. Infect. Immun. 64: 4078-4084.

## Claims

**1.** A genetically modified *Bordetella* strain having a partial or complete loss of function in the endogenous *aroQ* gene and a lower capacity to propagate in a mammalian host but remaining viable in the host for a period of time sufficient to induce an immune response against a pathogenic *Bordetella* strain.

**2.** The genetically modified strain of claim 1, wherein the pathogenic strain is selected from *Bordetella avium, Bordetella bronchiseptica, Bordetella holmesii, Bordetella parapertussis* and *Bordetella pertussis.*

**3.** The genetically modified strain of claim 1, wherein the pathogenic *Bordetella* strain is *Bordetella pertussis.*

**4.** The genetically modified strain of claim 1, comprising a disruption in the endogenous *aroQ* gene.

**5.** The genetically modified strain of claim 4, wherein the disruption has been introduced into the genome of a pathogenic strain of *Bordetella* by homologous recombination with a DNA targeting construct such that the targeting construct is stably integrated in the genome, wherein the disruption of the *aroQ* gene results in a reduced level and/or functional activity of the 3-dehydroquinase.

6. The genetically modified strain of claim 4, comprising an exogenous nucleic acid sequence in its genome, or on an extrachromosomal element, which is capable of abolishing or otherwise reducing the expression of *aroQ* or the level and/or functional activity of the 3-dehydroquinase encoded by *aroQ*.

7. The genetically modified strain of claim 4, comprising an exogenous nucleic acid sequence in its genome, or on an extrachromosomal element, which is capable of abolishing or otherwise reducing the expression of *aroQ* or the level and/or functional activity of the 3-dehydroquinase encoded by *aroQ,* wherein the nucleic acid sequence comprises at least a portion of *aroQ,* in the sense or anti-sense orientation, which is operably linked to a transcriptional element.

8. The genetically modified strain of claim 4, comprising an exogenous nucleic acid sequence in its genome, or on an extrachromosomal element, which is capable of abolishing or otherwise reducing the expression of *aroQ* or the level and/or functional activity of the 3-dehydroquinase encoded by *aroQ,* wherein the nucleic acid sequence comprises a ribozyme-encoding polynucleotide that is operably linked to a transcriptional control element, wherein the ribozyme specifically binds to or otherwise interacts with a transcript of the *aroQ* gene.

9. The genetically modified strain of claim 1, further having a partial or complete loss of function in at least one other endogenous gene selected from a *pur* gene, another *aro* gene, a pertussis toxin gene, or any other gene which contributes to survival in the host and/or to bacterial virulence, or a combination thereof.

10. The genetically modified strain of claim 9, wherein the *pur* gene is selected from *purA, purE or purH.*

11. The genetically modified strain of claim 9, wherein the another *aro* gene is selected from *aroA, aroB, aroC* or *aroE.*

12. The genetically modified *Bordetella* strain of claim 1, comprising at least one exogenous gene which is capable for expressing an antigen that is heterologous or foreign to the *Bordetella* strain.

13. The genetically modified *Bordetella* strain of claim 12, wherein the heterologous or foreign antigen is derived from a pathogen that is not a *Bordetella* strain.

14. The genetically modified *Bordetella* strain of claim 12, wherein the heterologous or foreign antigen is derived from a pathogen that infects by the mucosal route.

15. An isolated polynucleotide comprising a nucleotide sequence that corresponds or is complementary to the sequence set forth in SEQ ID NO: 1 or 3, wherein said isolated polynucleotide encodes a polypeptide with 3-dehydroquinase activity.

16. An isolated polynucleotide comprising a nucleotide sequence having at least 80% sequence identity to at least a portion of the sequence set forth in SEQ ID NO: 3, wherein said portion is at least 150 nucleotides in length and wherein said isolated polynucleotide encodes a polypeptide with 3-dehydroquinase activity.

17. The isolated polynucleotide of claim 16, wherein the nucleotide sequence has at least 90% sequence identity.

18. An isolated polypeptide encoded by the isolated polynucleotide of claim 16 or claim 17, wherein said isolated polypeptide has 3-dehydroquinase activity.

19. An isolated polypeptide comprising an amino acid sequence as set forth in SEQ ID NO: 2, wherein said isolated polypeptide has 3-dehydroquinase activity.

20. An isolated polypeptide comprising an amino acid sequence that has at least 80% sequence identity to at least a 70 amino acid fragment of the sequence set forth in SEQ ID NO: 2, wherein said isolated polypeptide has 3-dehydroquinase activity.

21. The isolated polypeptide of claim 20, wherein said amino acid sequence has at least 90% sequence identity.

22. A nucleic acid construct for disrupting an *aroQ* gene in a *Bordetella* cell, comprising: a) a non-homologous replacement portion; b) a first homology region located upstream of the non-homologous replacement portion, the first homology region having a nucleotide sequence with substantial identity to a first *aroQ* gene sequence; and c) a second homology region located downstream of the non-homologous replacement portion, the second homology region having a nucleotide sequence with substantial identity to a second *aroQ* gene sequence, the second *aroQ* gene sequence having a location downstream of the first *aroQ* gene sequence in a naturally occurring endogenous *aroQ* gene of the *Bordetella* cell, wherein the *aroQ* gene comprises the isolated polynucleotide as set forth in any one of claims 15-17.

**23.** A vector comprising a nucleotide sequence that corresponds or is complementary to the isolated polynucleotide sequence as set forth in any one of claims 15 to 17.

**24.** The vector of claim 23, wherein the vector is a DNA targeting vector.

**25.** A host cell containing the construct of claim 22 or the vector of claim 23.

**26.** An antigen-binding molecule that is specifically interactive with the polypeptide of any one of claims 19 to 21.

**27.** A method for producing a genetically modified *Bordetella* strain, comprising introducing the nucleic acid construct of claim 22 into a *Bordetella* cell under conditions such that the nucleic acid construct is homologously recombined into the *aroQ* gene in the genome of that cell to produce a genetically modified *Bordetella* cell containing a disrupted *aroQ* gene.

**28.** The method of claim 27, wherein the genetically modified *Bordetella* cell containing the homologously recombined nucleic acid construct is further **characterised by** expressing reduced or undetectable levels of *aroQ.*

**29.** The method of claim 27, wherein the genetically modified *Bordetella* cell lacks the ability to produce a functional 3-dehydroquinase encoded by said *aroQ* gene.

**30.** A composition, comprising the genetically modified *Bordetella* strain of claim 1, together with a pharmaceutically acceptable carrier.

**31.** The composition of claim 30, further comprising an adjuvant.

**32.** A composition of matter comprising dendritic cells which have been exposed to the genetically modified *Bordetella* strain of claim 1 for a time and under conditions sufficient to express a processed or modified antigen derived from the *Bordetella* strain for presentation to, and modulation of, T cells.

**33.** The composition of matter of claim 32, which is in the form of an *in vitro* cell culture.

**34.** Use of the genetically modified *Bordetella* strain of claim 1 in the manufacture of a medicament for the modulation of an immune response in a mammal.

**35.** Use of the genetically modified *Bordetella* strain of claim 1 in the manufacture of a medicament for the treatment and/or prophylaxis of whooping cough or related condition in a mammal.

**36.** The genetically modified *Bordetella* strain of claim 1 for use in the study, and modulation of an immune response.

**37.** The genetically modified *Bordetella* strain of claim 36, wherein the immune response is against a pathogenic strain of *Bordetella* or related organism.

**38.** The genetically modified *Bordetella* strain of claim 1 for use in the modulation of an immune response in a mammal.

**Patentansprüche**

**1.** Gentechnisch veränderter *Bordetella*-Stamm mit teilweisem oder vollständigem Funktionsverlust in dem endogenen *aroQ*-Gen und einer geringeren Fortpflanzungskapazität in einem Säugerwirt, jedoch mit einer noch bestehenden Lebensfähigkeit in dem Wirt über einen Zeitraum, der zur Induktion einer Immunantwort gegen einen pathogenen *Bordetella*-Stamm ausreicht.

**2.** Gentechnisch veränderter Stamm nach Anspruch 1, wobei der pathogene Stamm aus *Bordetella avium, Bordetella*

*bronchiseptica, Bordetella holmesii, Bordetella parapertussis* und *Bordetella pertussis* ausgewählt ist.

3. Gentechnisch veränderter Stamm nach Anspruch 1, wobei es sich bei dem pathogenen *Bordetella*-Stamm um *Bordetella pertussis* handelt.

4. Gentechnisch veränderter Stamm nach Anspruch 1, umfassend eine Unterbrechung in dem endogenen *aroQ*-Gen.

5. Gentechnisch veränderter Stamm nach Anspruch 4, wobei die Unterbrechung in das Genom eines pathogenen *Bordetella*-Stamms über homologe Rekombination mit einem DNA-Zielkonstrukt so eingeführt wurde, dass das Zielkonstrukt stabil in dem Genom integriert ist, wobei die Unterbrechung des *aroQ*-Gens zur einer verringerten Konzentration und/oder funktionellen Aktivität der 3-Dehydrochinase führt.

6. Gentechnisch veränderter Stamm nach Anspruch 4, umfassend eine exogene Nukleinsäuresequenz in seinem Genom oder auf einem extrachromosomalen Element, die in der Lage ist, die Expression von *aroQ* oder die Konzentration und/oder funktionelle Aktivität der von *aroQ* codierten 3-Dehydrochinase aufzuheben oder anderweitig zu verringern.

7. Gentechnisch veränderter Stamm nach Anspruch 4, umfassend eine exogene Nukleinsäuresequenz in seinem Genom oder auf einem extrachromosomalen Element, die in der Lage ist, die Expression von *aroQ* oder die Konzentration und/oder funktionelle Aktivität der von *aroQ* codierten 3-Dehydrochinase aufzuheben oder anderweitig zu verringern, wobei die Nukleinsäuresequenz wenigstens einen Anteil von *aroQ,* und zwar in Sense- oder Antisense-Orientierung, umfasst, der in operativer Verknüpfung mit einem Transkriptionselement steht.

8. Gentechnisch veränderter Stamm nach Anspruch 4, umfassend eine exogene Nukleinsäuresequenz in seinem Genom oder auf einem extrachromosomalen Element, die in der Lage ist, die Expression von *aroQ* oder die Konzentration und/oder funktionelle Aktivität der von *aroQ* codierten 3-Dehydrochinase aufzuheben oder anderweitig zu verringern, wobei die Nukleinsäuresequenz ein für Ribozym codierendes Polynukleotid umfasst, das in operativer Verknüpfung mit einem Transkriptionskontrollelement steht, wobei das Ribozym spezifisch an ein Transkript des *aroQ*-Gens bindet oder mit diesem eine anderweitige Wechselwirkung eingeht.

9. Gentechnisch veränderter Stamm nach Anspruch 1, mit einem weiteren teilweisen oder vollständigen Funktionsverlust in wenigstens einem anderen aus einem pur-Gen, einem weiteren *aro*-Gen, einem Pertussistoxin-Gen oder einem beliebigen anderen Gen, das zum Überleben in dem Wirt und/oder zu bakterieller Virulenz beiträgt, oder einer Kombination davon ausgewählten endogenen Gen.

10. Gentechnisch veränderter Stamm nach Anspruch 9, wobei das *pur*-Gen aus *purA, purE* oder *purH* ausgewählt ist.

11. Gentechnisch veränderter Stamm nach Anspruch 9, wobei das weitere aro-Gen aus *aroA, aroB, aroC* oder *aroE* ausgewählt ist.

12. Gentechnisch veränderter *Bordetella*-Stamm nach Anspruch 1, umfassend wenigstens ein exogenes Gen, das zur Expression eines für den *Bordetella*-Stamm heterologen oder fremden Antigens in der Lage ist.

13. Gentechnisch veränderter *Bordetella*-Stamm nach Anspruch 12, wobei das heterologe oder fremde Antigen aus einem Krankheitserreger stammt, bei dem es sich nicht um einen *Bordetella*-Stamm handelt.

14. Gentechnisch veränderter *Bordetella*-Stamm nach Anspruch 12, wobei das heterologe oder fremde Antigen aus einem Krankheitserreger stammt, dessen Infektionsweg über die Schleimhaut verläuft.

15. Isoliertes Polynukleotid, umfassend eine Nukleotidsequenz, die der in SEQ ID NO: 1 oder 3 angegebenen Sequenz entspricht oder dazu komplementär ist, wobei das isolierte Polynukleotid für ein Polypeptid mit 3-Dehydrochinase-Aktivität codiert.

16. Isoliertes Polynukleotid, umfassend eine Nukleotidsequenz mit einer Sequenzidentität von wenigstens 80% zu wenigstens einem Anteil der in SEQ ID NO: 3 angegebenen Sequenz, wobei der Anteil eine Länge von wenigstens 150 Nukleotiden aufweist und wobei das isolierte Polynukleotid für ein Polypeptid mit 3-Dehydrochinase-Aktivität codiert.

**17.** Isoliertes Polynukleotid nach Anspruch 16, wobei die Nukleotidsequenz eine Sequenzidentität von wenigstens 90% aufweist.

**18.** Isoliertes Polypeptid, codiert von dem isolierten Polynukleotid nach Anspruch 16 oder Anspruch 17, wobei das isolierte Polypeptid 3-Dehydrochinase-Aktivität aufweist.

**19.** Isoliertes Polypeptid, umfassend eine wie in SEQ ID NO: 2 angegebene Aminosäuresequenz, wobei das isolierte Polypeptid 3-Dehydrochinase-Aktivität aufweist.

**20.** Isoliertes Polypeptid, umfassend eine Aminosäuresequenz, die eine Sequenzidentität von wenigstens 80% zu wenigstens einem 70 Aminosäuren langen Fragment der in SEQ ID NO: 2 angegebenen Sequenz aufweist, wobei das isolierte Polypeptid 3-Dehydrochinase-Aktivität aufweist.

**21.** Isoliertes Polypeptid nach Anspruch 20, wobei die Aminosäuresequenz eine Sequenzidentität von wenigstens 90% aufweist.

**22.** Nukleinsäurekonstrukt zur Unterbrechung eines aroQ-Gens in einer *Bordetella*-Zelle, umfassend: a) einen Nichthomologer-Ersatz-Anteil; b) einen stromaufwärts des Nichthomologer-Ersatz-Anteils liegenden ersten Homologiebereich, der eine Nukleotidsequenz mit weitgehender Identität zu einer ersten *aroQ*-Gensequenz aufweist; und c) einen stromabwärts des Nichthomologer-Ersatz-Anteils liegenden zweiten Homologiebereich, der eine Nukleotidsequenz mit weitgehender Identität zu einer zweiten *aroQ*-Gensequenz aufweist, wobei die zweite *aroQ*-Gensequenz in einem natürlich vorkommenden endogenen *aroQ*-Gen der *Bordetella*-Zelle stromabwärts zu der ersten *aroQ*-Gensequenz liegt, wobei das *aroQ*-Gen das wie in einem der Ansprüche 15-17 angegebene isolierte Polynukleotid umfasst.

**23.** Vektor, umfassend eine Nukleotidsequenz, die der wie in einem der Ansprüche 15 bis 17 angegebenen isolierten Polynukleotidsequenz entspricht oder dazu komplementär ist.

**24.** Vektor nach Anspruch 23, bei dem es sich um einen DNA-Zielvektor handelt.

**25.** Wirtszelle, enthaltend das Konstrukt nach Anspruch 22 oder den Vektor nach Anspruch 23.

**26.** Antigenbindendes Molekül, das spezifisch mit dem Polypeptid nach einem der Ansprüche 19 bis 21 wechselwirkt.

**27.** Verfahren zur Herstellung eines gentechnisch veränderten *Bordetella*-Stamms, umfassend das Einführen des Nukleinsäurekonstrukts nach Anspruch 22 in eine *Bordetella*-Zelle unter solchen Bedingungen, bei denen eine homologe Rekombination des Nukleinsäurekonstrukts in das *aroQ*-Gen in dem Genom der Zelle erfolgt, so dass eine gentechnisch veränderte *Bordetella*-Zelle mit einem unterbrochenen aroQ-Gen erhalten wird.

**28.** Verfahren nach Anspruch 27, wobei die gentechnisch veränderte *Bordetella*-Zelle mit dem homolog rekombinierten Nukleinsäurekonstrukt ferner durch die Expression verringerter oder nicht nachweisbarer Konzentrationen an *aroQ* gekennzeichnet ist.

**29.** Verfahren nach Anspruch 27, wobei der gentechnisch veränderten *Bordetella*-Zelle die Fähigkeit fehlt, eine von dem *aroQ*-Gen codierte funktionsfähige 3-Dehydrochinase zu produzieren.

**30.** Zusammensetzung, umfassend den gentechnisch veränderten *Bordetella*-Stamm nach Anspruch 1 zusammen mit einem pharmazeutisch unbedenklichen Trägerstoff.

**31.** Zusammensetzung nach Anspruch 30, ferner ein Adjuvans umfassend.

**32.** Stoffzusammensetzung, umfassend dendritische Zellen, die eine Zeit lang mit dem gentechnisch veränderten *Bordetella*-Stamm nach Anspruch 1 in Kontakt gekommen sind, und zwar unter für die Expression eines aus dem *Bordetella*-Stamm stammenden prozessierten oder modifizierten Antigens zur Präsentation an und Modulation von T-Zellen hinreichenden Bedingungen.

**33.** Stoffzusammensetzung nach Anspruch 32, die in Form einer In-vitro-Zellkultur vorliegt.

**34.** Verwendung des gentechnisch veränderten *Bordetella*-Stamms nach Anspruch 1 bei der Herstellung eines Arzneimittels zur Modulation einer Immunantwort bei einem Säuger.

**35.** Verwendung des gentechnisch veränderten *Bordetella*-Stamms nach Anspruch 1 bei der Herstellung eines Arzneimittels zur Behandlung und/oder Prophylaxe von Keuchhusten oder einem verwandten Leiden bei einem Säuger.

**36.** Gentechnisch veränderter *Bordetella*-Stamm nach Anspruch 1 zur Verwendung bei der Untersuchung und Modulation einer Immunantwort.

**37.** Gentechnisch veränderter *Bordetella*-Stamm nach Anspruch 36, wobei die Immunantwort gegen einen pathogenen *Bordetella*-Stamm oder einen verwandten Organismus gerichtet ist.

**38.** Gentechnisch veränderter *Bordetella*-Stamm nach Anspruch 1 zur Verwendung bei der Modulation einer Immunantwort bei einem Säuger.

## Revendications

**1.** Souche de *Bordetella* génétiquement modifiée, ayant une perte partielle ou complète de fonction dans le gène *aroQ* endogène et une capacité plus faible à se propager dans un hôte mammifère, mais restant viable dans l'hôte pendant un laps de temps suffisant pour induire une réponse immune contre une souche de *Bordetella* pathogène.

**2.** Souche génétiquement modifiée de la revendication 1, dans laquelle la souche pathogène est choisie parmi *Bordetella avium, Bordetella bronchiseptica, Bordetella holmesii, Bordetella parapertussis* et *Bordetella pertussis.*

**3.** Souche génétiquement modifiée de la revendication 1, dans laquelle la souche de *Bordetella* pathogène est *Bordetella pertussis.*

**4.** Souche génétiquement modifiée de la revendication 1, comprenant une disruption dans le gène *aroQ* endogène.

**5.** Souche génétiquement modifiée de la revendication 4, dans laquelle la disruption a été introduite dans le génome d'une souche pathogène de *Bordetella* par recombinaison homologue avec une construction de ciblage d'ADN de telle sorte que la construction de ciblage soit intégrée d'une manière stable dans le génome, la disruption du gène *aroQ* conduisant à un niveau et/ou à une activité fonctionnelle réduits de la 3-déshydroquinase.

**6.** Souche génétiquement modifiée de la revendication 4, comprenant une séquence d'acide nucléique exogène dans son génome, ou sur un élément extrachromosomique, qui est capable d'abolir ou de réduire autrement l'expression de l'*aroQ* ou le niveau et/ou l'activité fonctionnelle de la 3-déshydroquinase codée par l'*aroQ*.

**7.** Souche génétiquement modifiée de la revendication 4, comprenant une séquence d'acide nucléique exogène dans son génome, ou sur un élément extrachromosomique, qui est capable d'abolir ou de réduire autrement l'expression de l'*aroQ* ou le niveau et/ou l'activité fonctionnelle de la 3-déshydroquinase codée par l'*aroQ*, dans laquelle la séquence d'acide nucléique comprend au moins une portion de l'*aroQ*, selon une orientation sens ou anti-sens, qui est liée de manière opérationnelle à un élément transcriptionnel.

**8.** Souche génétiquement modifiée de la revendication 4, comprenant une séquence d'acide nucléique exogène dans son génome, ou sur un élément extrachromosomique, qui est capable d'abolir ou de réduire autrement l'expression de l'*aroQ* ou le niveau et/ou l'activité fonctionnelle de la 3-déshydroquinase codée par l'*aroQ*, dans laquelle la séquence d'acide nucléique comprend un polynucléotide codant pour un ribozyme, qui est lié de manière opérationnelle à un élément régulateur transcriptionnel, dans lequel le ribozyme se lie d'une manière spécifique à un transcrit du gène *aroQ,* ou interagit autrement avec ce transcrit.

**9.** Souche génétiquement modifiée de la revendication 1, ayant en outre une perte de fonction partielle ou complète dans au moins un autre gène endogène choisi parmi un gène *pur*, un autre gène aro, un gène de la toxine de la coqueluche, ou tout autre gène qui contribue à la survie dans l'hôte et/ou à la virulence bactérienne, ou une combinaison de ceux-ci.

**10.** Souche génétiquement modifiée de la revendication 9, dans laquelle le gène *pur* est choisi parmi *purA*, *purE* ou *purH*.

**11.** Souche génétiquement modifiée de la revendication 9, dans laquelle l'autre gène *aro* est choisi parmi *aroA*, *aroB*, *aroC* ou *aroE*.

**12.** Souche de *Bordetella* génétiquement modifiée de la revendication 1, comprenant au moins un gène exogène qui est capable d'exprimer un antigène qui est hétérologue ou étranger à la souche *Bordetella.*

**13.** Souche de *Bordetella* génétiquement modifiée de la revendication 12, dans laquelle l'antigène hétérologue ou étranger dérive d'un microorganisme pathogène qui n'est pas une souche de *Bordetella.*

**14.** Souche de *Bordetella* génétiquement modifiée de la revendication 12, dans laquelle l'antigène hétérologue ou étranger dérive d'un microorganisme pathogène qui infecte par la voie muqueuse.

**15.** Polynucléotide isolé comprenant une séquence nucléotidique qui correspond à la séquence présentée dans SEQ ID NO:1 ou 3 ou en est complémentaire, ledit polynucléotide isolé codant pour un polypeptide ayant une activité de 3-déshydroquinase.

**16.** Polynucléotide isolé comprenant une séquence nucléotidique ayant une identité de séquence d'au moins 80 % avec au moins une portion de la séquence présentée dans SEQ ID NO:3, ladite portion ayant une longueur d'au moins 150 nucléotides, et ledit polynucléotide isolé codant pour un polypeptide ayant une activité de 3-déshydroquinase.

**17.** Polynucléotide isolé de la revendication 16, dans lequel la séquence nucléotidique a une identité de séquence d'au moins 90 %.

**18.** Polypeptide isolé codé par le polynucléotide isolé de la revendication 16 ou 17, ledit polypeptide isolé ayant une activité de 3-déshydroquinase.

**19.** Polypeptide isolé comprenant une séquence d'acides aminés telle que présentée dans SEQ ID NO:2, ledit polypeptide isolé ayant une activité de 3-déshydroquinase.

**20.** Polypeptide isolé comprenant une séquence d'acides aminés qui a une identité de séquence d'au moins 80 % avec au moins un fragment de 70 acides aminés de la séquence présentée dans SEQ ID NO:2, ledit polypeptide isolé ayant une activité de 3-déshydroquinase.

**21.** Polypeptide isolé de la revendication 20, dans lequel ladite séquence d'acides aminés a une identité de séquence d'au moins 90 %.

**22.** Construction d'acide nucléique pour la disruption d'un gène *aroQ* dans une cellule de *Bordetella,* comprenant : a) une portion de remplacement non homologue ; b) une première région d'homologie, située en amont de la portion de remplacement non homologue, la première région d'homologie ayant une séquence nucléotidique présentant une identité importante avec une première séquence du gène *aroQ* ; et c) une deuxième région d'homologie, située en aval de la portion de remplacement non homologue, la deuxième région d'homologie ayant une séquence nucléotidique présentant une identité importante avec une deuxième séquence du gène *aroQ*, la deuxième séquence du gène *aroQ* étant située en aval de la première séquence du gène *aroQ* dans un gène *aroQ* endogène naturel de la cellule de *Bordetella,* le gène *aroQ* comprenant le polynucléotide isolé tel que présenté dans l'une quelconque des revendications 15-17.

**23.** Vecteur comprenant une séquence nucléotidique qui correspond à la séquence polynucléotidique isolée telle que présentée dans l'une quelconque des revendications 15 à 17, ou en est complémentaire.

**24.** Vecteur de la revendication 23, le vecteur étant un vecteur de ciblage de l'ADN.

**25.** Cellule hôte contenant la construction de la revendication 22 ou le vecteur de la revendication 23.

**26.** Molécule de liaison à l'antigène, qui présente une interactivité spécifique avec le polypeptide de l'une quelconque des revendications 19 à 21.

**27.** Procédé de production d'une souche de *Bordetella* génétiquement modifiée, comprenant l'introduction de la construction d'acide nucléique de la revendication 22 dans une cellule de *Bordetella* dans des conditions telles que la construction d'acide nucléique subisse une recombinaison homologue dans le gène *aroQ* dans le génome de cette cellule, pour produire une cellule de *Bordetella* génétiquement modifiée contenant un gène *aroQ* ayant subi une disruption.

**28.** Procédé de la revendication 27, dans lequel la cellule de *Bordetella* génétiquement modifiée contenant la construction d'acide nucléique ayant subi une recombinaison homologue est en outre **caractérisée par** l'expression de niveaux réduits ou indétectables *d'aroQ.*

**29.** Procédé de la revendication 27, dans lequel la cellule de *Bordetella* génétiquement modifiée ne possède pas la capacité à produire une 3-déshydroquinase fonctionnelle codée par ledit gène *aroQ.*

**30.** Composition comprenant la souche de *Bordetella* génétiquement modifiée de la revendication 1, en même temps qu'un support pharmaceutiquement acceptable.

**31.** Composition de la revendication 30, comprenant en outre un adjuvant.

**32.** Composition de matière comprenant des cellules dendritiques qui ont été exposées à la souche de *Bordetella* génétiquement modifiée de la revendication 1 pendant un laps de temps et dans des conditions suffisants pour exprimer un antigène maturé ou modifié dérivant de la souche de *Bordetella,* pour présentation à des cellules T et modulation de ces dernières.

**33.** Composition de matière de la revendication 32, qui se présente sous la forme d'une culture cellulaire *in vitro.*

**34.** Utilisation d'une souche de *Bordetella* génétiquement modifiée de la revendication 1 pour la fabrication d'un médicament destiné à la modulation d'une réponse immune chez un mammifère.

**35.** Utilisation d'une souche de *Bordetella* génétiquement modifiée de la revendication 1 pour la fabrication d'un médicament destiné au traitement et/ou à la prophylaxie de la coqueluche ou d'un état pathologique apparenté chez un mammifère.

**36.** Souche de *Bordetella* génétiquement modifiée de la revendication 1 pour utilisation dans l'étude et la modulation d'une réponse immune.

**37.** Souche de *Bordetella* génétiquement modifiée de la revendication 36, dans laquelle la réponse immune est dirigée contre une souche pathogène de *Bordetella* ou d'un organisme apparenté.

**38.** Souche de *Bordetella* génétiquement modifiée de la revendication 1 pour utilisation dans la modulation d'une réponse immune chez un mammifère.

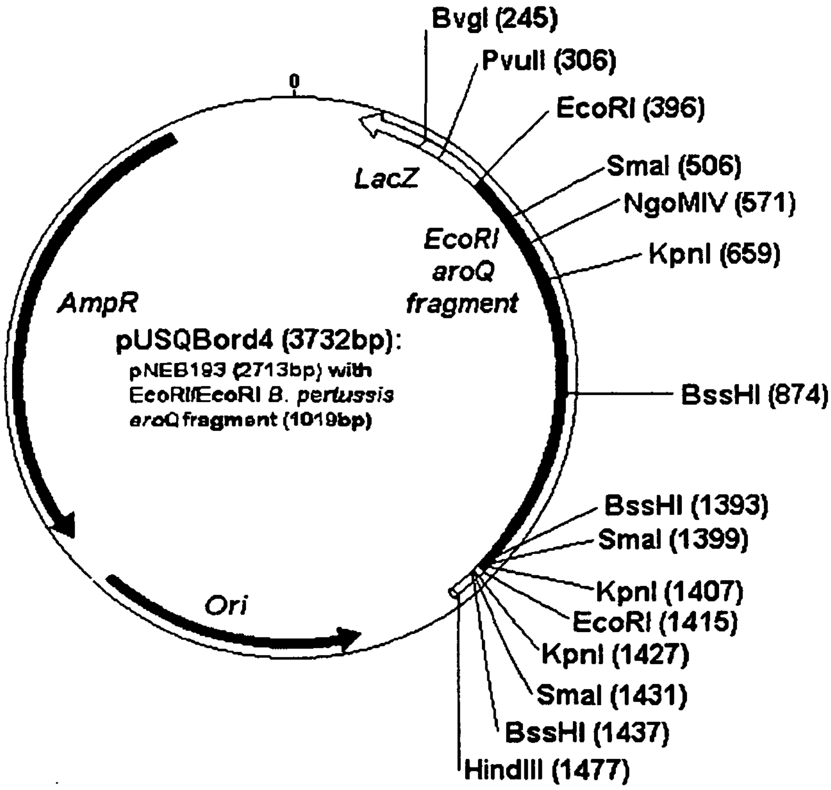
Bvgl (245)
PvuII (306)
EcoRI (396)
SmaI (506)
NgoMIV (571)
KpnI (659)
BssHI (874)
BssHI (1393)
SmaI (1399)
KpnI (1407)
EcoRI (1415)
KpnI (1427)
SmaI (1431)
BssHI (1437)
HindIII (1477)
0
LacZ
EcoRI
aroQ
fragment
AmpR
Ori
pUSQBord4 (3732bp):
pNEB193 (2713bp) with
EcoRI/EcoRI B. pertussis
aroQ fragment (1019bp)

FIGURE 1

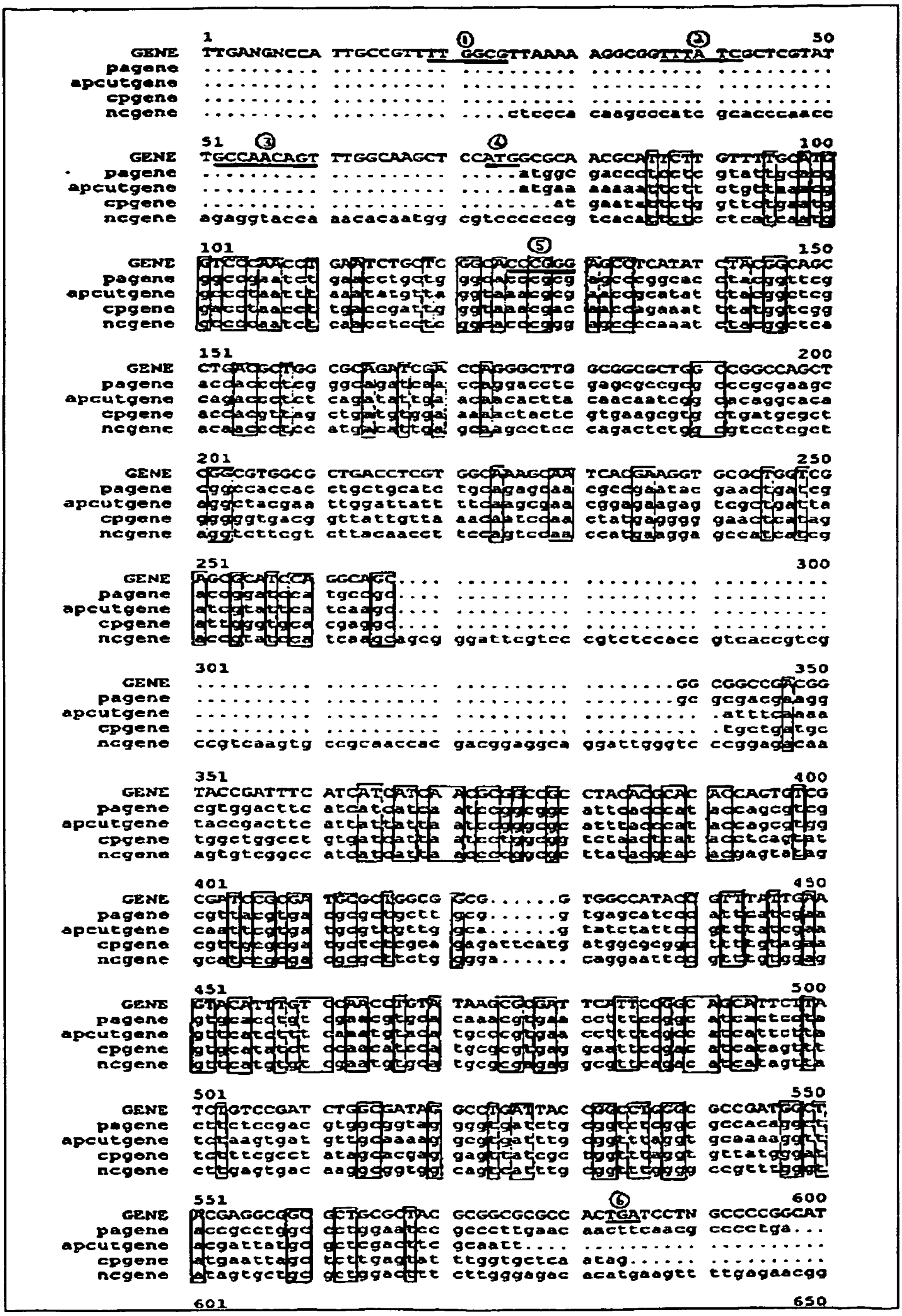
GENE
pagene
apcutgene
cpgene
ncgene
1
50
51
100
101
150
151
200
201
250
251
300
301
350
351
400
401
450
451
500
501
550
551
600
601
650

FIGURE 2

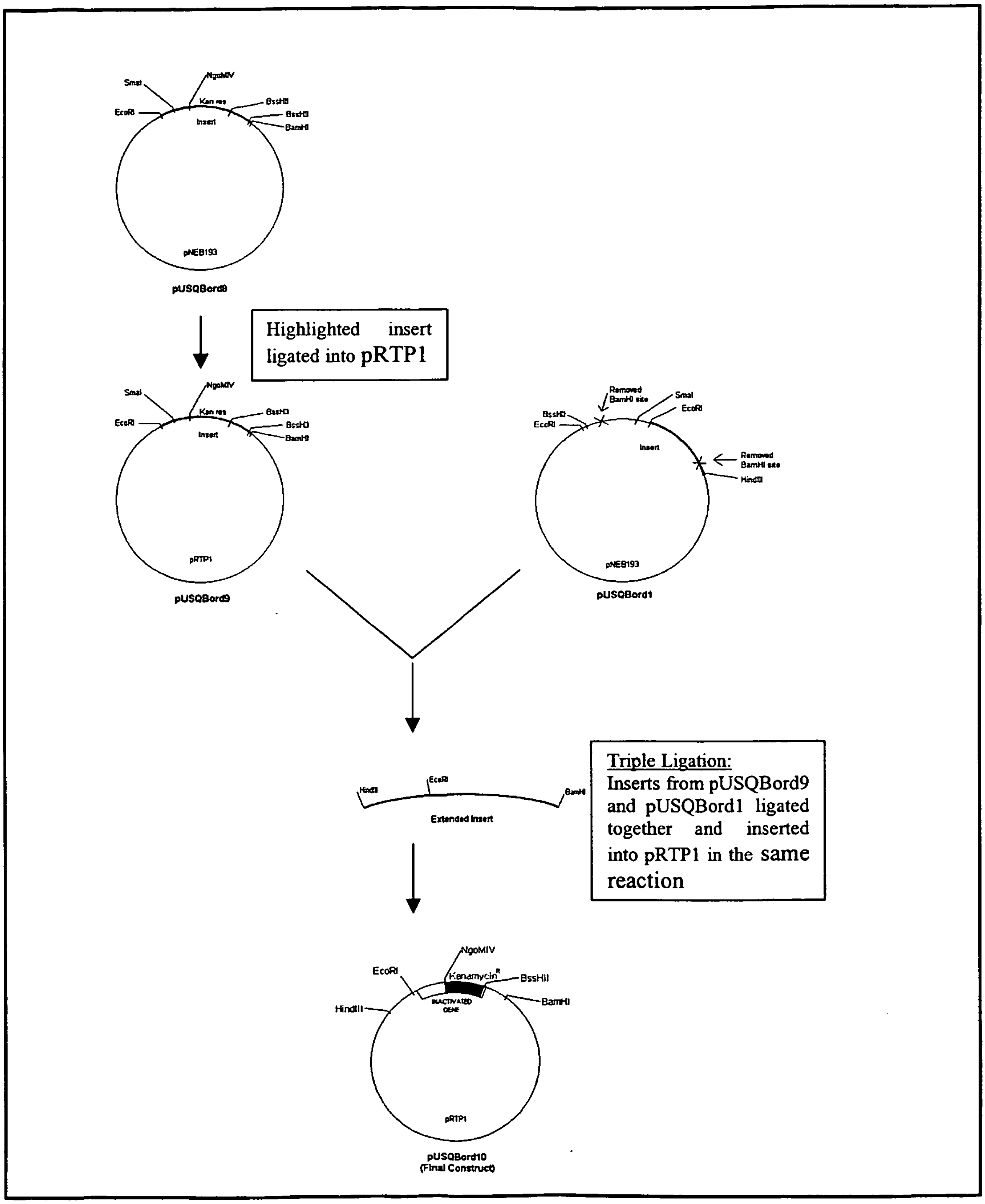
pNEB193
pUSQBord8
Highlighted insert ligated into pRTP1
pRTP1
pUSQBord9
pNEB193
pUSQBord1
Extended Insert
Triple Ligation:
Inserts from pUSQBord9 and pUSQBord1 ligated together and inserted into pRTP1 in the same reaction
NgoMIV
EcoRI
Kanamycin
BssHII
HindIII
BamHI
pRTP1
pUSQBord10
(Final Construct)

FIGURE 3

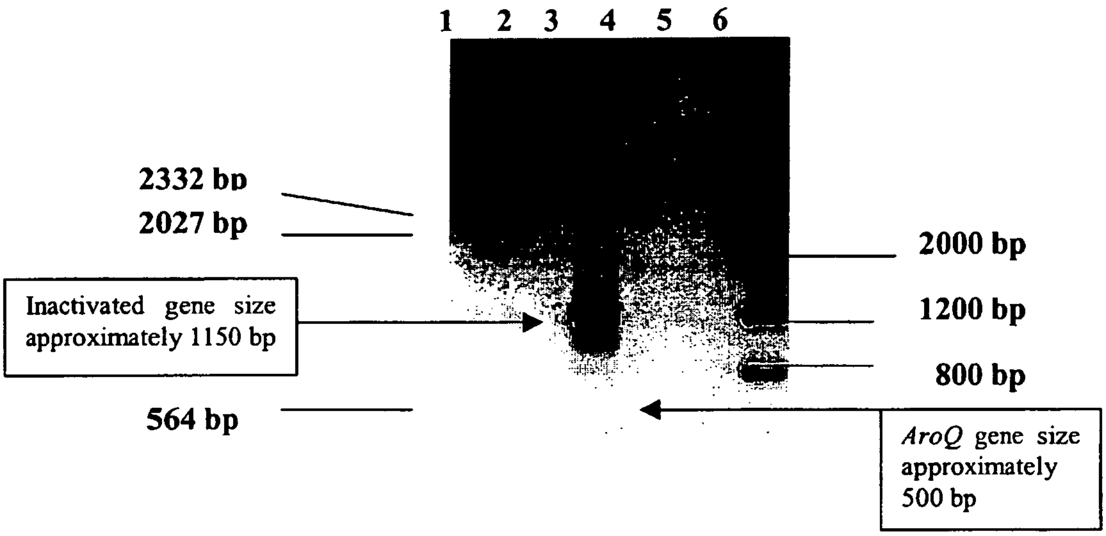
1 2 3 4 5 6
2332 bp
2027 bp
Inactivated gene size approximately 1150 bp
564 bp
2000 bp
1200 bp
800 bp
AroQ gene size approximately 500 bp

FIGURE 4

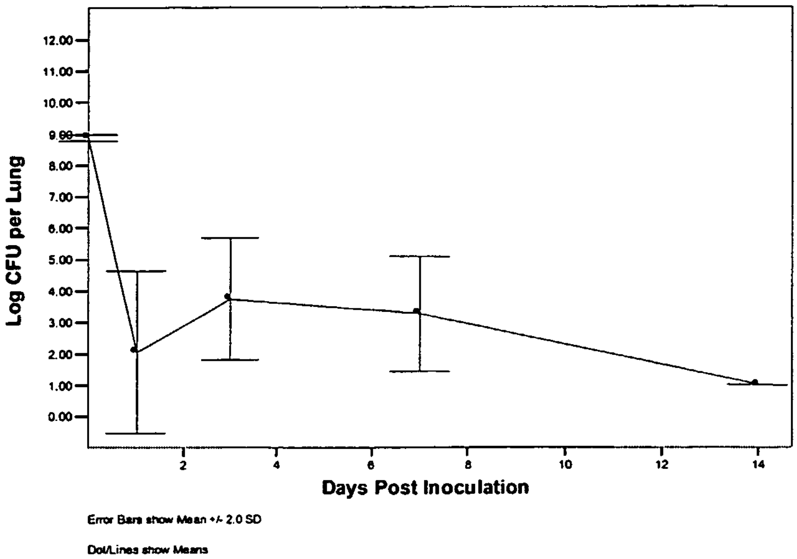


FIGURE 5A

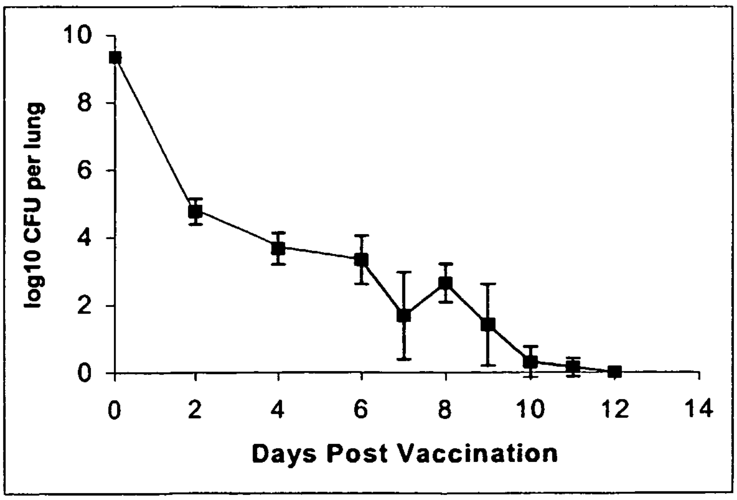


FIGURE 5B

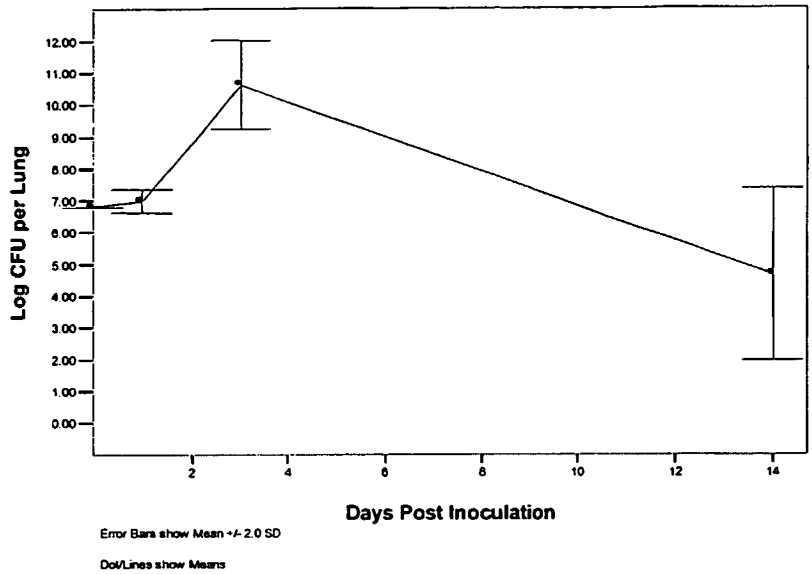


FIGURE 5C

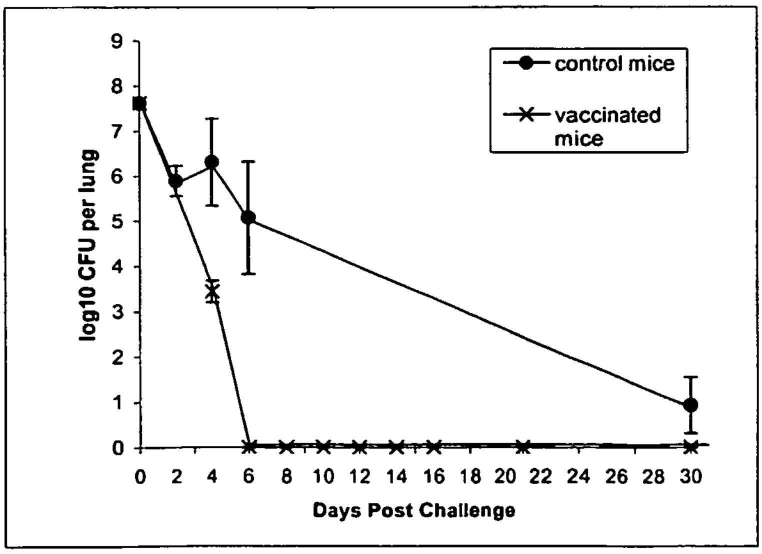


FIGURE 6

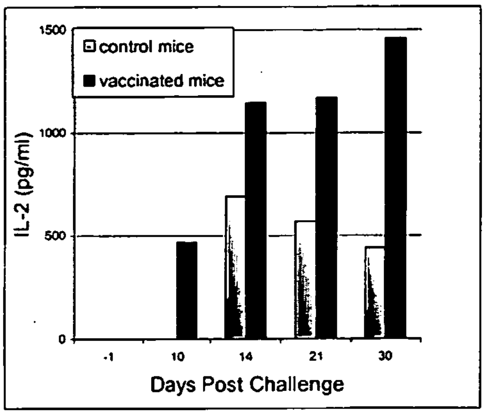


FIGURE 7A

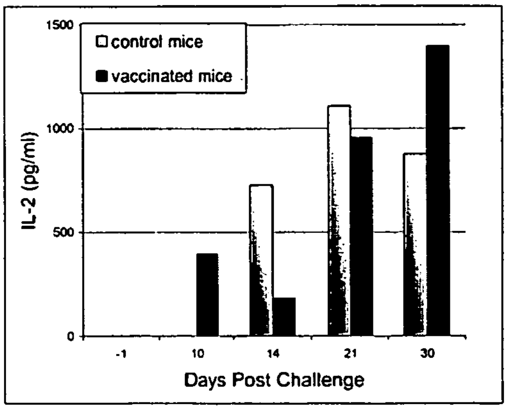


FIGURE 7B

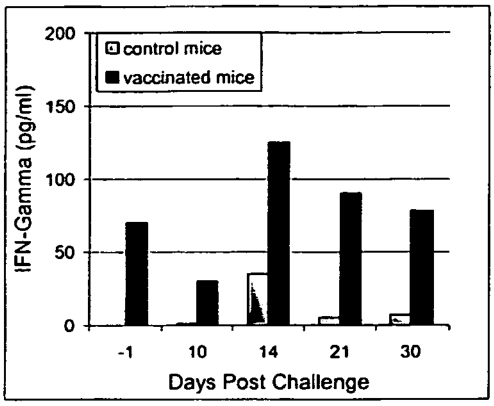


FIGURE 8A

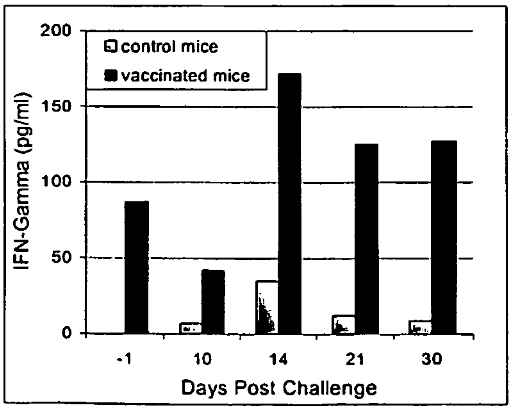


FIGURE 8B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5422252 A
- WO 9201813 A
- WO 9719193 A, Lizardi
- US 4946778 A
- US 5091513 A
- EP 239400 A
- US 5837821 A
- US 5892020 A
- WO 9208447 A
- WO 9206675 A
- WO 9104011 A
- WO 9942564 A, Albert
- WO 9729182 A, Steinman


### Non-patent literature cited in the description


- **ATHERTON ; SHEPHARD.** Peptide Synthesis
- Synthetic Vaccines. Blackwell Scientific Publications
- **DEVERAUX et al.** *Nucleic Acids Research,* 1984, vol. 12, 387-395
- **ALTSCHUL et al.** *Nucl. Acids Res.,* 1997, vol. 25, 3389
- **AUSUBEL et al.** Current Protocols in Molecular Biology. John Wiley & Sons Inc, 1994
- **ADELMAN et al.** *DNA,* 1983, vol. 2, 183
- **VIERA et al.** *Methods Enzymol.,* 1987, vol. 153, 3
- **LIU et al.** *J. Am. Chem. Soc.,* 1996, vol. 118, 1587-1594
- **SOOKNANAN et al.** *Biotechniques,* 1994, vol. 17, 1077-1080
- **TYAGI et al.** *Proc. Natl. Acad. Sci. USA,* 1996, vol. 93, 5395-5400
- **SAMBROOK et al.** Molecular Cloning. A Laboratory Manual. Cold Spring Harbour Press, 1989
- **PELICIC et al.** *Mol. Microbiol.,* 1996, vol. 20, 919-925
- **MARLOWE.** *Biorganic & Medicinal Chemistry Letters,* 1993, vol. 3, 437-44
- **PALLIN ; TAM.** *J. Chem. Soc. Chem. Comm.,* 1995, 2021-2022
- **ALGIN et al.** *Tetrahedron Letters,* 1994, vol. 35, 9633-9636
- **KATES et al.** *Tetrahedron Letters,* 1993, vol. 34, 1549-1552
- **TUMELTY et al.** *J. Chem. Soc. Chem. Comm.,* 1994, 1067-1068
- **MCMURRAY et al.** *Peptide Research,* 1994, vol. 7, 195-206
- **HRUBY et al.** *Reactive Polymers,* 1994, vol. 22, 231-241
- **SCHMIDT ; LANGER.** *J. Peptide Res.,* 1997, vol. 49, 67-73
- **COLIGAN et al.** CURRENT PROTOCOLS IN IMMUNOLOGY. John Wiley & Sons, Inc, 1991
- **KÖHLER ; MILSTEIN.** *Nature,* 1975, vol. 256, 495-497
- **KREBER et al.** *J. Immunol. Methods,* 1997, vol. 201 (1), 35-55
- **WINTER ; MILSTEIN.** *Nature,* 1991, vol. 349, 293
- **PLIICKTHUN et al.** *Antibody engineering: A practical approach.,* 203-252
- **GLOCKSCUTHER et al.** *Biochem.,* vol. 29, 1363-1367
- **REITER et al.** *J. Biol. Chem,* 1994, vol. 269, 18327-18331
- **REITER et al.** *Biochem.,* 1994, vol. 33, 5451-5459
- **REITER et al.** *Cancer Res.,* 1994, vol. 54, 2714-2718
- **WEBBER et al.** *Mol. Immunol.,* 1995, vol. 32, 249-258
- **WARD et al.** *Nature,* 1989, vol. 341, 544-546
- **HAMERS-CASTERMAN et al.** *Nature,* 1993, vol. 363, 446-448
- **DAVIES ; RIECHMANN.** *FEBS Lett.,* 1994, vol. 339, 285-290
- **KU ; SCHULTZ.** *Proc. Natl. Acad. Sci. USA,* 1995, vol. 92, 652-6556
- **ADAMS et al.** *Cancer Res.,* vol. 53, 4026-4034
- **CUMBER et al.** *J. Immunol.,* 1992, vol. 149, 120-126
- **PACK P. PLÜNCKTHUN.** *Biochem.,* 1992, vol. 31, 1579-1584
- **KOSTELNY et al.** *J. Immunol.,* 1992, vol. 148, 1547-1553

- Pharmaceutical Inhalation Aerosol Technology. Marcel Dekker
- **TAKAMIZAWA et al.** *J Immunol,* 1997, vol. 158 (5), 2134-2142
- **THOMAS ; LIPSKY.** *J Immunol,* 1994, vol. 153 (9), 4016-4028
- **O'DOHERTY et al.** *Immunology,* 1994, vol. 82 (3), 487-93
- **FEARNLEY et al.** *Blood,* 1997, vol. 89 (10), 3708-3716
- **WEISSMAN et al.** *Proc Natl Acad Sci U S A,* 1995, vol. 92 (3), 826-830
- **FREUDENTHAL ; STEINMAN.** *Proc Natl Acad Sci U S A,* 1990, vol. 87 (19), 7698-7702
- **ROMANI et al.** *J Immunol Methods,* 1996, vol. 196 (2), 137-151
- **REDDY et al.** *Blood,* 1997, vol. 90 (9), 3640-3646
- **THURNHER et al.** *Exp Hematol,* 1997, vol. 25 (3), 232-237
- **CAUX et al.** *J Exp Med,* 1996, vol. 184 (2), 695-706
- *Blood,* 1996, vol. 87 (6), 2376-85
- **LUFT et al.** *Exp Hematol,* 1998, vol. 26 (6), 489-500
- *J Immunol,* 1998, vol. 161 (4), 1947-1953
- **CELLA et al.** *J Exp Med,* 1999, vol. 189 (5), 821-829
- *Nature,* 1997, vol. 388 (644), 782-787
- *J Exp Med,* 1996, vol. 184 (2), 747-572
- **AHONEN et al.** *Cell Immunol,* 1999, vol. 197 (1), 62-72
- **PIEMONTI et al.** *J Immunol,* 1999, vol. 162 (11), 6473-6481
- **PAGLIA et al.** *J Exp Med,* 1993, vol. 178 (6), 1893-1901
- **KOUP et al.** *J. Exp. Med.,* 1991, vol. 174, 1593-1600
- **CARMICHAEL et al.** *J. Exp. Med.,* 1993, vol. 177, 249-256
- **JOHNSON et al.** *J. Exp. Med.,* 1992, vol. 175, 961-971
- **HILL et al.** *Nature,* 1992, vol. 360, 434-439
- **VAN DER BROGEN et al.** *Science,* 1991, vol. 254, 1643-1647
- **YOUNG ; STEINMAN.** *J. Exp. Med.,* 1990, vol. 171, 1315-1332
- **ALLEN et al.** *J. Immunol.,* 2000, vol. 164 (9), 4968-4978
- **BARAFF, L. J. ; C. L. CODY ; J. D. CHERRY.** DTP associated reactions: an analysis by injection site, manufacturer, prior reactions and dose. *Pediatrics,* 1984, vol. 73, 31-36
- **CANTHABOO, C. ; L. WILLIAMS ; D. K. L. XING ; M. J. CORBEL.** Investigation of cellular and humoral immune responses to whole cell and acellular pertussis vaccines. *Vaccine,* 2000, vol. 19, 637-643
- **COHEN, S.M. ; M.W. WHEELER.** Pertussis vaccine prepared with phase-I cultures grown in fluid medium. *Am. J. Pub. Health.,* 1946, vol. 36, 371
- **EWANOWICH, C. A. ; A.R. MELTON ; A. A. WEISS ; R. K. SHERBURNE ; A. PEPPLER.** Invasion of HeLa 299 cells by virulent Bordetella pertussis. *Infec. Immun.,* 1989, vol. 57, 2698-2704
- **FARRELL, D. J.** Bordetella pertussis: diagnosis, infection and immunity. *PhD Dissertation; University of Southern Queensland,* 2000
- **FROHLICH, B. T. ; E. R. DE BERNARDEZ CLARK ; G. R. SIBER ; R. W. SWARTZ.** Improved pertussis toxin production by Bordetella pertussis through adjusting the growth medium's ionic composition. *J. Biotech.,* 1995, vol. 39, 205-219
- **GALAZKA, A.** Control of pertussis in the world. *World. Health Stat. Q.,* 1992, vol. 45, 238-247
- **GUISO, N. ; C. CAPIAU ; G. CARLETTI ; J. POOLMAN ; P. HAUSER.** Intranasal murine model of protection in human infants by acellular vaccines. *Vaccine,* 1999, vol. 17, 2366-2376
- **HOISETH, S. K. ; B. A. D. STOCKER.** Aromatic-dependent Salmonella typhimurium are non-virulent and effective as live vaccines. *Nature (London,* 1981, vol. 291, 238-239
- **HOWSON, C. P. ; H. V. FINEBERG.** Adverse events following pertussis and rubella vaccines. *JAMA,* 1992, vol. 267, 392-396
- **IMAIZUMI, A. ; Y. SUZUKI ; S. ONO ; H. SATO ; Y. SATO.** Heptakis (2,6-O-dimethyl)β-cyclodextrin: a novel growth stimulent for Bordetella pertussis phase I. *J. Clin. Microbiol.,* 1983, vol. 17, 781-786
- **LALONDE, G. ; P. D. O'HANLEY ; B. A. D. STOCKER ; K. T. DENICH.** Characterisation of a 3-dehydroquinase gene from Actinobacilus pleuropneumoniae with hology to the eukaryotic genes qa-2 and QUTE. *Mol. Microbiol.,* 1994, vol. 11, 273-280
- **MANETTI, R. ; P. PARRONCHI ; M. G. GIUDIZI ; M. P. PICCINNI ; E. MAGGI. ; G. TRINCHIERI. ; S. ROMAGNANI.** Natural killer cell stimulatory factor (interleukin 12 [IL-12]) induces T helper type 1 (Th1)-specific immune response and inhibits the development of IL-4-producing Th cells. *J. Exp. Med.,* 1993, vol. 177, 199-204
- **MARBLE, M. ; K. K. KEY.** Bioengineered Bordetella pertussis used for intranasal immunisation. *Disease Weekly Plus,* 08 September 1996, 23-24
- **MIELCAREK, N. ; G. RIVEAU ; F. REMOUÉ ; R. ANTOINE ; A. CAPRON ; C. LOCHT.** Homologous and heterologous protection after single intranasal administration of live attenuated recombinant Bordetella pertussis. *Nature Biotech.,* 1998, vol. 16, 454-457

- **MILLS, K. H. G. ; A. BARNARD ; J. WATKINS ; K. REDHEAD.** Cell-mediated immunity to Bordetella pertussis: role of Th1 cella in bacterial clearance in a murine respiratory infection model. *Infect. Immun.,* 1993, vol. 61, 399-410
- **MILLS, K.H.G.** Immunity to Bordetella pertussis. *Microbes Infect,* 2001, vol. 3, 655-677
- **MUKKUR, T. K. S. ; G. H. MCDOWELL ; B. A. D. STOCKER ; A. K. LASCELLES.** Protection against experimental salmonellosis in mice and sheep by immunisation with aromatic-dependent Salmonella typhimurium. *J. Med. Microbiol.,* vol. 24, 11-19
- **POLLOCK, T. M. ; J. Y. MORTIMER ; E. MILLER ; G. SMITH.** Symptoms after primary immunisation with DTP and DT vaccine. *Lancet,* 1984, vol. ii, 146-149
- **REDHEAD, K. ; J. WATKINS ; A. BARNARD ; K.H.G. MILLS.** Effective immunization against Bordetella pertussis respiratory infection in mice is dependent on induction of cell-mediated immunity. *Infect. Immun.,* 1993, vol. 61, 3190-3198
- **RENNELS, M.B. ; DELORIA, M.A. ; PICHICHERO, M.E. ; LOSONSKY, G.A. ; ENGLUND, J.A. ; MEAD, B.D. ; ANDERSON, E.L. ; STEINHOFF, M.C. ; EDWARDS, K.M.** Extensive swelling after booster doses of acellular pertussis-tetanus-diphtheria vaccines. *Pediatrics,* 2000, vol. 1, e1-12
- **ROBERTS, M. ; D. MASKELL ; P. NOVOTNY ; G. DOUGAN.** Construction and characterisation in vivo of Bordetella pertussis aroA mutants. *Infect. Immun.,* 1990, vol. 58, 732-739
- **ROSSETTI, T. R.** *Cloning the aroD gene of Bordetella pertussis (BSc. Hons. Thesis,* 1997
- **SAMBROOK, J. ; E. F. FRITSCH ; T. MANIATIS.** Molecular Cloning: a laboratory manual. Cold Spring Harbour Laboratory Press, 1989
- **SAUKKONEN, K. ; C. CABELLOS ; M. BURROUGHS ; S. PRASAD ; E. TUOMANEN.** Integrin mediated localization of Bordetella pertussis within macrophages: Role in pulmonary colonization. *J. Exp. Med.,* 1991, vol. 173, 1143-1149
- **SIMMONS, C. P. ; S. J. DUNSTAN ; M. TACHEDJIAN ; J. KRYWULT ; A. L. HODGSON ; R. A. STRUGNELL.** Vaccine potential of attenuated mutants of Corynebacterium pseudotuberculosis in sheep. *Infect. Immun.,* 1998, vol. 66, 474-479
- **STAINER, D. W ; M. J. SCHOLTE.** A simple and chemically defined medium for the production of phase I Bordetella pertussis. *J. Gen. Microbiology,* 1971, vol. 34, 778-784
- **STIBITZ, S. ; G. W. BLACK ; S. FALKOW.** The construction of a cloning vector designed for gene replacement in Bordetella pertussis. *Gene,* 1986, vol. 50, 133-140
- **TOWBIN, H. ; T. STACHELIN ; J GORDON.** Electrophoretic transfer of proteins from polyacrylamide gels to nitrocellulose sheets: procedure and some applications. *Proc. Natl. Acad. Sci., USA,* 1979, vol. 76, 4350-4354
- **VERMA, N.K. ; A. A. LINDBERG.** Construction of aromatic dependent Shigella flexneri 2a live vaccine candidate strains: deletion mutations in the aroA and the aroD genes. *Vaccine,* 1991, vol. 9, 6-9
- **WEISS, A. A. ; E. L. HEWLETT.** Virulence factors of Bordetella pertussis. *Ann. Rev. Microbiol.,* 1986, vol. 40, 661-686
- **ZEPP, F. ; M. KNUF ; P. HABERMEHL ; J. H. SCMITT ; C. REBSCH ; P. SCHMIDTKE ; R. CLEMENS ; M. SLAOUI.** Pertussis-specific cell-mediated immunity in infants after vaccination with tricomponent acellular pertussis vaccine. *Infect. Immun.,* 1996, vol. 64, 4078-4084

**SEQUENCE LISTING**

<110> Delta Biotechnology Limited (all states except US)
Cornford-Nairn, Renee (US only)
Daggard, Grant Edward (US only)
Mukkur, Trilochan Kanwaljit Singh (US only)
Rossetti, Tony Robert (US only)

<120> Defective entities and uses therefor

<130> 2601959/VPA

<140> Not yet assigned
<141> 2002-12-30

<150> AU PR9776/01
<151> 2001-12-28

<160> 7

<170> PatentIn version 3.1

<210> 1

<211> 942
<212> DNA
<213> Bordetella pertussis

<220>
<221> misc feature
<222> (5) .. (5)
<223> n = unknown nucleotide

<220>
<221> misc_feature
<222> (7) .. (7)
<223> n = unknown nucleotide

<220>
<221> misc_feature
<222> (512)..(512)
<223> n = unknown nucleotide

<220>
<221> misc_feature
<222> (523)..(523)
<223> n = unknown nucleotide

<220>
<221> misc_feature
<222> (527)..(527)
<223> n = unknown nucleotide

<220>
<221> misc_feature
<222> (536)..(536)
<223> n = unknown nucleotide

<220>
<221> misc_feature
<222> (552)..(552)
<223> n = unknown nucleotide

<220>
<221> misc_feature
<222> (561)..(561)
<223> n = unknown nucleotide

<220>
<221> misc_feature
<222> (582)..(582)
<223> n = unknown nucleotide

<220>
<221> misc_feature
<222> (584)..(584)
<223> n = unknown nucleotide

<220>
<221> misc_feature
<222> (587)..(587)
<223> n = unknown nucleotide

<220>
<221> misc_feature
<222> (594)..(594)
<223> n = unknown nucleotide

<220>
<221> misc_feature
<222> (597) .. (597)
<223> n = unknown nucleotide

<220>
<221> misc_feature
<222> (745)..(745)
<223> n = unknown nucleotide

<220>
<221> misc_feature
<222> (746)..(746)
<223> n = unknown nucleotide

<220>
<221> misc_feature
<222> (747)..(747)
<223> n = unknown nucleotide

<220>
<221> misc_feature
<222> (765)..(765)
<223> n = unknown nucleotide

<220>
<221> misc_feature
<222> (768)..(768)
<223> n = unknown nucleotide

<220>
<221> misc_feature
<222> (778)..(778)
<223> n = unknown nucleotide

<220>
<221> misc_feature
<222> (791)..(791)
<223> n = unknown nucleotide

<220>
<221> misc_feature
<222> (806)..(806)
<223> n = unknown nucleotide

<220>
<221> misc_feature
<222> (812)..(812)
<223> n = unknown nucleotide

<220>
<221> misc_feature
<222> (823)..(823)

<223> n = unknown nucleotide

<220>
<221> misc_feature
<222> (829) .. (829)
<223> n = unknown nucleotide

<220>
<221> misc_feature
<222> (853)..(853)
<223> n = unknown nucleotide

<220>
<221> misc_feature
<222> (854)..(854)
<223> n = unknown nucleotide

<220>
<221> misc_feature
<222> (870)..(870)
<223> n = unknown nucleotide

<220>
<221> misc_feature
<222> (871)..(871)
<223> n = unknown nucleotide

<220>
<221> misc_feature
<222> (881)..(881)
<223> n = unknown nucleotide

<220>
<221> misc_feature
<222> (884)..(884)
<223> n = unknown nucleotide

<220>
<221> misc_feature
<222> (889)..(889)
<223> n = unknown nucleotide

<220>
<221> misc_feature
<222> (892)..(892)
<223> n = unknown nucleotide

<220>
<221> misc_feature
<222> (894) .. (894)
<223> n = unknown nucleotide

<220>
<221> misc_feature
<222> (896)..(896)
<223> n = unknown nucleotide

<220>

<221> misc_feature
<222> (898)..(898)
<223> n = unknown nucleotide

<220>
<221> misc_feature
<222> (899)..(899)
<223> n = unknown nucleotide

<220>
<221> misc_feature
<222> (914) .. (914)
<223> n = unknown nucleotide

<220>
<221> misc_feature
<222> (915)..(915)
<223> n = unknown nucleotide

<220>
<221> misc_feature
<222> (916)..(916)
<223> n = unknown nucleotide

<220>
<221> misc_feature
<222> (917)..(917)
<223> n = unknown nucleotide

<220>
<221> misc_feature
<222> (918)..(918)
<223> n = unknown nucleotide

<220>
<221> misc_feature
<222> (921)..(921)
<223> n = unknown nucleotide

<220>
<221> misc_feature
<222> (926)..(926)
<223> n = unknown nucleotide

<220>
<221> misc_feature
<222> (929)..(929)
<223> n = unknown nucleotide

<220>
<221> misc_feature
<222> (930)..(930)
<223> n = unknown nucleotide

<220>
<221> misc_feature
<222> (935)..(935)
<223> n = unknown nucleotide

<220>
<221> misc_feature
<222> (936)..(936)
<223> n = unknown nucleotide

<220>
<221> misc_feature
<222> (938)..(938)
<223> n = unknown nucleotide

<220>
<221> misc_feature
<222> (939)..(939)
<223> n = unknown nucleotide

<220>
<221> misc_feature
<222> (940)..(940)
<223> n = unknown nucleotide

<220>
<221> misc_feature
<222> (942)..(942)
<223> n = unknown nucleotide

<220>
<221> CDS
<222> (73) .. (504)
<223>

<220>
<221> -10_signal
<222> (19)..(24)
<223>

<220>
<221> -35_signal
<222> (37) .. (42)
<223>

<220>
<221> RBS
<222> (52)..(60)
<223>

<400> 1

```
ttgangncca ttgccgtttt ggcgttaaaa aggcggttta tcgctcgtat tgccaacagt      60

ttggcaagct cc atg gcg caa cgc att ctt gtt ttg cat ggt ccc aac ctg     111
              Met Ala Gln Arg Ile Leu Val Leu His Gly Pro Asn Leu
              1               5                   10

aat ctg ctc ggc acc cgg gag cct cat atc tac ggc agc ctg acg ctg       159
Asn Leu Leu Gly Thr Arg Glu Pro His Ile Tyr Gly Ser Leu Thr Leu
    15                  20                  25

gcg cag atc gac cag ggc ttg gcg gcg ctg gcc ggc cag ctc ggc gtg       207
Ala Gln Ile Asp Gln Gly Leu Ala Ala Leu Ala Gly Gln Leu Gly Val
30                  35                  40                  45

gcg ctg acc tcg tgg caa agc aat cac gaa ggt gcg ctg gtc gag cgc       255
Ala Leu Thr Ser Trp Gln Ser Asn His Glu Gly Ala Leu Val Glu Arg
                50                  55                  60

atc cag gca gcg gcg gcc gac ggt acc gat ttc atc atc atc aac gcg       303
Ile Gln Ala Ala Ala Ala Asp Gly Thr Asp Phe Ile Ile Ile Asn Ala
            65                  70                  75

gcc gcc tac acg cac acc agt gtc gcg atc cgc gat gcg ctg gcg gcg       351
Ala Ala Tyr Thr His Thr Ser Val Ala Ile Arg Asp Ala Leu Ala Ala
        80                  85                  90

gtg gcc ata ccg ttt att gaa gta cat ttg tcc aac ctg tat aag cgc       399
Val Ala Ile Pro Phe Ile Glu Val His Leu Ser Asn Leu Tyr Lys Arg
    95                  100                 105

gat tca ttc cgg cag cat tct tat ctg tcc gat ctg gcg ata ggc ctg       447
Asp Ser Phe Arg Gln His Ser Tyr Leu Ser Asp Leu Ala Ile Gly Leu
110                 115                 120                 125

att acc ggc ctg ggc gcc gat ggc tac gag gcg gcg ctg cgc tac gcg       495
Ile Thr Gly Leu Gly Ala Asp Gly Tyr Glu Ala Ala Leu Arg Tyr Ala
                130                 135                 140

gcg cgc cac tgatcctngc cccggcatnt ttnacattta cnggaatttg               544
Ala Arg His

accccggncc cgaaatncgg gccgcggcca aaacacantn ggnaagcagn ttntatggac     604

ctccgaaaac tcaaaaccct gatcgacctc gtggctgaat cgggtatcgc cgagcttgaa     664

atcaccgaag cgaaggcaag gttcgcatcg tcaaattctc gcaagccctg caaccggttg     724

gctatacatg cccaggccga nnncccggcg gccgctcctg nggnggctgc cagngcccgt     784

cgacgangcg gtgcccgcgg cnccgaangg ccatgtggnc aaggngccca tggacggacc     844

ttttaccgnn cgcccaatct cggcgnngcg cctatcntcn atgtnggntn ancnntcaag     904

gaaaggcgan nnnnatntgc ancanntagg nnannnan                             942
```

<210> 2
<211> 144
<212> PRT

<213> Bordetella pertussis

<220>
<221> misc_feature
<222> (5) .. (5)
<223> n = unknown nucleotide

<220>
<221> misc_feature
<222> (7) .. (7)
<223> n = unknown nucleotide

<220>
<221> misc_feature
<222> (512)..(512)
<223> n = unknown nucleotide

<220>
<221> misc_feature
<222> (523)..(523)
<223> n = unknown nucleotide

<220>
<221> misc_feature
<222> (527)..(527)
<223> n = unknown nucleotide

<220>
<221> misc_feature
<222> (536)..(536)
<223> n = unknown nucleotide

<220>
<221> misc_feature
<222> (552)..(552)
<223> n = unknown nucleotide

<220>
<221> misc_feature
<222> (561) .. (561)
<223> n = unknown nucleotide

<220>
<221> misc_feature
<222> (582)..(582)
<223> n = unknown nucleotide

<220>
<221> misc_feature
<222> (584)..(584)
<223> n = unknown nucleotide

<220>
<221> misc_feature
<222> (587)..(587)
<223> n = unknown nucleotide

<220>

<221> misc_feature
<222> (594)..(594)
<223> n = unknown nucleotide

<220>
<221> misc_feature
<222> (597)..(597)
<223> n = unknown nucleotide

<220>
<221> misc_feature
<222> (745)..(745)
<223> n = unknown nucleotide

<220>
<221> misc_feature
<222> (746)..(746)
<223> n = unknown nucleotide

<220>
<221> misc_feature
<222> (747)..(747)
<223> n = unknown nucleotide

<220>
<221> misc_feature
<222> (765)..(765)
<223> n = unknown nucleotide

<220>
<221> misc_feature
<222> (768)..(768)
<223> n = unknown nucleotide

<220>
<221> misc_feature
<222> (778)..(778)
<223> n = unknown nucleotide

<220>
<221> misc feature
<222> (791) .. (791)
<223> n = unknown nucleotide

<220>
<221> misc_feature
<222> (806)..(806)
<223> n = unknown nucleotide

<220>
<221> misc_feature
<222> (812)..(812)
<223> n = unknown nucleotide

<220>
<221> misc_feature
<222> (823)..(823)
<223> n = unknown nucleotide

<220>
<221> misc_feature
<222> (829)..(829)
<223> n = unknown nucleotide

<220>
<221> misc_feature
<222> (853) .. (853)
<223> n = unknown nucleotide

<220>
<221> misc_feature
<222> (854)..(854)
<223> n = unknown nucleotide

<220>
<221> misc_feature
<222> (870)..(870)
<223> n = unknown nucleotide

<220>
<221> misc_feature
<222> (871)..(871)
<223> n = unknown nucleotide

<220>
<221> misc_feature
<222> (881)..(881)
<223> n = unknown nucleotide

<220>
<221> misc_feature
<222> (884)..(884)
<223> n = unknown nucleotide

<220>
<221> misc_feature
<222> (889)..(889)
<223> n = unknown nucleotide

<220>
<221> misc_feature
<222> (892)..(892)
<223> n = unknown nucleotide

<220>
<221> misc_feature
<222> (894)..(894)
<223> n = unknown nucleotide

<220>
<221> misc_feature
<222> (896)..(896)
<223> n = unknown nucleotide

<220>
<221> misc_feature
<222> (898)..(898)

<223> n = unknown nucleotide

<220>
<221> misc_feature
<222> (899)..(899)
<223> n = unknown nucleotide

<220>
<221> misc_feature
<222> (914)..(914)
<223> n = unknown nucleotide

<220>
<221> misc_feature
<222> (915)..(915)
<223> n = unknown nucleotide

<220>
<221> misc_feature
<222> (916) .. (916)
<223> n = unknown nucleotide

<220>
<221> misc feature
<222> (917) .. (917)
<223> n = unknown nucleotide

<220>
<221> misc_feature
<222> (918)..(918)
<223> n = unknown nucleotide

<220>
<221> misc_feature
<222> (921)..(921)
<223> n = unknown nucleotide

<220>
<221> misc_feature
<222> (926)..(926)
<223> n = unknown nucleotide

<220>
<221> misc_feature
<222> (929)..(929)
<223> n = unknown nucleotide

<220>
<221> misc_feature
<222> (930)..(930)
<223> n = unknown nucleotide

<220>
<221> misc_feature
<222> (935) .. (935)
<223> n = unknown nucleotide

<220>

<221> misc_feature
<222> (936) .. (936)
<223> n = unknown nucleotide

<220>
<221> misc_feature
<222> (938)..(938)
<223> n = unknown nucleotide

<220>
<221> misc_feature
<222> (939)..(939)
<223> n = unknown nucleotide

<220>
<221> misc_feature
<222> (940)..(940)
<223> n = unknown nucleotide

<220>
<221> misc_feature
<222> (942)..(942)
<223> n = unknown nucleotide

<400> 2

```
Met Ala Gln Arg Ile Leu Val Leu His Gly Pro Asn Leu Asn Leu Leu
1               5                   10                  15
```

Gly Thr Arg Glu Pro His Ile Tyr Gly Ser Leu Thr Leu Ala Gln Ile
20 25 30

Asp Gln Gly Leu Ala Ala Leu Ala Gly Gln Leu Gly Val Ala Leu Thr
35 40 45

Ser Trp Gln Ser Asn His Glu Gly Ala Leu Val Glu Arg Ile Gln Ala
50 55 60

Ala Ala Ala Asp Gly Thr Asp Phe Ile Ile Ile Asn Ala Ala Ala Tyr
65 70 75 80

Thr His Thr Ser Val Ala Ile Arg Asp Ala Leu Ala Ala Val Ala Ile
85 90 95

Pro Phe Ile Glu Val His Leu Ser Asn Leu Tyr Lys Arg Asp Ser Phe
100 105 110

Arg Gln His Ser Tyr Leu Ser Asp Leu Ala Ile Gly Leu Ile Thr Gly
115 120 125

Leu Gly Ala Asp Gly Tyr Glu Ala Ala Leu Arg Tyr Ala Ala Arg His
130 135 140

<210> 3
<211> 435
<212> DNA
<213> Bordetella pertussis

<400> 3

atggcgcaac gcattcttgt tttgcatggt cccaacctga atctgctcgg cacccgggag 60

cctcatatct acggcagcct gacgctggcg cagatcgacc agggcttggc ggcgctggcc 120

ggccagctcg gcgtggcgct gacctcgtgg caaagcaatc acgaaggtgc gctggtcgag 180

cgcatccagg cagcggcggc cgacggtacc gatttcatca tcatcaacgc ggccgcctac 240

acgcacacca gtgtcgcgat ccgcgatgcg ctggcggcgg tggccatacc gtttattgaa 300

gtacatttgt ccaacctgta taagcgcgat tcattccggc agcattctta tctgtccgat 360

ctggcgatag gcctgattac cggcctgggc gccgatggct acgaggcggc gctgcgctac 420

gcggcgcgcc actga 435

<210> 4
<211> 30
<212> DNA
<213> Artificial Sequence

<220>
<223> FORKAN primer

<400> 4
gtgccggcgt gaatctctga tgttacattg 30

<210> 5
<211> 30
<212> DNA
<213> Artificial Sequence

<220>
<223> BACKAN primer

<400> 5
gggcgcgcac tagtgttaca accaattaac 30

<210> 6
<211> 20
<212> DNA
<213> Artificial Sequence

<220>
<223> FORQ primer

<400> 6
atggcgcaac gcattcttgt 20

<210> 7
<211> 20
<212> DNA
<213> Artificial Sequence

<220>
<223> BACQ2 primer

<400> 7
gttttgagtt ttcggaggtc 20